# EUROPÄISCHE PATENTSCHRIFT

(11) **EP 1 879 847 B1**
(45) Veröffentlichungstag und Bekanntmachung des Hinweises auf die Patenterteilung: **12.09.2012**
(21) Anmeldenummer: 06754981.6
(22) Anmeldetag: 03.05.2006
(51) Int. Cl.: C07C 63/48, C07D 311/78, C09B 3/14, C09B 5/62, C07D 491/06, C07F 5/04, C07D 493/06, C07D 471/16, C07D 221/18, C07D 471/22, C07C 43/257, C07D 491/16

(54) **TERRYLEN- UND QUATERRYLENDERIVATE**
TERRYLENE AND QUATERRYLENE DERIVATIVES
DERIVES DE TERRYLENE ET DE QUATERRYLENE

(30) Priorität: 04.05.2005 DE 102005021362
(43) Veröffentlichungstag der Anmeldung: 23.01.2008
(73) Patentinhaber: BASF SE, 67056 Ludwigshafen (DE)
(72) Erfinder: PSCHIRER, Neil, Gregory, 55116 Mainz (DE); QU, Jianqiang, 67061 Ludwigshafen (DE); KÖNEMANN, Martin, 68163 Mannheim (DE)
(74) Vertreter: Rippel, Hans Christoph
(86) Internationale Anmeldenummer: PCT/EP2006/062015
(87) Internationale Veröffentlichungsnummer: WO 2006/117383

(56) Entgegenhaltungen:
- EP-A- 0 638 613
- WO-A-96/22332
- WO-A-02/066438
- WO-A-03/104232
- FORMER C ET AL: "Cyclodehydrogenation of poly(perylene) to poly(quaterrylene): toward poly(pery-naphthalene)" MACROMOLECULES, AMERICAN CHEMICAL SOCIETY, EASTON, PA, US, Bd. 35, Nr. 5, 2002, Seiten 1576-1582, XP002219190 ISSN: 0024-9297
- GEERTS Y ET AL: "QUATERRYLENEBIS(DICARBOXIMIDE)S: NEAR INFRARED ABSORBING AND EMITTING DYES" JOURNAL OF MATERIALS CHEMISTRY, THE ROYAL SOCIETY OF CHEMISTRY, CAMBRIDGE, GB, Bd. 8, Nr. 11, November 1998 (1998-11), Seiten 2357-2369, XP000803180 ISSN: 0959-9428
- HOLTRUP F O ET AL: "TERRYLENIMIDES: NEW NIR FLUORESCENT DYES" CHEMISTRY - A EUROPEAN JOURNAL, VCH PUBLISHERS, US, Bd. 3, Nr. 2, 1997, Seiten 219-225, XP000931226 ISSN: 0947-6539
- NOLDE F ET AL: "Synthesis and modification of terrylenediimides as high-performance fluorescent dyes" CHEMISTRY - A EUROPEAN JOURNAL, VCH PUBLISHERS, US, Bd. 11, Nr. 13, 21. April 2005 (2005-04-21), Seiten 3959-3967, XP002365259 ISSN: 0947-6539

## Beschreibung

Die vorliegende Erfindung betrifft neue Rylenderivate der allgemeinen Formel I 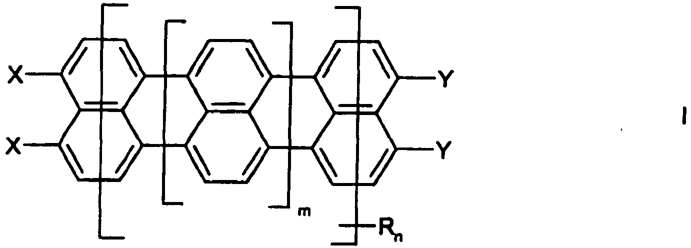 in der die Variablen folgende Bedeutung haben:
X miteinander unter Ausbildung eines Sechsrings verbunden zu einem Rest der Formel (a), (b) oder (c) 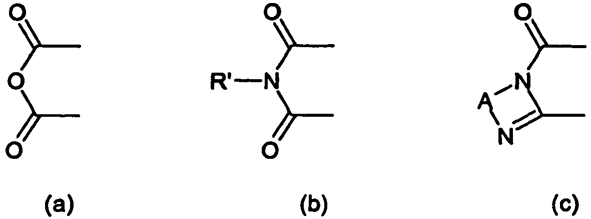 beide ein Rest -COOM;
   beide Wasserstoff oder einer der beiden Reste Wasserstoff und der andere Rest Halogen oder ein Rest der Formel (d) 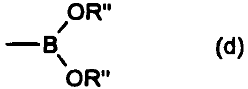
Y miteinander unter Ausbildung eines Sechsrings verbunden zu einem Rest der Formel (a), wenn einer der beiden Reste X Wasserstoff und der andere Rest X Halogen oder einen Rest der Formel (d) bedeutet oder wenn beide Reste X Wasserstoff oder zusammen einen Rest der Formel (a), (b) oder (c) bedeuten; miteinander unter Ausbildung eines Sechsrings verbunden zu einem Rest der Formel (b), wenn einer der beiden Reste X Wasserstoff und der andere Rest X Halogen oder einen Rest der Formel (d) bedeutet oder wenn beide Reste X Wasserstoff oder einen Rest -COOM oder zusammen einen Rest der Formel (c) bedeuten;
   miteinander unter Ausbildung eines Sechsrings verbunden zu einem Rest der Formel (c), wenn einer der beiden Reste X Wasserstoff und der an-dere Rest X Halogen oder einen Rest der Formel (d) bedeutet oder wenn beide Reste X Wasserstoff oder einen Rest -COOM oder zusammen einen Rest der Formel (c), der cis- oder trans-ständig zum anderen Rest (c) angeordnet sein kann, bedeuten; beide ein Rest -COOM, wenn einer der beiden Reste X Wasserstoff und der andere Rest X Halogen oder einen Rest der Formel (d) bedeutet oder wenn beide Reste X Wasserstoff oder einen Rest -COOM bedeuten, wobei für den Fall, daß ein Rest X einen Rest der Formel (d) bedeutet, M von Wasserstoff verschieden ist;
   beide Wasserstoff oder einer der beiden Reste Wasserstoff und der andere Rest Halogen oder ein Rest der Formel (d), wenn beide Reste X Wasserstoff oder einer der beiden Reste X Wasserstoff und der andere Rest X Halogen oder einen Rest der Formel (d) bedeutet;
R Phenoxy, Phenylthio, Pyridyloxy, Pyrimidyloxy, Pyridylthio oder Pyrimidyl-thio, das jeweils ein- oder mehrfach durch C₁-C₁₂-Alkyl, C₁-C₁₂-Alkoxy und/oder Aryl substituiert sein kann;
R' Wasserstoff;
   C₁-C₃₀-Alkyl, dessen Kohlenstoffkette durch eine oder mehrere Gruppierungen -O-und/oder -CO- unterbrochen sein kann und das ein- oder mehrfach substituiert sein kann durch: C₁-C₆-Alkoxy, Cyano und/oder Aryl, das durch C₁-C₁₈-Alkyl oder C₁-C₈-Alkoxy substituiert sein kann;
   Phenyl, Naphthyl, Pyridyl oder Pyrimidyl, das jeweils ein- oder mehrfach substituiert sein kann durch: C₁-C₁₈-Alkyl, C₁-C₈-Alkoxy, Halogen, Cyano, Nitro, -CONR²R³, - SO₂NR²R³, Phenyl- und/oder Naphthylazo, das jeweils durch C₁-C₁₀-Alkyl, C₁-C₈-Alkoxy und/oder Cyano substituiert sein kann;
   C₅-C₈-Cycloalkyl, das ein- oder mehrfach durch C₁-C₈-Alkyl substituiert sein kann;
A 1,2-Phenylen oder 1,8-Naphthylen;
M Alkalimetallkation;
R" miteinander verbunden unter Ausbildung eines die beiden Sauerstoffatome sowie das Boratom enthaltenden 5-glied/gen Rings, an den ein Benzolring oder ein bicyclischer gesättigter 7-gliedriger Ring anneliert sein kann, oder 6-gliedrigen Rings, die an den Kohlenstoffatomen durch bis zu 4 C₁-C₆-Alkylgruppen substituiert sein können;
   Wasserstoff, C₁-C₅-Alkyl oder Pinacolato;
R², R³ unabhängig voneinander Wasserstoff;
   C₁-C₁₈-Alkyl, das ein- oder mehrfach durch C₁-C₆-Alkoxy, Hydroxy, Halogen und/oder Cyano substituiert sein kann;
   Aryl oder Hetaryl, das jeweils ein- oder mehrfach durch C₁-C₆-Alkyl und/oder die vorstehenden, als Substituenten für Alkyl genannten Reste substituiert sein kann;
m 2;
n 4 oder 6,
und deren Mischungen.

Weiterhin betrifft die Erfindung die Herstellung der Rylenderivate I und ihre Verwendung zur Einfärbung von hochmolekularen organischen und anorganischen Materialien, zur Herstellung im nahinfraroten Bereich des elektromagnetischen Spektrums absorbierender wäßriger Polymerisatdispersionen, zur Erzeugung für das menschliche Auge nicht sichtbarer, Infrarotlicht absorbierender Markierungen und Beschriftungen, als Infrarotabsorber für das Wärmemanagement, als IR-lasertrahlenabsorbierende Materialien beim Schweißbehandeln von Kunststoffteilen, als Halbleiter in der organischen Elektronik, als Emitter in Elektro- und Chemilumineszenzanwendungen sowie als Aktivkomponenten in der Photovoltaik.

Im Nahinfrarotbereich des elektromagnetischen Spektrums absorbierende Verbindungen sind für eine Vielzahl von Anwendungen von zunehmendem Interesse. Eine Klasse derartiger organischer Verbindungen stellen polycyclische konjugierte aromatische Ringsysteme auf Rylenbasis dar.

Auf Basis der besonders interessanten höheren Rylene sind bislang unsubstituierte und tetra- sowie hexahalogen- und -aroxysubstituierte
Quaterrylentetracarbonsäurediimide (EP-A-596 292 bzw. WO-A-96/22332 und WO-A-02/76988), unsubstituierte und in peri-Position halogenierte Terrylen- und Quatenylendicarbonsäureimide
(WO-A-02/66438) sowie unsubstituierte und tetrahalogen- und -aroxysubstituierte Terrylentetracarbonsäurediimide (WO-A-03/104232) bekannt.

Der Erfindung lag die Aufgabe zugrunde, weitere Verbindungen bereitzustellen, die im Wellenlängenbereich von 550 bis 900 nm absorbieren und sich durch vorteilhafte Anwendungseigenschaften auszeichnen, insbesondere auch so funktionalisiert sind, daß sie an den gewünschten Anwendungszweck gezielt angepaßt oder auch zu noch längerwellig absorbierender Verbindungen umgesetzt werden können.

Demgemäß wurden die Quaterrylenderivate der eingangs definierten Formel I gefunden.

Die Quaterrylenderivate I können 3 bis 6 Substituenten R tragen, vorzugsweise sind sie vierfach in 1,6,11,16-Position oder sechsfach in 1,6,8,11,16,18- oder 1,6,8,11,16,19-Position substituiert.

In der Regel fallen sowohl die Quaterrylenderivate I in Form von Mischungen von Produkten mit unterschiedlichem Substitutionsgrad an, in denen das tetrasubstituierte bzw. das hexasubstitutierte Produkt jeweils den Hauptanteil ausmacht.

Weiterhin wurde ein Verfahren zur Herstellung von Rylentetracarbonsäuremonoimidmonoanhydriden der allgemeinen Formel Ia 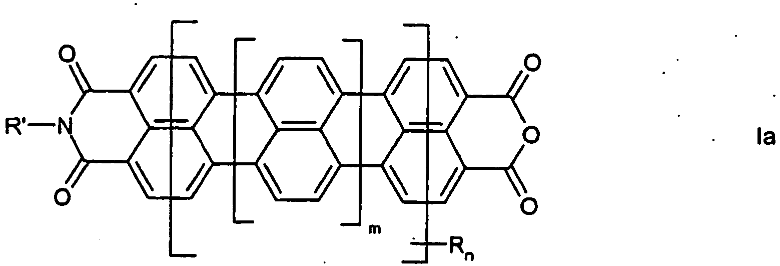 gefunden, das dadurch gekennzeichnet ist, daß man
a) ein Rylentetracarbonsäurediimid der allgemeinen Formel II 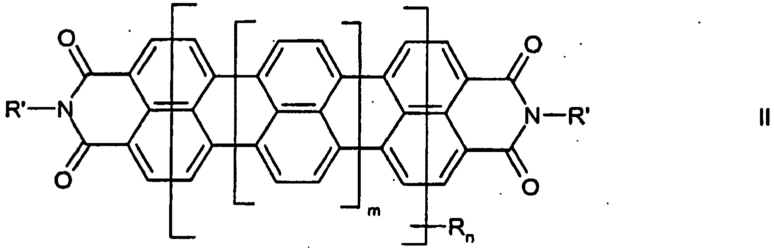 einer Verseifung unter alkalischen Bedingungen in Gegenwart eines polaren organischen Lösungsmittels unterzieht und das Rylentetracarbonsäuremonoimidmonoanhydrid Ia von dem hierbei ebenfalls entstehenden Rylentetracarbonsäuredianhydrid Ib abtrennt oder
b) das Rylentetracarbonsäurediimid II durch Anwendung milder Reaktionsbedingungen direkt weitgehend einseitig zum Rylentetracarbonsäuremonoimidmonoanhydrid Ia verseift.

Außerdem wurde ein Verfahren zur Herstellung von Rylentetracarbonsäuredianhydriden der allgemeinen Formel Ib 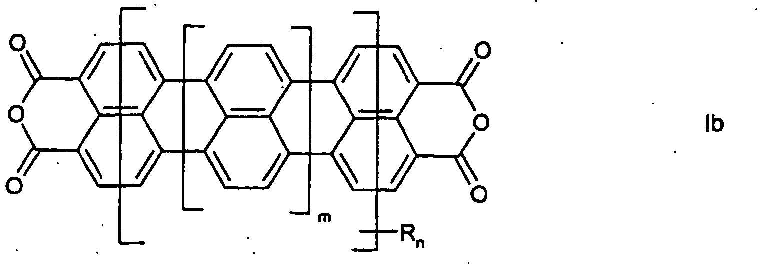 gefunden, das dadurch gekennzeichnet ist, daß man
a) ein Rylentetracarbonsäurediimid der allgemeinen Formel II 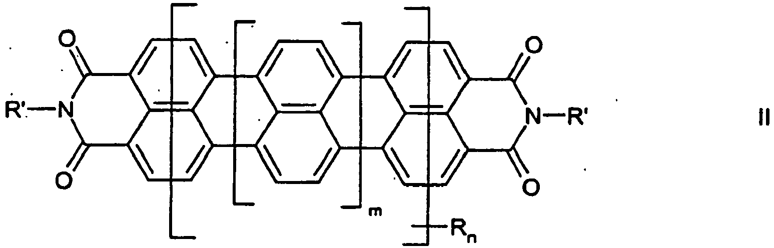 einer Verseifung unter alkalischen Bedingungen in Gegenwart eines polaren organischen Lösungsmittels unterzieht und das Rylentetracarbonsäuredianhydrid Ib von dem hierbei ebenfalls entstehenden Rylentetracarbonsäuremonoimidmonoanhydrid Ia abtrennt
   oder
b) das Rylentetracarbonsaurediimid II durch Anwendung verschärfter Reaktionsbedingungen direkt weitgehend beidseitig zum Rylentetracarbonsäuredianhydrid Ib verseift.

Weiterhin wurde ein Verfahren zur Herstellung von Rylendicarbonsäureimiden der allgemeinen Formel Ic 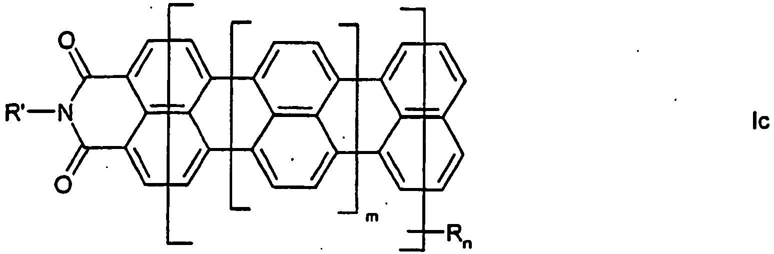 gefunden, das dadurch gekennzeichnet ist, daß man
a) ein Rylentetracarbonsäuremonoimidmonoanhydrid der allgemeinen Formel Ia 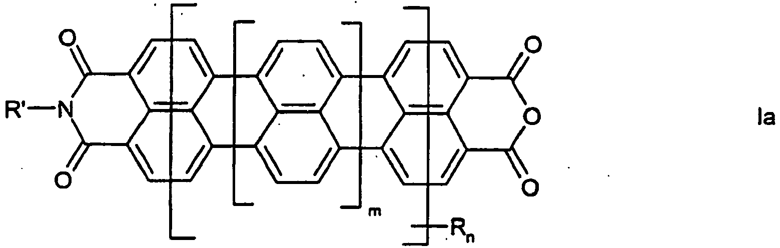 oder
b) das bei der alkalischen Verseifung eines Rytentetracarbonsäurediimids der allgemeinen Formel II 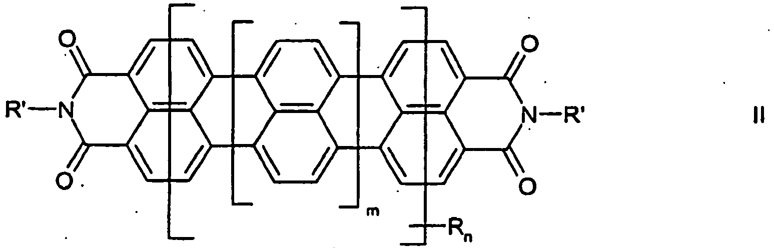 anfallende Gemisch von Rylentetracarbonsäuremonoimidmonoanhydrid Ia und Rylentetracarbonsäuredianhydrid Ib
   einer Decarboxylierung in Gegenwart einer tertiären stickstoffbasischen Verbindung als Lösungsmittel und eines Übergangsmetallkatalysators unterzieht und im Fall b) das Rylendicarbonsäureimid Ic von dem ebenfalls entstehenden vollständig decarboxylierten Rylen Id trennt.

Außerdem wurde eine Verfahren zur Herstellung von Rylenen der allgemeinen Formel Id 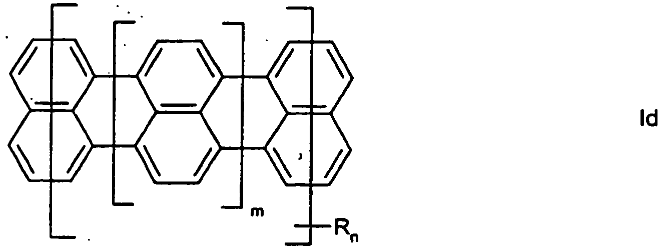 gefunden, das dadurch gekennzeichnet ist, daß man
a) ein Rylentetracarbonsäuredianhydrid der allgemeinen Formel Ib 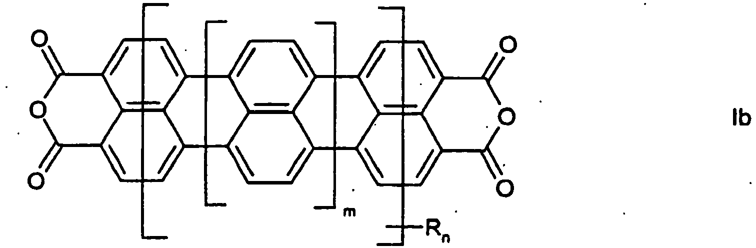 oder
b) das bei der alkalischen Verseifung eines Rylentetracarbonsäurediimids der allgemeinen Formel II 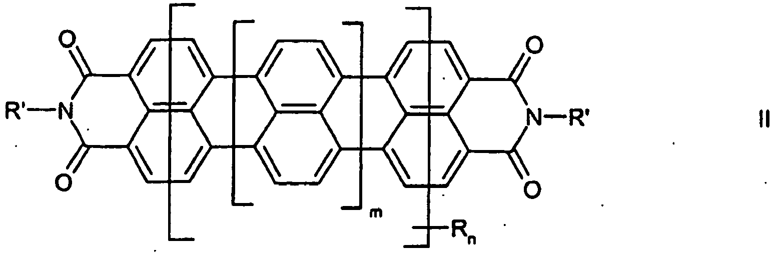 anfallende Gemisch von Rylentetracarbonsäuremonoimidmonoanhydrid Ia und Rylentetracarbonsäuredianhydrid Ib
   einer Decarboxylierung in Gegenwart einer tertiären stickstoffbasischen Verbindung als Lösungsmittel und eines Übergangsmetallkatalysators unterzieht und im Fall b) das Rylen Id von dem ebenfalls entstehenden Rylendicarbonsäureimid Ic trennt.

Weiterhin wurde ein Verfahren zur Herstellung von peri-halogenierten Rylendicarbonsäureimiden der allgemeinen Formel Ie 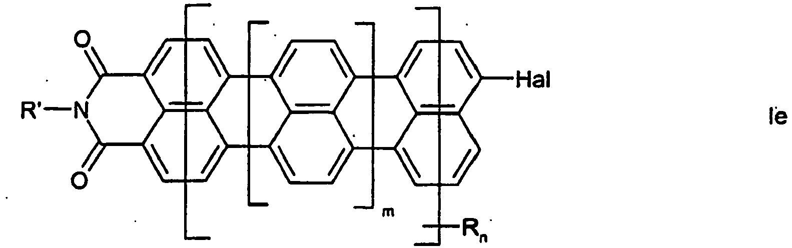 in der Hal Halogen bedeutet, gefunden, das dadurch gekennzeichnet ist, daß man
a) ein Rylendicarbonsäureimid der allgemeinen Formel Ic 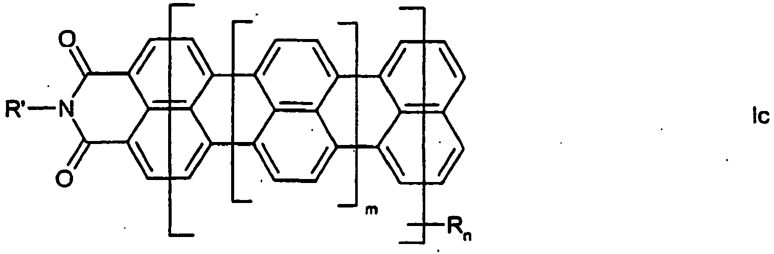 oder
b) das Gemisch von Rylendicarbonsäureimid Ic und Rylen Id, das bei der Decarboxylierung des bei der alkalischen Verseifung des Rylentetracarbonsäurediimids der allgemeinen Formel II 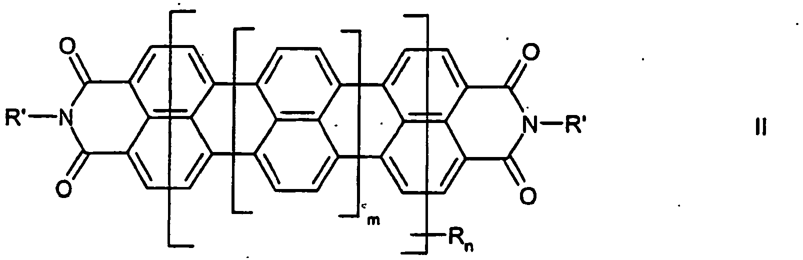 anfallenden Gemischs von Rylentetracarbonsäuremonoimidmonoanhydrid Ia und Ryfentetracarbonsäuredianhydrid Ib anfällt,
   in Gegenwart eines polaren organischen Lösungsmittels und einer Lewis-Säure als Katalysator mit 1 bis 6 mol N-Halogensuccinimid pro einzuführendes Halogenatom umsetzt und im Fall b) das peri-halogenierte Rylendicarbonsäureimid le von dem ebenfalls halogenierten Rylen If trennt

Außerdem wurde ein Verfahren zur Verfahren zur Herstellung von halogenierten Rylenen der allgemeinen Formel If 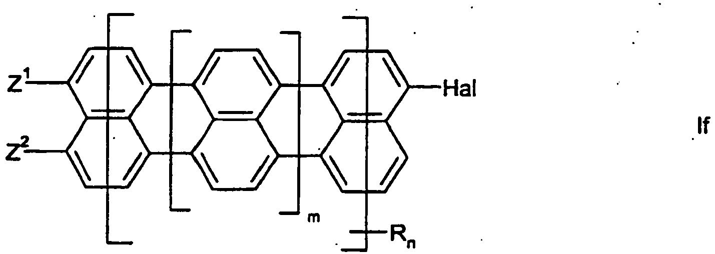 in der Hal für Halogen steht, Z¹ und Z² Wasserstoff bedeuten oder einer der beiden Reste Z¹ oder Z² Halogen und der andere Rest Wasserstoff bedeutet, gefunden, das dadurch gekennzeichnet ist, daß man
a) ein Rylen der allgemeinen Formel Id 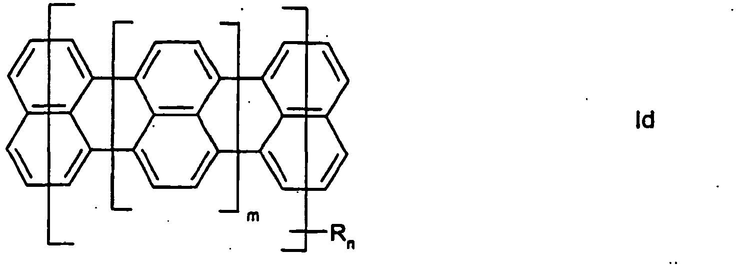 in Gegenwart eines polaren organischen Lösungsmittels und einer Lewis-Säure als Katalysator
   a1) direkt mit der zur Einführung der insgesamt gewünschten Anzahl Halogenatome erforderlichen Menge von 1 bis 6 mol N-Halogensuccinimid pro einzuführendes Halogenatom
      oder
   a2) zunächst mit 1 bis 3 mol/mol N-Halogensuccinimid zum monohalogenierten Rylen If (Z¹=Z²=H) und dann mit weiteren 1 bis 6 mol/mol N-Halogensuccinimid zum dihalogenierten Rylen If (Z¹ oder Z²=Halogen) umsetzt
      oder
b) das Gemisch von Rylendicarbonsäureimid Ic und Rylen Id, das bei der Decarboxylierung des bei der alkalischen Verseifung des Rylentetracarbonsäurediimids der allgemeinen Formel II 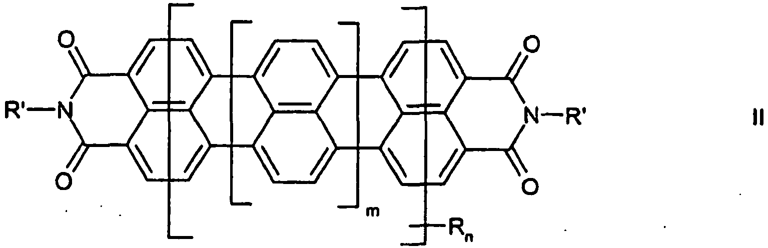 anfallenden Gemischs von Rylentetracarbonsäuremonoimidmonoanhydrid Ia und Rylentetracarbonsäuredianhydrid Ib anfällt,
   in Gegenwart eines polaren organischen Lösungsmittels und einer Lewis-Säure als Katalysator
   b1) direkt mit der zur Einführung der insgesamt gewünschten Anzahl Halogenatome erforderlichen Menge von 1 bis 6 mol N-Halogensuccinimid pro einzuführendes Halogenatom
      oder
   b2) zunächst mit 1 bis 3 mol N-Halogensuccinimid pro mol Id und Ic umsetzt und das monohalogenierte Rylen If (Z¹=Z²=H) von dem ebenfalls gebildeten peri-halogenierten Rylendicarbonsäureimid Ie trennt und dann mit weiteren 1 bis 6 mol/mol N-Halogensuccinimid zum dihalogenierten Rylen If (Z¹ oder Z²=Halogen) umsetzt.

Schließlich wurde ein Verfahren zur Herstellung von Rylendicarbonsäureanhydriden lgh 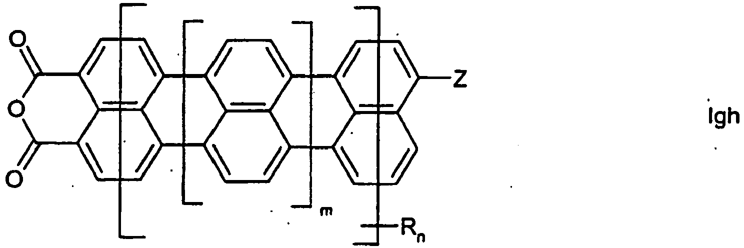 in der Z Wasserstoff oder Halogen bedeutet, gefunden, das dadurch gekennzeichnet ist, daß man ein Rylendicarbonsäureimid der allgemeinen Formel Ice 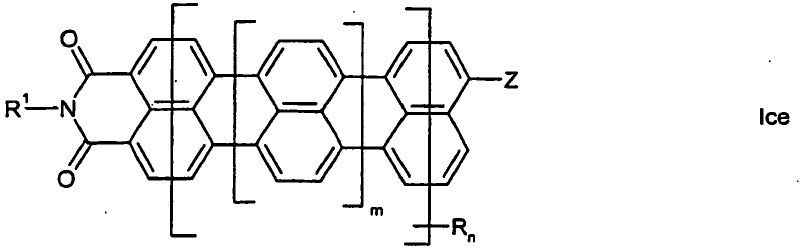 einer Verseifung unter alkalischen Bedingungen in Gegenwart eines polaren organischen Lösungsmittels unterzieht.

Außerdem wurde ein Verfahren zur Herstellung von peri-halogenierten Rylendicarbonsäureanhydriden Ih 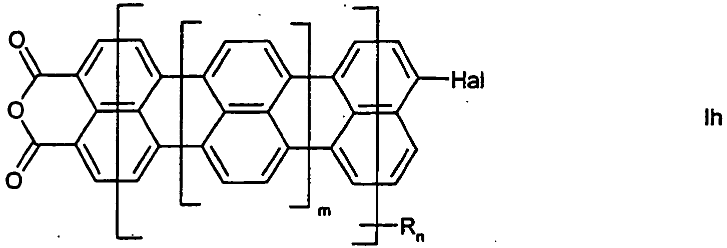 in der Hal Halogen bedeutet, gefunden, das dadurch gekennzeichnet ist, daß man ein Rylendicarbonsäureanhydrid der allgemeinen Formel Ig 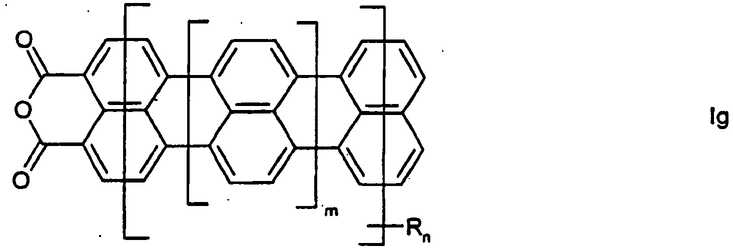 in Gegenwart eines polaren organischen Lösungsmittels und einer Lewis-Säure mit N-Halogensuccinimid umsetzt.

Weiterhin wurde ein Verfahren zur Herstellung von peri-(Dioxaborolan-2-yl)rylendicarbonsäureimiden der allgemeinen Formel Ii 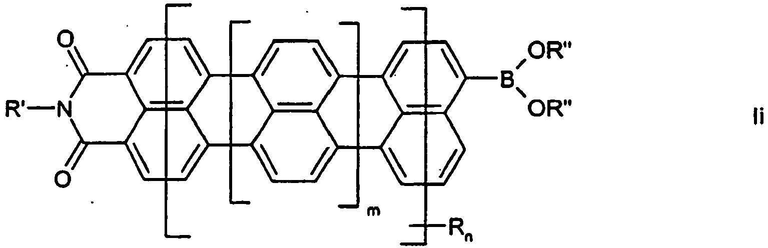 gefunden, das dadurch gekennzeichnet ist, daß man ein peri-halogeniertes Rylendicarbonsäureimid der allgemeinen Formel Ie 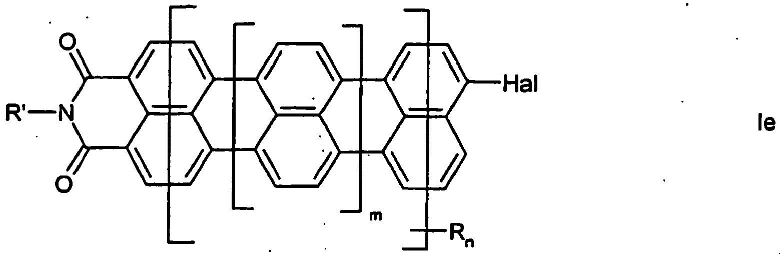 in der Hal Halogen bedeutet, in Gegenwart eines aprotischen organischen Lösungsmittels, eines Übergangsmetallkatalysators und einer Base mit einem Diboran der allgemeinen Formel III 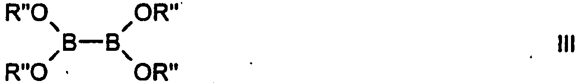 umsetzt.

Außerdem wurde ein Verfahren zur Herstellung von subsituierten Rylenen der allgemeinen Formel Ij 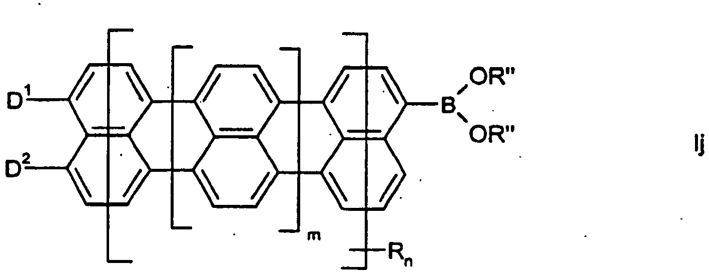 in der D¹ und D² Wasserstoff bedeuten oder einer der beiden Reste D¹ oder D² Halogen oder einen Rest der Formel (d) 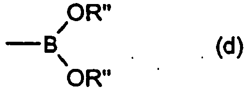 und der andere Rest Wasserstoff bedeutet, gefunden, das dadurch gekennzeichnet ist, daß man
a) zur Herstellung von Mono(dioxaborolan-2-yl)rylenen Ij (D¹=D²=H) ein Halogenrylen der allgemeinen Formel If 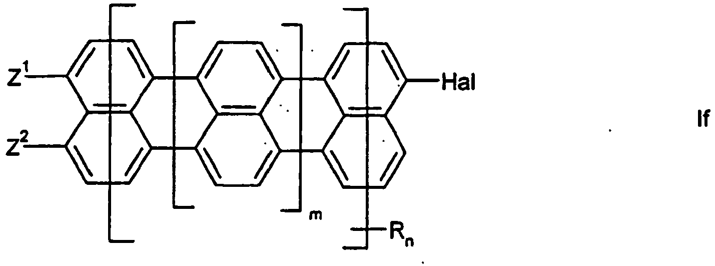 in der Z¹ und Z² Wasserstoff bedeuten,
   oder
b) zur Herstellung von Bis(dioxaborolan-2-yl-)rylenen Ij (einer der beiden Reste D¹ oder D² ein Rest (d) und der andere Rest Wasserstoff) oder gemischtsubstituierten Rylenen Ij (einer der beiden Reste D¹ oder D² Halogen und der andere Rest Wasserstoff) ein Halogenrylen der Formel If 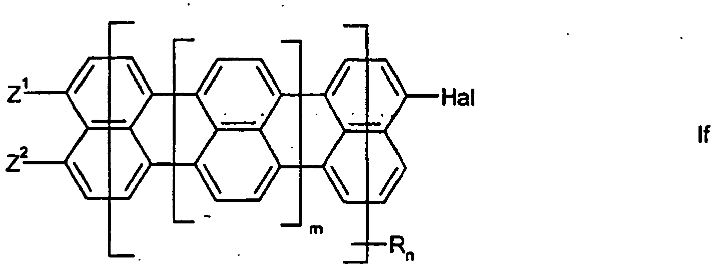 in der einer der beiden Reste Z¹ oder Z² Halogen und der andere Rest Wasserstoff bedeutet,
   in Gegenwart eines aprotischen organischen Lösungsmittels, eines Übergangsmetallkatalysators und einer Base mit der zur Einführung der insgesamt gewünschten Anzahl an Dioxaborolan-2-ylresten erforderlichen Menge von 1 bis 3 mol bzw. im Fall der gemischtsubstituierten Rylene Ij 1 bis 1,5 mol eines Diborans der allgemeinen Formel III 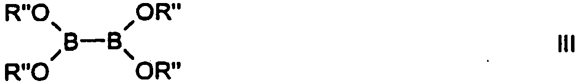 pro mol einzuführender Dioxaborolan-2-ylrest umsetzt

Schließlich wurde ein Verfahren zur Herstellung von peri-(Dioxaborolan-2-yl)rylendicarbonsäureanhydriden der allgemeinen Formel Ik 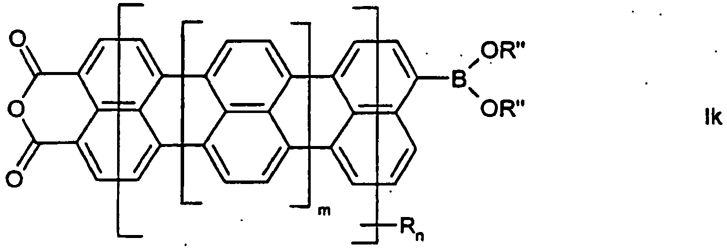 gefunden, das dadurch gekennzeichnet ist, daß man ein peri-halogeniertes Rylendicarbonsäureanhydrid der allgemeinen Formel Ih 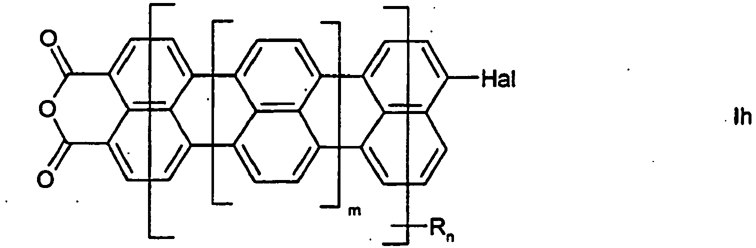 in der Hal Halogen bedeutet, in Gegenwart eines aprotischen organischen Lösungsmittels, eines Übergangsmetallkatalysators und einer Base mit einem Diboran der allgemeinen Formel III 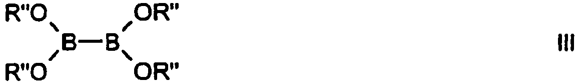 umsetzt.

Weiterhin wurde ein Verfahren zur Herstellung von symmetrischen Rylentetracarbonsäurederivaten der allgemeinen Formel Im_{cis}, oder Imₜᵣₐₙₛ 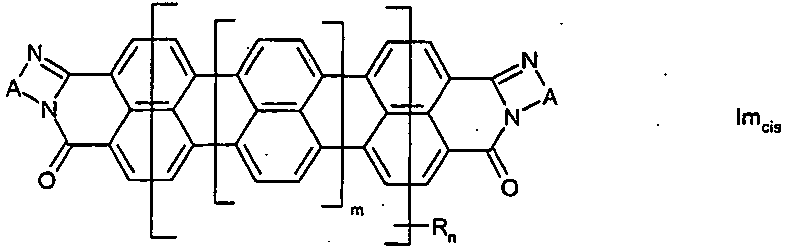 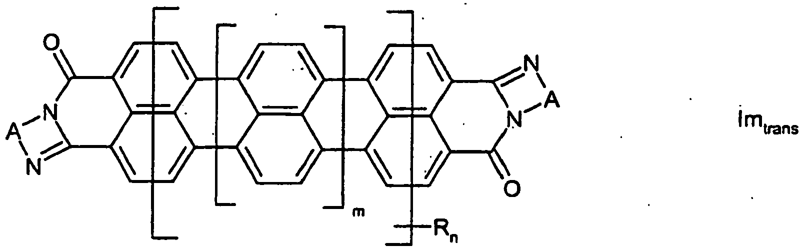 in der die beiden Reste A gleich sind, oder einer Mischung beider Isomere gefunden, das dadurch gekennzeichnet ist, daß man ein Rylentetracarbonsäuredianhydrid der allgemeinen Formel Ib 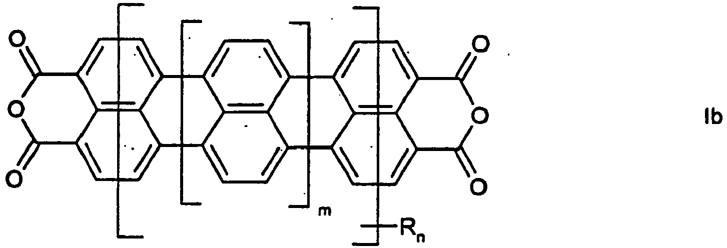 in Gegenwart einer stickstoffbasischen Verbindung oder von Phenol als Lösungsmittel und einer Lewis-Säure oder von Piperazin als Katalysator mit 2 bis 3 mol/mol eines aromatischen Diamins der allgemeinen Formel IV

H₂N-A-NH₂ IV

kondensiert.

Außerdem wurde ein Verfahren zur Herstellung von unsymmetrischen Rylentetracarbonsäurederivaten der allgemeinen Formel Im'_{cis} oder Im'ₜᵣₐₙₛ 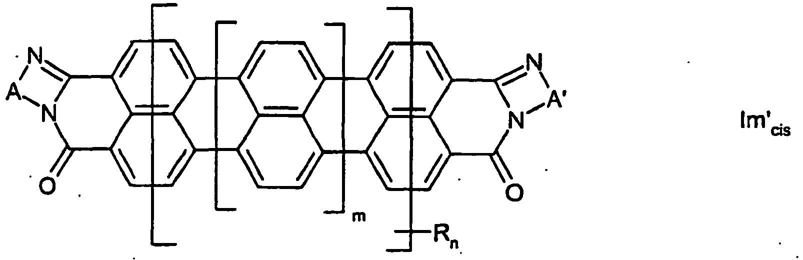 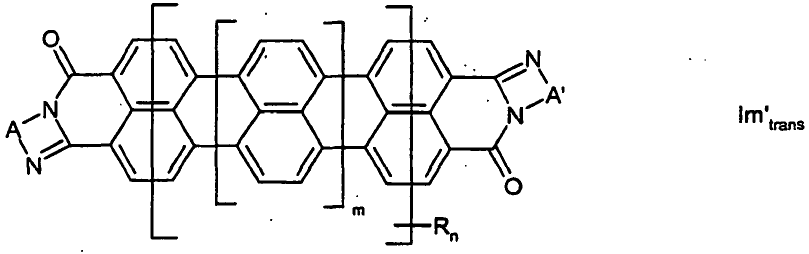 oder einer Mischung beider Isomere gefunden, das dadurch gekennzeichnet ist, daß man ein Rylentetracarbonsäuredianhydrid der allgemeinen Formel Ib 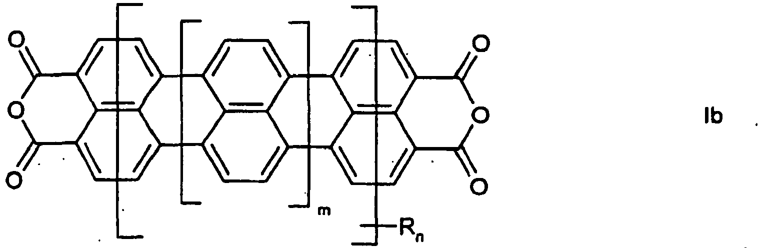 in Gegenwart einer stickstoffbasischen Verbindung oder von Phenol als Lösungsmittel und einer Lewis-Säure oder von Piperazin als Katalysator zunächst mit 1 bis 1,5 mol/mol eines aromatischen Diamins der allgemeinen Formel IV

H₂N-A-NH₂ IV

und dann mit 1 bis 1,5 mol/mol eines aromatischen Diamins der allgemeinen Formel IV'

H₂N-A'-NH₂ IV'

kondensiert.

Weiterhin wurde ein Verfahren zur Herstellung von Rylentetracarbonsäurederivaten der allgemeinen Formel In 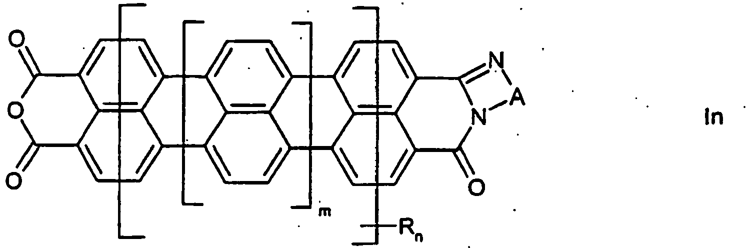 gefunden, das dadurch gekennzeichnet ist, daß man ein Rylentetracarbonsäuredianhydrid der allgemeinen Formel Ib 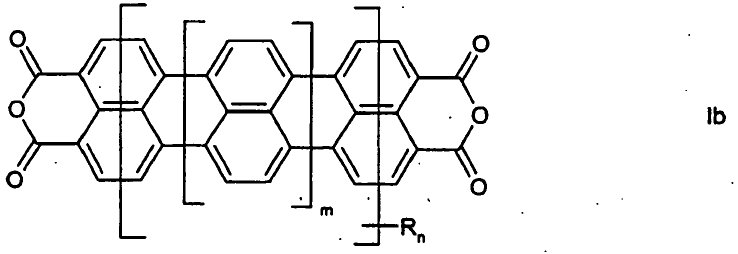 in Gegenwart einer stickstoffbasischen Verbindung oder von Phenol als Lösungsmittel und einer Lewis-Säure oder von Piperazin als Katalysator mit 1 bis 1,5 mol/mol eines aromatischen Diamins der allgemeinen Formel IV

H₂N-A-NH₂ IV

kondensiert.

Außerdem wurde ein Verfahren zur Herstellung von Rylentetracarbonsäurederivaten der allgemeinen Formel Io 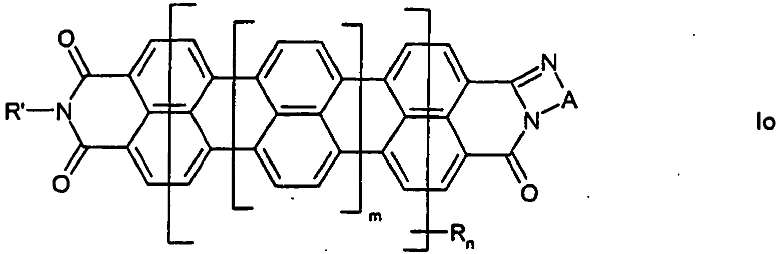 gefunden, das dadurch gekennzeichnet ist, daß man ein Rylentetracarbonsäuremonoimidmonoanhydrid der allgemeinen Formel Ia 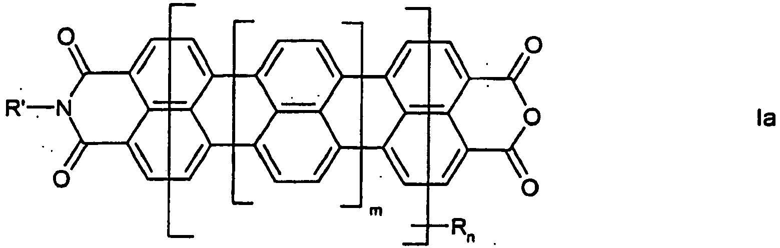 in Gegenwart einer stickstoffbasischen Verbindung oder von Phenol als Lösungsmittel und einer Lewis-Säure oder von Piperazin als Katalysator mit 1 bis 1,5 mol/mol eines aromatischen Diamins der allgemeinen Formel IV

H₂N-A-NH₂ IV

kondensiert.

Außerdem wurde ein Verfahren zur Herstellung von Rylendicarbonsäurederivaten der allgemeinen Formel Ip 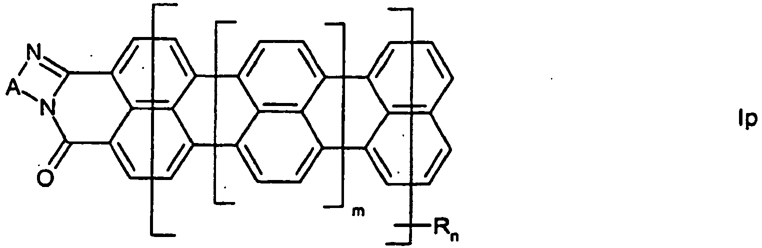 gefunden, das dadurch gekennzeichnet ist, daß man
a) ein Rylentetracarbonsäurederivat der allgemeinen Formel In 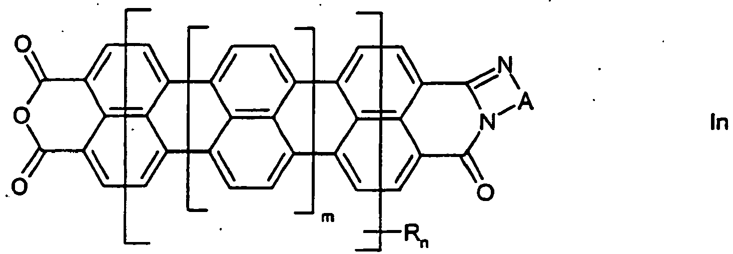 einer Decarboxylierung in Gegenwart einer tertiären stickstoffbasischen Verbindung und eines Übergangsmetallkatalysators unterzieht
   oder
b) ein Rylendicarbonsäureanhydrid der allgemeinen Formel Ig 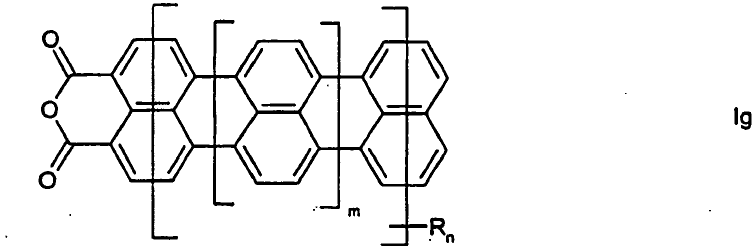 in Gegenwart einer stickstoffbasischen Verbindung oder von Phenol als Lösungsmittel und einer Lewis-Säure oder von Piperazin als Katalysator mit 1 bis 1,5 mol/mol eines aromatischen Diamins der allgemeinen Formel IV

   H₂N-A-NH₂ IV

   kondensiert.

Weiterhin wurde ein Verfahren zur Herstellung von peri-halogenierten Rylendicarbonsäurederivaten der allgemeinen Formel Iq 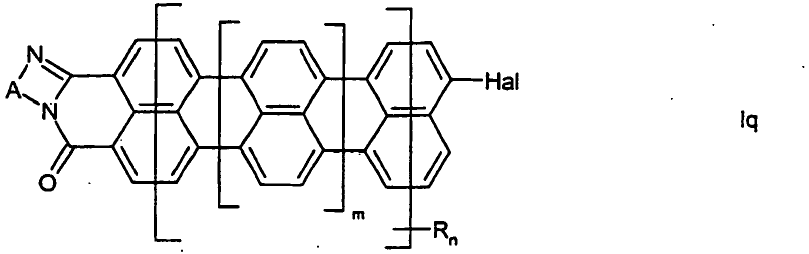 gefunden; das dadurch gekennzeichnet ist, daß man ein Rylendicarbonsäurederivat der allgemeinen Formel Ip 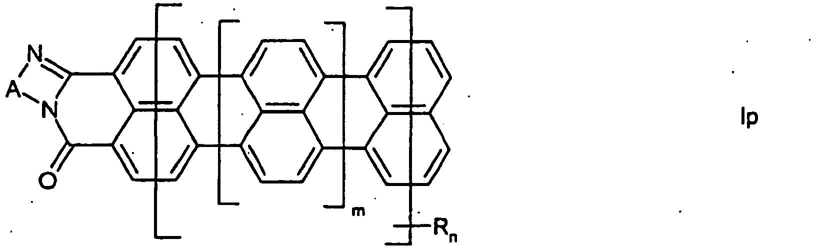 in Gegenwart eines polaren organischen Lösungsmittels und einer Lewis-Säure mit N-Halogensuccinimid umsetzt.

Schließlich wurde ein Verfahren zur Herstellung von peri-(Dioxaborolan-2-yl)rylendicarbonsäurederivaten der allgemeinen Formel Ir 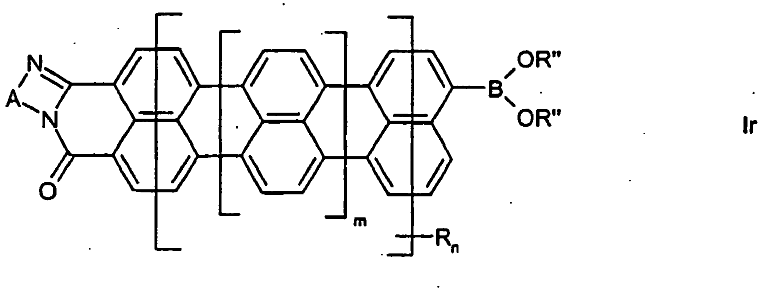 gefunden, das dadurch gekennzeichnet ist, daß man ein peri-halogeniertes Rylendicarbonsäurederivat der allgemeinen Formel Iq 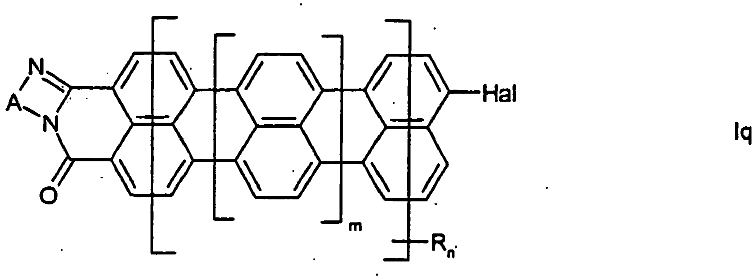 in der Hal Halogen bedeutet, in Gegenwart eines aprotischen organischen Lösungsmittels, eines Übergangsmetallkatalysators und einer Base mit einem Diboran der allgemeinen Formel III 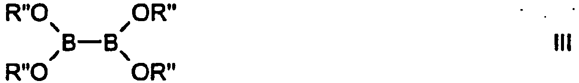 umsetzt.

Als Beispiele für die in den Formeln genannten Reste R, R', R", R² bis R³ sowie deren Substituenten seien im einzelnen genannt (diese Definitionen sind nur möglich, falls
die sich ergebenden Reste R, R', R", R² und R³ unter Anspruch 1 fallen):
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen);
2-Methoxyethyl, 2-Ethoxyethyl, 2-Propoxyethyl, 2-Isopropoxyethyl, 2-Butoxyethyl, 2-und 3-Methoxypropyl, 2- und 3-Ethoxypropyl, 2- und 3-Propoxypropyl, 2- und 3-Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2-und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2- und 3- Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
(1-Ethylethyliden)aminoethylen, (1-Ethylethyliden)aminopropylen, (1-Ethylethyliden)-aminobutylen, (1-Ethylethyliden)aminodecylen und (1-Ethylethyliden)aminododecylen;
Propan-2-on-1-yl, Butan-3-on-1-yl, Butan-3-on-2-yl und 2-Ethylpentan-3-on-1-yl;
2-Methylsulfoxidoethyl, 2-Ethylsulfoxidoethyl, 2-Propylsulfoxidoethyl, 2-Isopropylsulf oxidoethyl, 2-Butylsulfoxidoethyl, 2- und 3-Methylsulfoxidopropyl, 2- und 3-Ethylsulf oxidopropyl, 2- und 3-Propylsulfoxidopropyl, 2- und 3-Butylsulfoxidopropyl, 2- und 4-Methylsulfoxidobutyl, 2- und 4-Ethylsulfoxidobutyl, 2- und 4-Propylsulfoxidobutyl und 4-Butylsulfoxidobutyl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylproypl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxyethyl, 2- und 3-Hydroxypropyl, 1-Hydroxyprop-2-yl, 3- und 4-Hydroxybutyl, 1-Hydroxybut-2-yl und 8-Hydroxy-4-oxaoctyl;
2-Cyanoethyl, 3-Cyanopropyl, 3- und 4-Cyanobutyl, 2-Methyl-3-ethyl-3-cyanopropyl, 7-Cyano-7-ethylheptyl und 4-Methyl-7-methyl-7-cyanoheptyl;
2-Chlorethyl, 2- und 3-Chloropropyl, 2-, 3- und 4-Chlorbutyl, 2-Bromethyl, 2- und 3-Brompropyl und 2-, 3- und 4-Brombutyl;
2-Nitroethyl, 2- und 3-Nitropropyl und 2-, 3- und 4-Nitrobutyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Methylthio, Ethylthio, Propylthio, Isopropylthio, Butylthio, lsobutylthio, sec.-Butylthio, tert.-Butylthio, Pentylthio, Isopentylthio, Neopentylthio, tert.-Pentylthio und Hexylthio;
Ethinyl, 1- und 2-Propinyl, 1-, 2- und 3-Butinyl, 1-, 2-, 3- und 4-Pentinyl, 1-, 2-, 3-, 4-und 5-Hexinyl, 1-, 2-, 3-, 4-, 5-, 6-, 7-, 8- und 9-Decinyl, 1-, 2-, 3-, 4-, 5-, 6-, 7-, 8-, 9-, 10- und 11-Dodecinyl und 1-, 2-, 3-, 4-, 5-, 6-, 7-, 8-, 9-, 10-, 11-, 12-, 13-, 14-, 15-, 16-und 17-Octadecinyl;
Ethenyl, 1- und 2-Propenyl, 1-, 2- und 3-Butenyl, 1-, 2-, 3- und 4-Pentenyl, 1-, 2-, 3-, 4-und 5-Hexenyl, 1-, 2-, 3-, 4-, 5-, 6-, 7-, 8- und 9-Decenyl, 1-, 2-, 3-, 4-, 5-, 6-; 7-, 8-, 9-, 10- und 11-Dodecenyl und 1-, 2-, 3-, 4-, 5-, 6-, 7-, 8-, 9-, 10-, 11-, 12-, 13-, 14-, 15-, 16-und 17-Octadecenyl;
Methylamino, Ethylamino, Propylamino, Isopryplamino, Butylamino, Isobutylamino, Pentylamino, Hexylamino, Dimethylamino, Methylethylamino, Diethylamino, Dipropylamino, Diisopropylamino, Dibutylamino, Diisobutylamino, Dipentylamino, Dihexylamino, Dicyclopentylamino, Dicyclohexylamino, Dicycloheptylamino, Diphenylamino und Dibenzylamino;
Formylamino, Acetylamino, Propionylamine und Benzoylamino;
Carbamoyl, Methylaminodarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Aminosulfonyl, N,N-Dimethylaminosulfonyl, N,N-Diethylaminosulfonyl, N-Methyl-N-ethylaminosulfonyl, N-Methyl-N-dodecylaminosulfonyl, N-Dodecylaminosulfonyl, (N,N-Dimethylamino)ethylaminosulfonyl, N,N-(Propoxyethyl)dodecylaminosulfonyl, N,N-Diphenylaminosulfonyl, N,N-(4-tert.-Butylphenyl)octadecylaminosulfonyl und N,N-Bis(4-Chlorphenyl)aminosulfonyl;
Methoxycarbonyl, Ethoxycarbonyl, Propoxycarbonyl, Isopropoxycarbonyl, Hexoxycarbonyl, Dodecyloxycarbonyl, Octadecyloxycarbonyl, Phenoxycarbonyl, (4-tert.-Butylphenoxy)carbonyl und (4-Chlorphenoxy)carbonyl;
Methoxysulfonyl, Ethoxysulfonyl, Propoxysulfonyl, Isopropoxysulfonyl, Butoxysulfonyl, Isobutoxysulfonyl, tert.-Butoxysulfonyl, Hexoxysulfonyl, Dodecyloxysulfonyl, Octadecyloxysulfonyl, Phenoxysulfonyl, 1- und 2-Naphthyloxysulfonyl, (4-tert.-Butylphenoxy)-sulfonyl und (4-Chlorphenoxy)sulfonyl;
Diphenylphosphino, Di-(o-tolyl)phosphino und Diphenylphosphinoxido;
Chlor, Brom und iod;
Phenylazo, 2-Napthylazo, 2-Pyridylazo und 2-Pyrimidylazo;
Cyclopropyl, Cyclobutyl, Cyclopentyl, 2- und 3-Methylcyclopentyl, 2- und 3-Ethylcyclopentyl, Cyclohexyl, 2-, 3- und 4-Methylcyclohexyl, 2-,3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl und 3-, 4- und 5-Propylcyclooctyl; 3- und 4-Hydroxycyclohexyl, 3- und 4- Nitrocyclohexyl und 3- und 4-Chlorcyclohexyl;
1-, 2- und 3-Cyclopentenyl, 1-, 2-, 3- und 4-Cyclohexenyl, 1-, 2- und 3-Cycloheptenyl und 1-, 2-, 3- und 4-Cyclooctenyl;
2-Dioxanyl,- 1-Morpholinyl, 1-Thiomorpholinyl, 2- und 3-Tetrahydrofuryl, 1-, 2- und 3-Pyrrolidinyl, 1-Piperazyl, 1-Diketopiperazyl und 1-, 2-, 3- und 4-Piperidyl;
Phenyl, 2-Naphthyl, 2- und 3-Pyrryl, 2-, 3- und 4-Pyridyl, 2-, 4- und 5-Pyrimidyl, 3-, 4-und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5-Isochinolyl;
1-, 2-, 3-, 4-, 5-, 6- und 7-Indolyl, 1-, 2-, 3-, 4-, 5-, 6- und 7-Isoindolyl, 5-(4-Methylisoindolyl), 5-(4-Phenylisoindolyl), 1-, 2-, 4-, 6-, 7- und 8-(1,2,3,4-Tetrahydrolsochinolinyl), 3-(5-Phenyl)-(1,2,3,4-tetrahydroisochinolinyl), 5-(3-Dodecyl-(1,2,3,4-tetrahydroisochinolinyl), 1-, 2-, 3-, 4-, 5-, 6-, 7- und 8-(1,2,3,4-Tetrahydrochinolinyl) und 2-, 3-, 4-, 5-, 6-, 7- und 8-Chromanyl, 2-, 4- und 7-Chinolinyl, 2-(4-Phenylchinolinyl) und 2-(5-Ethylchinolinyl);
2-, 3- und 4-Methylphenyl, 2,4-, 3,5- und 2,6-Dimethylphenyl, 2,4,6-Trimethylphenyl, 2-, 3- und 4-Ethylphenyl, 2,4-, 3,5- und 2,6-Diethylphenyl, 2,4,6-Triethylphenyl, 2-, 3- und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Triisopropylphenyl, 2-, 3-und 4-Butylphenyl, 2,4-, 3,5- und 2,6-Dibutylphenyl, 2,4,6-Tributylphenyl, 2-, 3- und 4-Isobutylphenyl, 2,4-, 3,5- und 2,6-Diisobutylphenyl, 2,4,6-Triisobutylphenyl, 2-, 3- und 4-sec.-Butylphenyl, 2,4-, 3,5- und 2,6-Di-sec.-butylphenyl und 2,4,6-Tri-sec.-butylphenyl; 2-, 3- und 4-Methoxyphenyl, 2,4-, 3,5- und 2,6-Dimethoxyphenyl, 2,4,6-Trimethoxyphenyl, 2-, 3- und 4-Ethoxyphenyl, 2,4-, 3,5- und 2,6-Diethoxyphenyl, 2,4,6-Triethoxyphenyl, 2-, 3- und 4-Propoxyphenyl, 2,4-, 3,5- und 2,6-Dipropoxyphenyl, 2-, 3-und 4-Isopropoxyphenyl, 2,4- und 2,6-Diisopropoxyphenyl und 2-, 3- und 4-Butoxyphenyl; 2-, 3- und 4-Chlorphenyl und 2,4-, 3,5- und 2,6-Dichlorphenyl; 2-, 3- und 4-Hydroxyphenyl und 2,4-, 3,5- und 2,6-Dihydroxyphenyl; 2-, 3- und 4-Cyanophenyl; 3-und 4-Carboxyphenyl; 3- und 4-Carboxamidophenyl, 3- und 4-N-Methylcarboxamidophenyl und 3- und 4-N-Ethylcarboxamidophenyl; 3- und 4-Acetylaminophenyl, 3- und 4-Propionylaminophenyl und 3- und 4-Buturylaminophenyl; 3- und 4-N-Phenylaminophenyl, 3- und 4-N-(o-Tolyl)aminophenyl, 3- und 4-N-(m-Tolyl)aminophenyl und 3- und 4-(p-Tolyl)aminophenyl; 3- und 4-(2-Pyridyl)aminophenyl, 3- und 4-(3-Pyridyl)aminophenyl, 3- und 4-(4-Pyridyl)aminophenyl, 3- und 4-(2-Pyrimidyl)aminophenyl und 4-(4-Pyrimidyl)aminophenyl;
4-Phenylazophenyl, 4-(1-Naphthylazo)phenyl, 4-(2-Naphthylazo)phenyl, 4-(4-Naphthyl-, azo)phenyl,4-(2-Pyriylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;
Phenoxy, Phenylthio, 2-Naphthoxy, 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio.
Im folgenden werden die erfindungsgemäßen Rylenderivate I und die ebenfalls erfindungsgemäßen Verfahren zu ihrer Herstellung im einzelnen aufgeführt.

Die in den hierbei verwendeten Formeln auftretenden Variablen haben, soweit nicht anders angegeben, die eingangs genannte Bedeutung.

Die Carbonsäurefunktionen werden in diesen Formeln wie auch in den Ansprüchen stets in Anhydridform dargestellt und auch als Anhydrid bezeichnet Die freien Carbonsäuren oder ihre Salze sind aber ebenfalls durch die beschriebenen Vorgehensweisen erhältlich bzw. fallen dabei als Intermediate an und müssen lediglich isoliert werden.

Die Rylentetracarbonsäuremonoimidmonoanhydride Ia 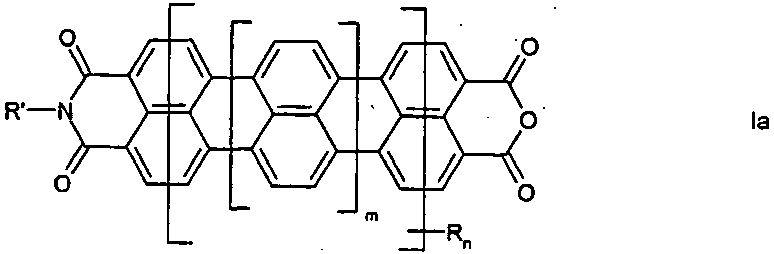 und die Rylentetracarbonsäuredianhydride Ib 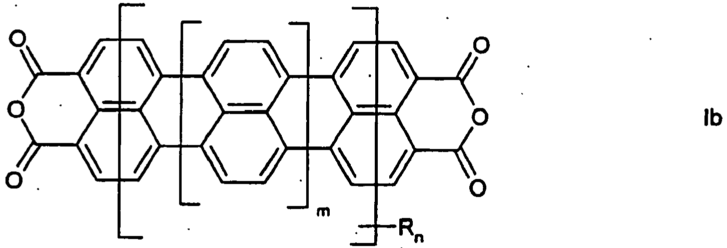 können erfindungsgemäß vorteilhaft hergestellt werden, indem man ein Rylentetracarbonsäurediimid II 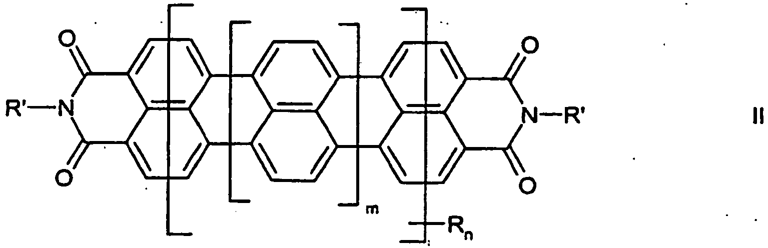 einer Verseifung unter alkalischen Bedingungen in Gegenwart eines polaren organischen Lösungsmittels unterzieht und das Rylentetracarbonsäuremonoimidmonoanhydrid Ia und das Rylentetracarbonsäuredianhydrid Ib vorzugsweise durch Säulenchromatographie isoliert und voneinander trennt.

Vorteilhaft bei dieser Vorgehensweise (Variante a) der jeweiligen Einzelverfahren) ist, daß sie auch für die Herstellung der Rylentetracarbonsäuremonoimidmonoanhydride von symmetrischen Rylentetracarbonsäuredümiden II ausgeht und die aufwendige Synthese der ansonsten erforderlichen unsymmetrischen Rylentetracarbonsäurediimidie II überflüssig macht.

Für die Verseifung als Reaktionsmedium geeignet sind polare, insbesondere protische, organische Lösungsmittel. Besonders geeignet sind aliphatische Alkohole, die 3 bis 8 Kohlenstoffatome aufweisen und unverzweigt sein können, vorzugsweise aber verzweigt sind. Beispielhaft seien neben n-Propanol und n-Butanol insbesondere Isopropanol, sec.- und tert.-Butanol und 2-Methyl-2-butanol genannt.

Selbstverständlich können auch Mischungen von Lösungsmitteln eingesetzt werden.

Im allgemeinen kommen 5 bis 500 ml, vorzugsweise 20 bis 100 ml, Lösungsmittel pro g II zum Einsatz.

Als Basen eignen sich alkalimetall- und erdalkalirnetallhaltige Basen, wobei die alkalimetallhaltigen Basen bevorzugt und die natrium- und kaliumhaltigen Basen besonders bevorzugt sind. Dabei sind sowohl anorganische Basen, vor allem die Hydroxide, wie Natriumhydroxid und Kaliumhydroxid, als auch organische Basen, vor allem die Alkoholate, wie Natriummethylat, Kaliummethylat, Kaliumisopropylat und Kalium-tert.-butylat, geeignet, die üblicherweise in wasserfreier Form eingesetzt werden. Ganz besonders bevorzugt ist Kaliumhydroxid.

Selbstverständlich können auch Mischungen von Basen verwendet werden.

In der Regel werden 10 bis 200 mol, bevorzugt 30 bis 70 mol, Base, pro mol II benötigt.

Insbesondere bei der Verseifung von Terrylentetracarbonsäurediimiden II hat es sich als vorteilhaft erwiesen, zusätzlich ein Metallfluorid, insbesondere ein Alkalimetallfluorid, z.B. Kaliumfluorid, Natriumfluorid oder Lithiumfluorid, als Hilfsmittel einzusetzen.

Geeignete Hilfsmittelmengen liegen in der Regel bei 0,1 bis 4 mol, insbesondere bei 0,5 bis 1,5 mol pro mol Base.

Die Reaktionstemperatur beträgt im allgemeinen 50 bis 120°C, vorzugsweise 60 bis 100°C.

Übliche Reaktionsdauem sind 0,5 bis 24 h, vor allem 2 bis 10 h.

Verfahrenstechnisch geht man zweckmäßigerweise wie folgt vor:
Man erhitzt eine Mischung von Base, gegebenenfalls Hilfsmittel und Lösungsmittel unter starkem Rühren auf die Reaktionstemperatur und gibt dann das Rylentetracarbonsäurediimid II zu. Nach der gewünschten Reaktionszeit tropft man so lange eine Säure, z.B. eine anorganische Säure wie Salzsäure oder vorzugsweise eine organische Säure wie Essigsäure, zu, bis ein pH-Wert von etwa 1 bis 4 erreicht ist, und rührt weitere 1 bis 4 h bei der Reaktionstemperatur. Das nach Abkühlen auf Raumtemperatur durch Verdünnen mit Wasser gefällte Reaktionsprodukt wird abfiltriert, mit heißem Wasser gewaschen und im Vakuum bei etwa 100°C getrocknet.

Wenn anstelle des jeweiligen Anhydrids das entsprechende Carbonsäuresalz isoliert werden soll, geht man zweckmäßigerweise so vor, daß man das Reaktionsgemisch nach der Verseifung nicht sauer stellt, sondern nur auf Raumtemperatur abkühlt, das ausgefallene Produkt abfiltriert, mit einem niederen aliphatischen Alkohol wie Isopropanol wäscht und bei etwa 100°C im Vakuum trocknet.

Rylentetracarbonsäuremonoimidmonoanhydrid Ia und Rylentetracarbonsäuredianhydrid Ib können durch Säulenchromatographie an Kieselgel mit Toluol oder Chloroform als Eluens isoliert und gereinigt, d.h. voneinander und von nichtverseiftem Edukt II getrennt, werden.

Die Ausbeute für das Gemisch aus Rylentetracarbonsäuremonoimidmonoanhydrid Ia und Rylentetracarbonsäuredianhydrid Ib liegt üblicherweise bei 70 bis 90%.

Durch Auswahl geeigneter Reaktionsbedingungen kann die Reaktion gewünschtenfalls auch in Richtung der einseitigen oder der beidseitigen Verseifung gelenkt werden (jeweilige Verfahrensvarianten b)).

So wird die einseitige Verseifung durch mildere Reaktionsbedingungen, wie geringere Basenmengen, niedrigere Reaktionstemperaturen und kürzere Reaktionszeiten, begünstigt, während bei schärfere Reaktionsbedingungen, wie größeren Basenmengen, Hilfsmittelzusatz, höheren Reaktionstemperaturen und längeren Reaktionszeiten, die beidseitige Verseifung überwiegt.

Rylentetracarbonsäuremonoimidmonoanhydrid Ia und Rylentetracarbonsäuredianhydrid Ib können auf diese Weise in der Regel in Ausbeuten von 30 bis 70% (für Ia) bzw. 50 bis 90% (für Ib) erhalten werden.

Bei der erfindungsgemäßen Herstellung der Rylendicarbonsäureimide Ic 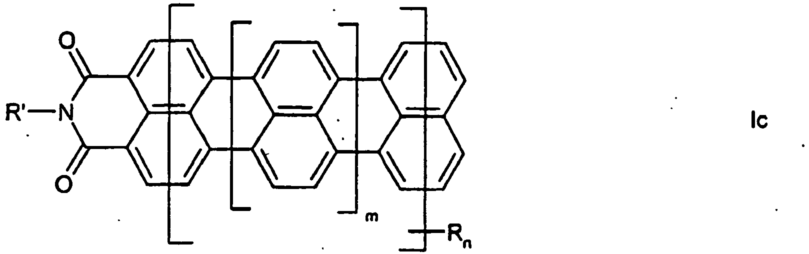 sowie der Rylene Id 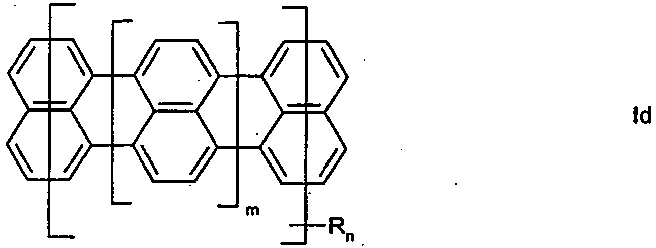 wird vorteilhaft das bei der Verseifung des Rylentetracarbonsäurediimids II anfallende Gemisch aus Rylentetracarbonsäuremonoimidmonoanhydrid Ia und Rylentetracarbonsäuredianhydrid Ib, das üblicherweise auch noch nichtverseiftes Rylentetracarbonsäurediimid II enthält, eingesetzt und einer Decarboxylierung in Gegenwart einer tertiären stickstoffbasischen Verbindung als Lösungsmittel und eines Ubergangsmetallkatalysators unterzogen (jeweilige Verfahrensvaranten b)). Die hierbei gebildeten Rylendicarbonsäureimide Ic und Rylene Id können leicht säulenchromatographisch voneinander getrennt werden.

Selbstverständlich können die Rylendicarbonsäureimide Ic und die Rylene Id entsprechend den Verfahrensvarianten a) auch aus dem jeweils isolierten Rylentetracarbonsäuremonoimidmonoanhydrid Ia bzw. Rylentetracarbonsäuredianhydrid Ib hergestellt werden.

Als Reäktionsmedium für die Decarboxylierung sind tertiäre stickstoffbasische Verbindungen geeignet, deren Siedepunkt vorzugsweise oberhalb der Reaktionstemperatur liegt. Beispiele für besonders geeignete Lösungsmittel sind N,N-disubstituierte aliphatische Carbonsäureamide (insbesondere N,N-Di-C₁-C₄-alkyl-C₁-C₄-carbonsäureamide) und stickstoffhaltige Heterocyclen.

Im einzelnen seien beispielhaft genannt: Dimethylformamid, Diethylformamid, Dimethylacetamid, Dimethylbutyramid, N-Methylpyrrolidon, 3-Methylpyridin, Chinolin, Isochinolin und Chinaldin, wobei Chinolin bevorzugt ist.

Selbstverständlich können auch Lösungsmittelmischungen verwendet werden.

Im allgemeinen kommen 5 bis 200 ml, insbesondere 10 bis 70 ml, Lösungsmittel pro g zu decarboxylierendes Edukt zum Einsatz.

Als Katalysatoren eignen sich vor allem die Übergangsmetalle Kupfer und Zink und ihre Verbindungen, insbesondere ihre Oxide und ihre anorganischen und organischen Salze, die vorzugsweise in wasserfreier Form eingesetzt werden.

Beispiele für bevorzugte Katalysatoren sind Kupfer, Kupfer(I)oxid, Kupfer(II)oxid, Kupfer(I)chlorid, Kupfer(II)acetat, Zinkacetat und Zinkpropionat, wobei Kupfer(I)oxid und Zinkacetat besonders bevorzugt sind.

Selbstverständlich kann man auch Mischungen der genannten Katalysatoren verwenden.

In der Regel werden 0,5 bis 2 mol, vorzugsweise 0,9 bis 1,2 mol, Katalysator pro mol zu decarboxylierendes Edukt eingesetzt.

Die Reaktionstemperatur beträgt üblicherweise 100 bis 250°C, vorzugsweise 160 bis 220°C.

Es empfiehlt sich, unter Schutzgas, z.B. Stickstoff oder Argon, zu arbeiten.

Die Decarboxylierung ist in der Regel in 0,5 bis 24 h, insbesondere 1 bis 5 h, abgeschlossen.

Verfahrenstechnisch geht man zweckmäßigerweise wie folgt vor:

Man erhitzt eine Mischung von zu decarboxylierendem Edukt, Lösungsmittel und Katalysator unter Rühren unter Schutzgas auf die gewünschte Reaktionstemperatur. Nach der gewünschten Reaktionszeit und Abkühlen auf Raumtemperatur fällt man das Reaktionsprodukt auf eine wäßrige Säure, insbesondere verdünnte Salzsäure, und rührt das Gemisch gewünschtenfalls etwa 1 h bei 60°C. Das Reaktionsprodukt wird abfiltriert, mit heißem Wasser gewaschen und im Vakuum bei etwa 100°C getrocknet.

Rylendicarbonsäureimid Ic und Rylen Id können durch Säulenchromatographie an Kieselgel mit Toluol als Eluens isoliert und gereinigt, d.h. voneinander und von nichtumgesetztem Edukt getrennt, werden.

Die Ausbeute für das Gemisch von Rylendicarbonsäureimid Ic und Rylen Id beträgt, ausgehend vom Rylentetracarbonsäurediimid II, üblicherweise 65 bis 85%.

Auch bei der Herstellung der peri-halogenierten Rylendicarbonsäureimide le 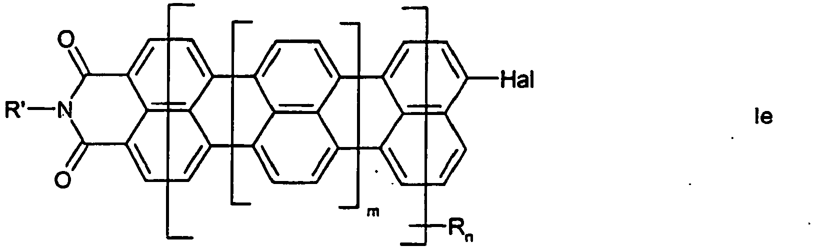 (Hal: Halogen, bevorzugt Chlor, Brom oder Iod, besonders bevorzugt Chlor oder Brom, ganz besonders bevorzugt Brom)
und der halogenierten Rylene If 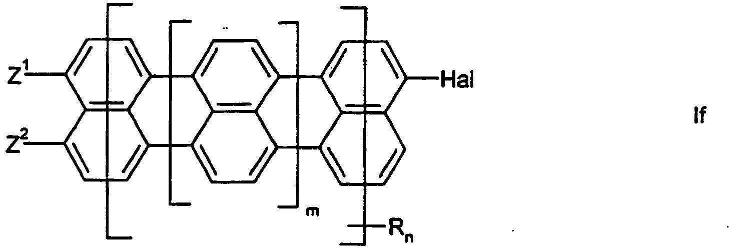 (Hal: Halogen, bevorzugt Chlor, Brom oder Iod, besonders bevorzugt Chlor oder Brom, ganz besonders bevorzugt Brom; Z¹, Z²: Wasserstoff oder einer der Reste Z¹ oder Z² Halogen, bevorzugt Chlor, Brom oder Iod, besonders bevorzugt Chlor oder Brom, ganz besonders bevorzugt Brom, und der andere Rest Wasserstoff)
kann man gemäß den erfindungsgemäßen Verfahrensvarianten b) vorteilhaft das bei der vorstehend beschriebenen Decarboxylierung anfallende Gemisch von Rylendicarbonsäureimid Ic und Rylen Id, das üblicherweise auch noch nichtverseiftes Rylentetracarbonsäurediimid II enthält, einsetzen, das wiederum auf dem bei der Verseifung des Rylentetracarbonsäurediimids II anfallenden Gemisch von Rylentetracarbonsäuremonoimidmonoanhydrid Ia und Rylentetracarbonsäuredianhydrid Ib basiert. Die erhaltenen halogenierten Rylenderivate le und lf lassen sich einfach durch Säulenchromatographie trennen.

Selbstverständlich können die halogenierten Rylenderivate Ie und If auch durch Halogenierung der isolierten Einzelverbindungen Ic bzw. Id hergestellt werden (Verfahrensvarianten a)).

Die erfindungsgemäße Halogenierung wird in Gegenwart eines polaren organischen Lösungsmittels und einer Lewissäure als Katalysator mit N-Halogensuccinimid vorgenommen.

Diese Vorgehensweise ist anwendbar, um die chlorierten, bromierten oder iodierten Rylenderivate Ie und If herzustellen, wobei die chlorierten Produkte bevorzugt und die bromierten Produkte besonders bevorzugte sind.

Die Rylendicarbonsäureimide Ic werden regioselektiv in peri-Position rnonohalogeniert, die Rylenderivate Id können mono- und dihalogeniert werden.

Bei der Dihalogenierung der Rylenderivate Id besteht sowohl bei Verfahrensvariante a) als auch bei Verfahrensvariante b) die Möglichkeit, die Halogenierung in einem Schritt unter Zugabe der insgesamt erforderlichen Menge an N-Halogensuccinimid vorzunehmen (Verfahrensvarianten a1) bzw. b1)).

In der Regel wird jedoch eine stufenweise Halogenierung gemäß den Verfahrensvarianten a2) bzw. b2) vorzuziehen sein, bei der in einem ersten Schritt das monohalogenierte Rylen lf (Z¹=Z²=H) und in einem zweiten Schritt, vorzugsweise nach Zwischenisolierung des Monohalogenrylens, das dihalogenierte Rylen If (Z¹ oder Z²=Halogen) hergestellt wird. Die zweistufige Vorgehensweise ermöglicht zudem durch Einsatz verschiedener N-Halogensuccinimide die gezielte Herstellung von gemischthalogenierten Rylenen If, die auf der einen Molekülseite (in 3-Position) durch das eine Halogen und auf der anderen Molektilseite (entsprechend in der in der 13- oder 14-Position beim Quaterrylen) durch das andere Halogen substituiert sind. In der Regel werden bei der Dihalogenierung immer Gemische der der 3,13- und 3,14-Dihalogenquaterrylene anfallen.

Als polare organische Lösungsmittel eignen sich für die Halogenierung insbesondere aprotische Lösungsmittel. Bevorzugte Beispiele für diese Lösungsmittel sind die vorstehend genannten aliphatischen Carbonsäureamide, wie Dimethylformamid und Dimethylacetamid, und halogenierte Kohlenwasserstoffe, wie Chloroform und Methylenchlorid. Besonders bevorzugt ist Dimethylformamid.

In der Regel kommen 25 bis 200 ml, vorzugsweise 50 bis 150 ml, Lösungsmittel pro g zu halogenierendes Edukt zum Einsatz.

Als Lewis-Säure-Katalysatoren eignen sich vor allem Metallhalogenide, wobei Eisen(III)halogenide, Aluminiumtrihalogenide und Zinkhalogenide bevorzugt sind. Als Beispiele im einzelnen genannt-seien Eisen(III)chlorid, Eisen(III)bromid, Eisen(III)iodid, Aluminiumtrichlorid, Aluminiumtribromid, Aluminiumtriiodid und Zinkchlorid, wobei die Eisenhalogenide besonders bevorzugt sind.

Im allgemeinen werden 0,01 bis 0,5 mol, vorzugsweise 0,05 bis 0,2 mol, Lewis-Säure pro mol zu halogenierendes Edukt, eingesetzt.

Die Menge an N-Halogensuccinimid hängt von dem gewünschten Halogenierungsgrad ab. Üblicherweise werden pro einzuführendes Halogenatom 1 bis 6 mol, vor allem 1 bis 4 mol, N-Halogensuccinimid benötigt. Werden Gemische von Rylendicarbonsäureimid Ic und Rylen Id eingesetzt, so ist zu berücksichtigen, daß beide Edukte zu halogenieren sind. Sollen die Dihalogenrylene If stufenweise hergestellt werden, so werden im ersten Schritt zweckmäßigerweise 1 bis 3 mol N-Halogensuccinimid pro mol Rylen Id bzw. pro mol Rylen Id und Rylendicarbonsäureimid Ic bei Einsatz eines Eduktgemischs und im zweiten Schritt in der Regel weitere 1 bis 6 mol, insbesondere 1 bis 4 mol, pro mol monohalogeniertes Rylen If eingesetzt.

Die Halogenierungstemperatur liegt im allgemeinen bei 20 bis 100°C, bevorzugt bei 40 bis 80°C.

Es empfiehlt sich, unter Schutzgas, z.B. Stickstoff oder Argon, zu arbeiten.

Übliche Reaktionsdauem betragen 0,5 bis 24 h, vor allem 1 bis 2 h.

Verfahrenstechnisch geht man zweckmäßigerweise wie folgt vor:

Man erhitzt eine Mischung von zu halogenierendem Edukt, Lewis-Säure, N-Halogensuccinimid und Lösungsmittel unter Rühren unter Schutzgas auf die gewünschte Reaktionstemperatur. Nach beendeter Halogenierung und Abkühlen auf Raumtemperatur fällt man das Reaktionsprodukt mit verdünnter anorganischer Säure, z.B. mit verdünnter Salzsäure. Das Reaktionsprodukt wird abfiltriert, mit heißem Wasser gewaschen und im Vakuum bei etwa 100°C getrocknet.

Das halogenierte Rylendicarbonsäureimid Ie und Rylen If können durch Säulenchromatographie an Kieselgel mit Toluol als Eluens isoliert und gereinigt, d.h. voneinander und von nichtumgesetztem Edukt getrennt, werden.

Bei zweistufiger Herstellung von Dihalogenrylenen If kann das auf diese Weise isolierte Monohalogenrylen If einer weiteren wie oben beschriebenen Halogenierung unterzogen werden.

Die Ausbeuten für das peri-halogenierte Rylendicarbonsäureimid Ie und das halogenierte Rylen If betragen, ausgehend vom Rytentetracarbonsäurediimid II, üblicherweise jeweils 25 bis 40%.

Die nichthalogenierten oder peri-halogenierten Rylendicarbonsäureanhydride Igh 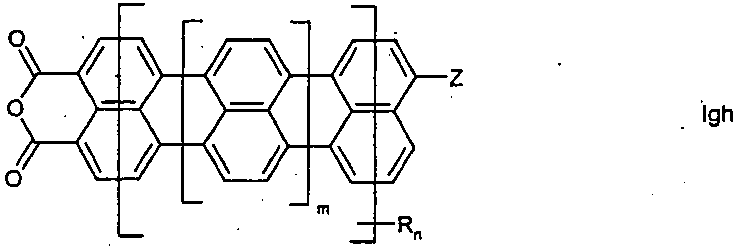 (Z: Wasserstoff oder Halogen, wobei als Halogen Chlor, Brom oder Iod bevorzugt und Chlor oder Brom besonders bevorzugt sind und Brom ganz besonders bevorzugt ist)
sind erfindungsgemäß durch Verseifung von nichthalogenierten oder peri-halogenierten Rylendicarbonsäureimiden der allgemeinen Formel Ice 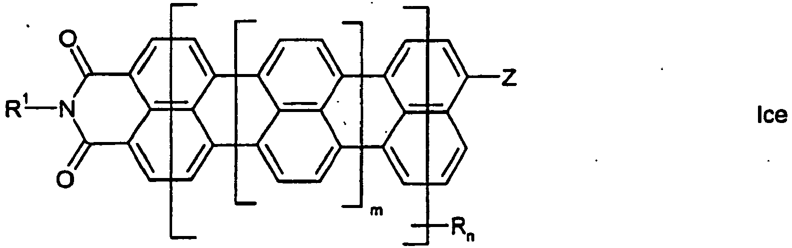 erhältlich.

Die Verseifung kann analog der oben beschriebenen Verseifung der Rylentetracarbonsäurediimide II unter alkalischen Bedingungen in Gegenwart eines polaren organischen Lösungsmittels vorgenommen werden.

Auch hier empfiehlt sich insbesondere bei der Verseifung der Tenylendicarbonsäureimide Ice die Anwesenheit eines Fluorids, vor allem von Kaliumfluorid, das in der Regel in Mengen von 0,1 bis 2 Äquivalenten, vorzugsweise 0,7 bis 1,3 Äquivalenten, bezogen auf die Base, eingesetzt wird.

Die weiteren Reaktionsbedingungen sowie die verfahrenstechnische Vorgehensweise entsprechen dem oben beschriebenen Verseifungsverfahren.

Die Rylendicarbonsäuresalze bzw. die freie Säure können, wie bei der Verseifung des Rylentetracarbonsäurediimids II beschrieben, isoliert bzw. hergestellt werden.

Gewünschtenfalls kann das Rylendicarbonsäureanhydrid Igh einer Reinigung durch Säulenchromatographie mit Chloroform als Eluens unterzogen werden, im allgemeinen wird dies jedoch nicht erforderlich sein.

Die Ausbeute liegt üblicherweise bei 70 bis 90%, bezogen auf das eingesetzte Rylendicarbonsäureimid Ice.

Selbstverständlich können die peri-halogenierten Rylendicarbonsäureanhydride Ih 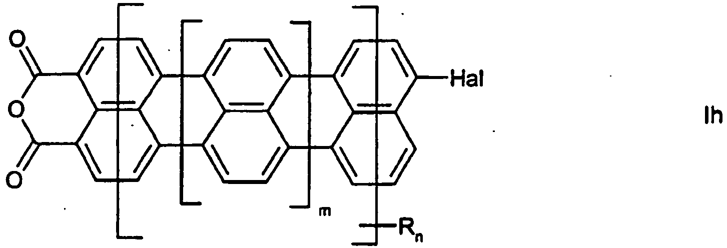 (Hal: Halogen, bevorzugt Chlor, Brom oder Iod, besonders bevorzugt Chlor oder Brom, ganz besonders bevorzugt Brom)
erfindungsgemäß auch durch Halogenierung des entsprechenden Rylendicarbonsäureanhydrids Ig 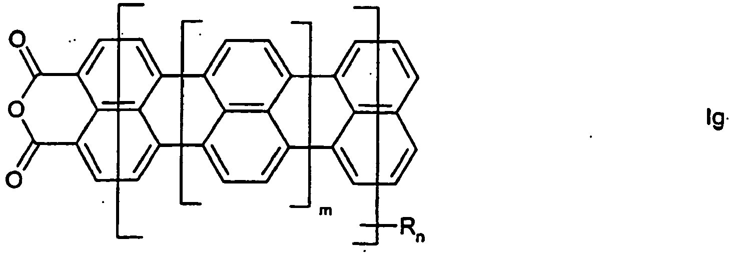 hergestellt werden.

Die Halogenierung kann vorteilhaft mit N-Halogensuccinimid in Gegenwart eines polaren organischen Lösungsmittels und einer Lewis-Säure als Katalysator analog zu der oben beschriebenen Halogenierung der Rylendicarbonsäureimide Ic und der Rylene Id vorgenommen werden.

Die peri-(Dioxaborolan-2-yl)rylendicarbonsäureimide Ii 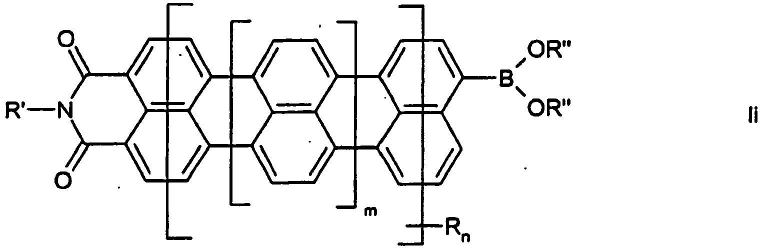 und die peri-(Dioxaborolan-2-yl)rylendicarbonsäureanhydride lk 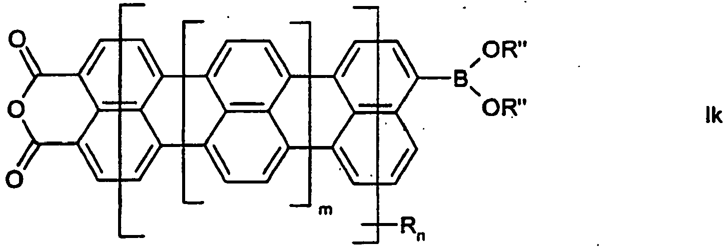 sind erfindungsgemäß durch Umsetzung des entsprechenden peri-halogenierten Rylendicarbonsäureimids le 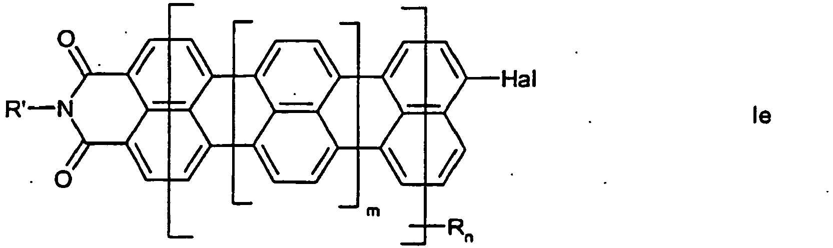 (Hal: Halogen, bevorzugt Chlor, Brom oder Iod, besonders bevorzugt Chlor oder Brom, ganz besonders bevorzugt Brom)
bzw. des peri-halogenierten Rylendicarbonsäureanhydrids Ih 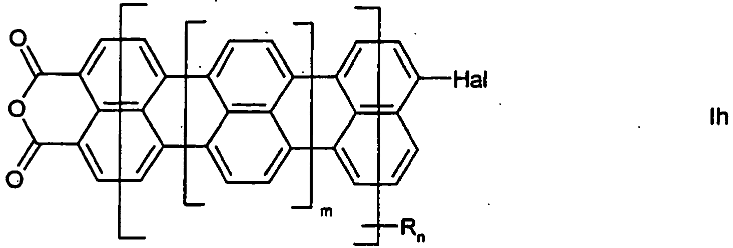 (Hal: Halogen, insbesondere bevorzugt Chlor, Brom oder Iod, besonders bevorzugt Chlor oder Brom, ganz besonders bevorzugt Brom)
mit einem Diboran der allgemeinen Formel III 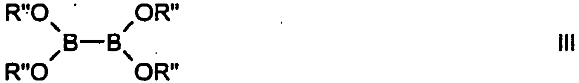 in Gegenwart eines aprotischen organischen Lösungsmittels, eines Übergangsmetallkatalysators und einer Base erhältlich.

Die (dioxaborolan-2-yl)substituierten Rylene Ij 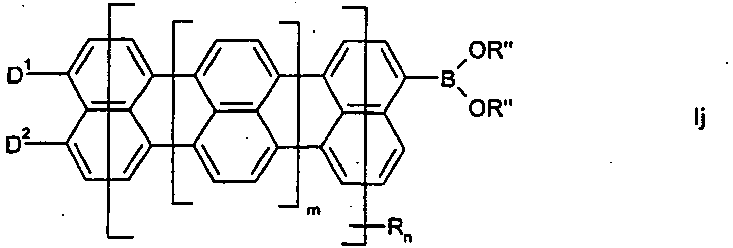 können erfindungsgemäß analog aus den Halogenrylenen If 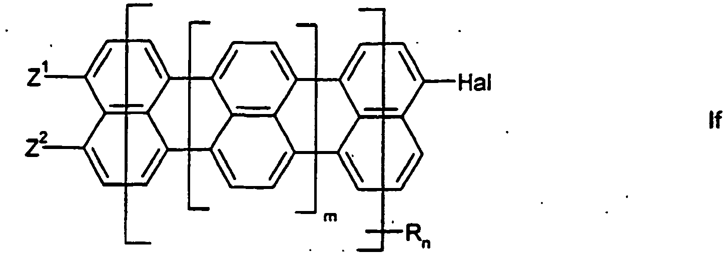 hergestellt werden.

Zur Herstellung der Mono(dioxaborolan-2yl)rylene Ij (D¹=D²=H) werden dabei Monohalogenrylene If (Z¹=Z²=H) eingesetzt.

Bis(dioxaborolan-2yl)rylene lj (D¹ oder D²=Rest (d)) 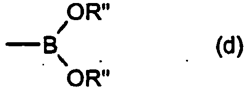 sind entsprechend aus den Dihalogenrylenen If (Z¹ oder Z²=Halogen) erhältlich.

Durch Austausch nur eines der im Dihalogenrylen If enthaltenen Halogenatome sind schließlich auch gemischtsubstituierte Rylene Ij (D¹ oder D²=Halogen) zugänglich.

Da es sich bei den Dihalogenrylenen If in der Regel um die vorstehend beschriebenen Isomerengemische handelt, fallen auch die aus ihnen hergestellten (dioxaborolan-2-yl)-substituierten Rylene Ij als Isomerengemische an.

In der Regel werden zur Einführung eines (Dioxaborolan-2-yl)restes in das Rylenderivat le, Ih bzw. If 1 bis 3 mol, vorzugsweise 1 bis 2 mol, Diboran III pro mole Rylenderivat eingesetzt.

Soll nur eines der in den Dihalogenrylenen If enthaltenen Halogenatome durch einen (Dioxaborolan-2-yl)rest ersetzt werden, so empfiehlt es sich, die Menge an Diboran III geringfügig zu erniedrigen und etwa 1 bis 1,5 mol Diboran III pro mol Rylenderivat If einzusetzen, um eine doppelte Substitution zu vermeiden.

Zur Herstellung der Bis(dioxaborolan-2-yl)rylene Ij wird dementsprechend üblicherweise die doppelte Menge an Diboran III benötigt.

Als Diborane III eignen sich insbesondere Bis(1,2- und 1,3-diolato)diborane, Tetraalkoxydiborane, Tetracycloalkoxydiborane, Tetraaryloxydiborane und Tetrahetaryloxydiborane sowie deren Mischformen. Als Beispiele für diese Verbindungen seien genannt Bis(pinacolato)diboran, Bis(1,2-benzoldiolato)diboran, Bis(2,2-dimethyl-1,3-propandiolato)diboran, Bis(1,1,3,3-tetramethyl-1,3-propandiolato)diboran, Bis(4,5-pinandiolato)diboran, Bis(tetramethoxy)diboran, Bis(tetracyclopentoxy)diboran, Bis(tetraphenoxy)diboran und Bis(4-pyridlyloxy)liboran.

Bevorzugt sind Diborane III, bei denen die beiden an einem Boratom befindlichen Reste R" unter Ausbildung eines die beiden Sauerstoffatome sowie das Boratom enthaltenden Fünf- oder Sechsrings miteinander verbunden sind. An die gebildeten Fünfringe können aromatische oder gesättigte, auch bicyclische, Ringe, mit 5 bis 7 Atomen als Ringgliedern anneliert sein. Alle Ringe bzw. Ringsysteme können durch bis zu 4 C₁-C₃₀-Alkyl-, C₅-C₈-Cycloalkyl-, Aryl- und/oder Hetarylreste substituiert sein, vorzugsweise sind sie durch bis zu 4 C₁-C₄-Alkylreste substituiert. Beispiele für diese bevorzugten Diborane sind die bereits oben genannten Bis(1,2- und 1,3-diolato)diborane, wobei Bis(pinacolato)diboran besonders bevorzugt ist.

Als Lösungsmittel sind für diese Umsetzung grundsätzlich alle unter den Reaktionsbedingungen gegen Basen stabilen aprotischen Lösungsmittel mit einem Siedepunkt oberhalb der gewählten Reaktionstemperatur geeignet, in denen sich die halogenierten Edukte Ie, If bzw. Ih bei Reaktionstemperatur vollständig und die verwendeten Katalysatoren und Basen zumindest partiell lösen, so daß weitgehend homogene Reaktionsbedingungen vorliegen. Es können sowohl unpolar-aprotische als auch polar-aprotische Lösungsmittel eingesetzt werden, wobei die unpolar-aprotischen Lösungsmittel bevorzugt sind.

Beispiele für bevorzugte unpolar-aprotische Lösungsmittel sind bei > 100°C siedende Lösungsmittel aus den folgenden Gruppen: Aliphaten (insbesondere C₈-C₁₈-Alkane), unsubstituierte, alkylsubstituierte und kondensierte Cycloaliphaten (insbesondere unsubstituierte C₇-C₁₀-Cycloalkane, C₆-C₈-Cycloalkane, die durch ein bis drei C₁-C₆-Alkylgruppen substituiert sind, polycyclische gesättigte Kohlenwasserstoffe mit 10 bis 18 C-Atomen), alkyl- und cycloalkylsubstituierte Aromaten (insbesondere Benzol, das durch ein bis drei C₁-C₆-Alkylgruppen oder einen C₅-C₈-Cycloalkylrest substituiert ist) und kondensierte Aromaten, die alkylsubstituiert und/oder teilhydriert sein können (insbesondere Naphthalin, das durch ein bis vier C₁-C₆-Alkylgruppen substituiert ist) sowie Mischungen dieser Lösungsmittel.

Als Beispiele für besonders bevorzugte Lösungsmittel seien im einzelnen genannt: Octan, Isooctan, Nonan, Isononan, Decan, Isodecan, Undecan, Dodecan, Hexadecan und Octadecan; Cycloheptan, Cyclooctan, Methylcyclohexan, Dimethylcyclohexan, Trimethylcyclohexan, Ethylcyclohexan, Diethylcyclohexan, Propylcyclohexan, Isopropylcyclohexan, Dipropylcyclohexan, Butylcyclohexan, tert.-Butylcyclohexan, Methylcycloheptan und Methylcyclooctan; Toluol, o-, m- und p-Xylol, 1,3,5-Trimethylbenzol (Mesitylen), 1,2,4- und 1,2,3-Trimethylbenzol, Ethylbenzol, Propylbenzol, Isopropylbenzol, Butylbenzol, Isobutylbenzol, tert.-Butylbenzol und Cyclohexylbenzol; Naphthalin, Decahydronaphthalin (Dekalin), 1- und 2-Methylnaphthalin und 1- und 2-Ethylnaphthalin; Kombinationen aus den zuvor genannten Lösungsmitteln, wie sie aus den hochsiedenden, teil- oder durchhydrierten Fraktionen thermischer und katalytischer Crackprozesse bei der Rohöl- oder Naphthaverarbeitung gewonnen werden können, z.B. Gemische vom Exxsol^{®} Typ und Alkylbenzolgemische vom Solvesso^{®} Typ.

Ganz besonders bevorzugte Lösungsmittel sind Xylol (alle Isomeren), Mesitylen und vor allem Toluol.

Beispiele für geeignete polar-aprotische Lösungsmittel sind N,N-disubstituierte aliphatische Carbonsäureamide (insbesondere N,N-Di-C₁-C₄-alkyl-C₁-C₄-carbonsäureamide) und stickstoffhaltige Heterocyclen, die oben bereits aufgeführt wurden, sowie aprotische Ether (insbesondere cyclische Ether, Diarylether und Di-C₁-C₆-alkylether von monomeren und oligomeren C₂-C₃-Alkylenglykolen, die bis zu 6 Alkylenoxideinheiten enthalten können, vor allem Diethylenglykoldi-C₁-C₄-alkylether), wie:

Tetrahydrofuran, Dioxan, Diphenylether, Diethylenglykoldimethyl-, -diethyl-, -dipropyl-, -diisopropyl-, -di-n-butyl-, -di-sec.-butyl- und -di-tert.-butylether, Diethylenglykolmethylethylether, Triethylenglykoldimethyl- und -diethylether und Triethylenglykolmethylethylether.

Im Fall der Rylene Id sind die polar-aprotischen Lösungsmittel, insbesondere Dioxan und Dimethylformamid, besonders bevorzugt, für die anderen zu halogenierenden Edukte sind die unpolar-aprotischen Lösungsmittel, vor allem Toluol, besonders bevorzugt.

Die Lösungsmittelmenge beträgt in der Regel 10 bis 1000 ml, bevorzugt 20 bis 300 ml, pro g halogeniertes Edukt.

Als Obergangsmetallkatalysator eignen sich insbesondere Palladiumkomplexe, wie Tetrakis(triphenylphosphin)palladium(0), Tetrakis(tris-o-tolylphosphin)palladium(0), [1,2-Bis(diphenylphosphino)ethan]palladium(II)chlorid, [1,1'-Bis(diphenylphosphino)-ferrocen]palladium(II)chlorid, Bis(triethylphosphin)palladium(II)chlorid, Bis(tricyclohexylphosphin)palladium(II)acetat, (2,2'-Bipyridyl)palladium(II)chlorid, Bis(triphenylphosphin)palladium(II)chlorid, Tris(dibenzylidenaceton)dipalladium(0), 1,5-Cyclooctadienpalladium(II)chlorid, Bis(acetonitril)palladium(II)chlorid und Bis(benzonitril)palladium(II)chlorid, wobei [1,1'-Bis(diphenylphosphino)ferrocen]palladium(II)chlorid und Tetrakis(triphenylphosphin)palladium(0) bevorzugt sind.

Üblicherweise wird der Übergangsmetallkatalysator in einer Menge von 1 bis 20 mol-%, vor allem 2 bis 10 mol-%, bezogen auf das halogenierte Edukt, eingesetzt.

Als Base kommen vorzugsweise die Alkalimetallsalze, insbesondere die Natrium- und vor allem die Kaliumsalze, schwacher organischer und anorganischer Säuren, wie Natriumacetat, Kaliumacetat, Natriumcarbonat, Natriumhydrogencarbonat, Kaliumcarbonat und Kaliumhydrogencarbonät, zum Einsatz. Bevorzugte Basen sind die Acetate, vor allem Kaliumacetat.

Im allgemeinen werden 1 bis 5 mol, bevorzugt 2 bis 4 mol, Base pro mol halogeniertes Edukt verwendet.

Die Reaktionstemperatur liegt üblicherweise bei 20 bis 180°C, vor allem bei 60 bis 120°C.

Es empfiehlt sich, unter Schutzgas, z.B. Stickstoff oder Argon, zu arbeiten.

Die Reaktionszeit beträgt in der Regel 0,5 bis 30 h, insbesondere 1 bis 20 h.

Verfahrenstechnisch geht man zweckmäßigerweise wie folgt vor

Man legt halogeniertes Edukt und Lösungsmittel vor, gibt Diboran III, den Übergangsmetallkatalysator und die Base nacheinander zu und erhitzt die Mischung 0,5 bis 30 h unter Schutzgas auf die gewünschte Reaktionstemperatur. Nach Abkühlen auf Raumtemperatur filtriert man die festen Bestandteile aus dem Reaktionsgemisch ab und destilliert das Lösungsmittel unter vermindertem Druck ab.

Das Rylenderivat Ik fällt hierbei nicht in Anhydridform an, sondern liegt in der Regel als Rylendicarbonsäuresalz vor. Das Anhydrid selbst kann auf einfache Weise durch Zugabe einer Säure erhalten werden. Hierdurch wird ein als Ester vorliegender Dioxaborolan-2-ylrest (R"≠H) zur Boronsäure hydrolysiert (R"=H):

Gewünschtenfalls können die (dioxaborolan-2-yl)substituierten Rylenderivate Ii, Ij und Ik einer Reinigung durch Säulenchromatographie mit einem 2:1-Gemisch von Chloroform und Hexan als Eluens unterzogen werden, im allgemeinen wird dies jedoch nicht erfordedich sein.

Die Ausbeute liegt üblicherweise bei 80 bis 100%.

Die Anhydridfunktionen (bzw. Säurefunktionen) aufweisenden Rylenderivate I können mit aromatischen Diaminen IV kondensiert werden.

Die ausgehend von den Rylentetracarbonsäuredianhydriden Ib erhältlichen doppelten Kondensationsprodukte fallen dabei in der Regel in Form von Mischungen des cis-Isomers Im_{cis} und des trans-Isomers Imₜᵣₐₙₛ 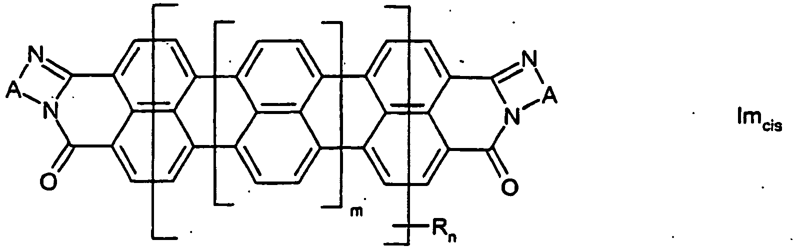 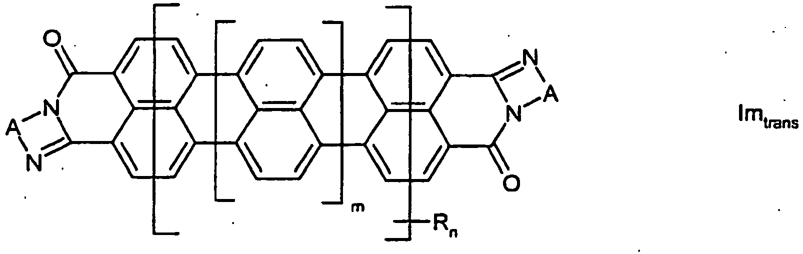 an.

Durch stufenweise Umsetzung mit zwei unterschiedlichen Diaminen IV und IV' sind auch die entsprechenden unsymmetrischen Kondensationsprodukte Im'_{cis} und Im'ₜᵣₐₙₛ. 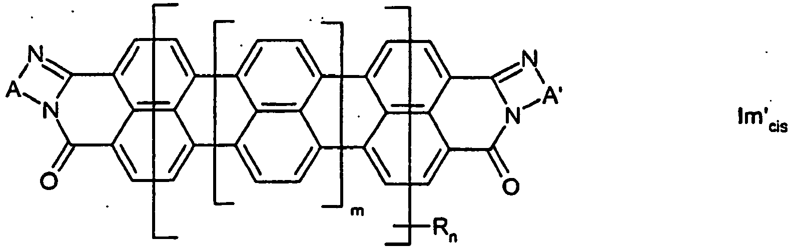 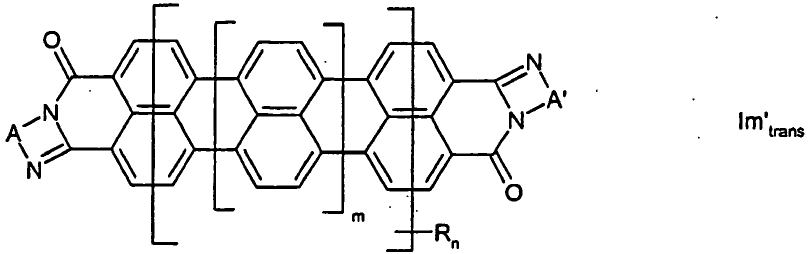 zu erhalten.

Die Rylentetracarbonsäuredianhydride Ib können jedoch auch nur einer einseitigen Kondensationsreaktion mit dem aromatischen Diamin IV unterzogen werden, wodurch die Rylentetracarbonsäurederivate In 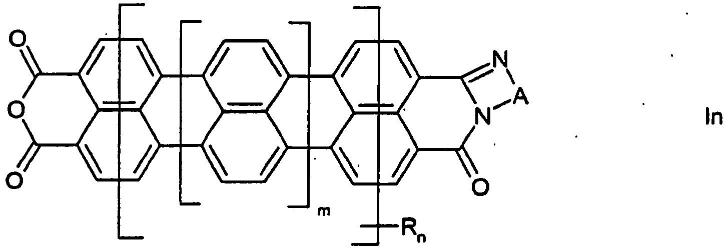 zu erhalten sind.

Die Rylentetracarbonsäurederivate Io 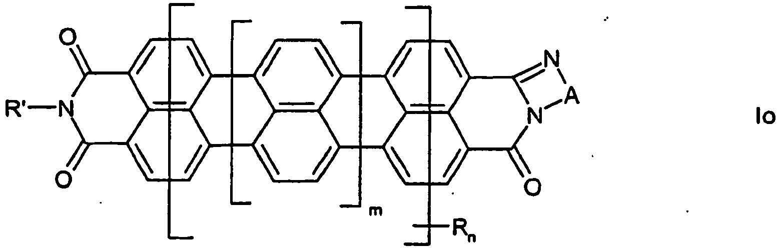 sind durch entsprechende Umsetzung der Rylentetracarbonsäuremonoimidmonoanhydride Ia herzustellen.

Schließlich können die Rylendicarbonsäurederivate Ip 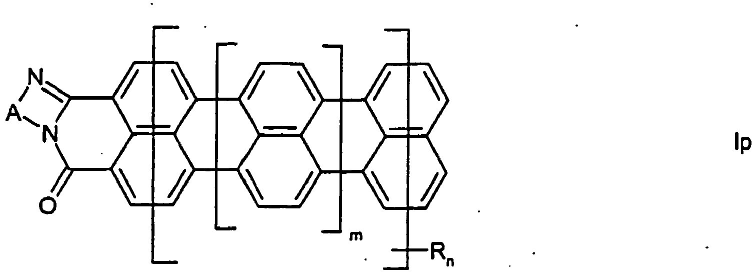 durch Kondensation der Rylendicarbonsäureanhydride Ig mit den aromatischen Diaminen IV hergestellt werden.

Die Kondensation der Anhydridfunktionen aufweisenden Rylenderivate I mit den aromatischen Diaminen IV wird erfindungsgemäß in Gegenwart einer stickstoffbasischen Verbindung oder von Phenol als Lösungsmittel und einer Lewis-Säure oder von Piperazin als Katalysator durchgeführt.

Zur Herstellung der Monokondensationsprodukte werden üblicherweise 1 bis 1,5 mol, bevorzugt 1,05 bis 1,2 mol, aromatisches Diamin IV pro mole Anhydridedukt eingesetzt. Zur Herstellung der symmetrischen Dikondensationsprodukte Im werden entsprechend im allgemeinen 2 bis 3 mol, insbesondere 2,1 bis 2,4 mol, Diamin IV verwendet.

Sollen die unsymmetrischen Dikondensationsprodukte Im' hergesteAt werden, so empfiehtt es sich, die Rylentetracarbonsäuredianhydride Ib zunächst mit nur 1 bis 1,5 mol, vor allem 1 bis 1,2 mol, des Diamins IV und dann mit 1 bis 1,5 mol, insbesondere 1 bis 1,2 mol, des Diamins IV' umzusetzen.

Als aromatische Diamine IV eignen sich o-Phenylendiamin, 1,8-Diaminonaphthalin und 3,4-Diaminopyridin. Die Diamine können durch C₁-C₁₂-Alkyl, C₁-C₆-Alkoxy, Hydroxy, Nitro und/oder Halogen substituiert sein, sind jedoch vorzugsweise unsubstituiert. Bevorzugte Diamine IV sind o-Phenylendiamin und 1,8-Diaminonaphthalin.

Als stickstoffbasische Verbindung eignen sich vor allem stickstoffhaltige Heterocyclen, die vorzugsweise nicht weiter funktionalisiert sind, wie Chinolin, Isochinolin, Chinaldin, Pyrimidin, N-Methylpiperidin, Pyridin, Pyrrol, Pyrazol, Triazol, Tetrazol, Imidazol und Methylimidazol. Bevorzugt sind tertiäre stickstoffbasische Verbindungen; vor allem Chinolin.

In der Regel kommen 5 bis 200 ml, vorzugsweise 10 bis 50 ml, Lösungsmittel pro g Rylenderivat zum Einsatz.

Als Katalysator eignen sich Lewis-Säuren, z.B. Zinkverbindungen, vor allem Zinksalze, wie Zinkacetat und Zinkchlorid, und Zinkoxid, anorganische und organische Säuren, wie Salzsäure, Essigsäure und p-Toluolsulfonsäure, wobei Zinkacetat bevorzugt ist.

Ebenfalls als Katalysator geeignet ist Piperazin, das vorzugsweise in Kombination mit Phenol als Lösungsmittel zum Einsatz kommt.

Üblicherweise werden 0,25 bis 5,0 mol, insbesondere 1,0 bis 2,0 mol, Katalysator pro umzusetzende Anhydridgruppe eingesetzt.

Die Reaktionstemperatur beträgt im allgemeinen 100 bis 240°C, vorzugsweise 160 bis 240°C.

Es empfiehlt sich, unter Schutzgas, z.B. Stickstoff oder Argon, zu arbeiten.

Im allgemeinen ist die Kondensation in 0,5 bis 24 h, vor allem 2 bis 6 h, beendet.

Verfahrenstechnisch geht man zweckmäßigerweise wie folgt vor:

Man erhitzt eine Mischung von Anhydridedukt, Katalysator, Diamin und Lösungsmittel unter Rühren unter Schutzgas auf die gewünschte Reaktionstemperatur. Nach beendeter Kondensation und Abkühlen auf Raumtemperatur wird das Reaktionsprodukt mit verdünnter Salzsäure ausgefällt, abfiltriert, mit heißem Wasser gewaschen und im Vakuum bei etwa 100°C getrocknet.

Gewünschtenfalls können die erhaltenen Kondensationsprodukte einer Reinigung durch Säulenchromatographie mit Chloroform als Eluens unterzogen werden, im allgemeinen wird dies jedoch nicht erforderlich sein.

Die Ausbeute liegt üblicherweise bei 90 bis 95%.

Die Rylendicarbonsäurederivate Ip 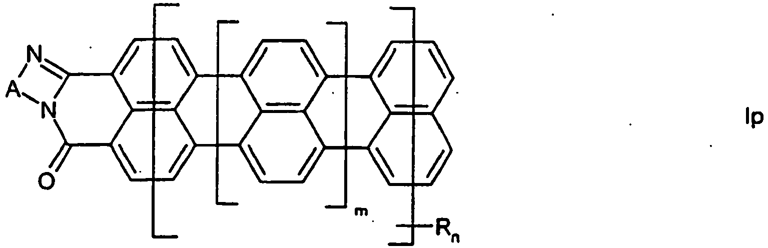 sind erfindungsgemäß außerdem durch Decarboxylierung der Rylentetracarbonsäurederivate In 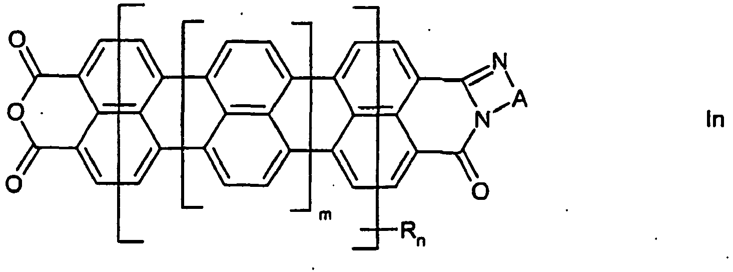 zugänglich.

Die Decarboxylierung wird vorteilhaft in Gegenwart einer tertiären stickstoffbasischen Verbindung als Lösungsmittel und eines Übergangsmetallkatalysators analog zu der oben beschriebenen Decarboxylierung von Rylentetracarbonsäuremonoimidmonoanhydrid Ia und Rylentetracarbonsäuredianhydrid Ib vorgenommen.

Die peri-halogenierten Rylendicarbonsäurederivate Iq 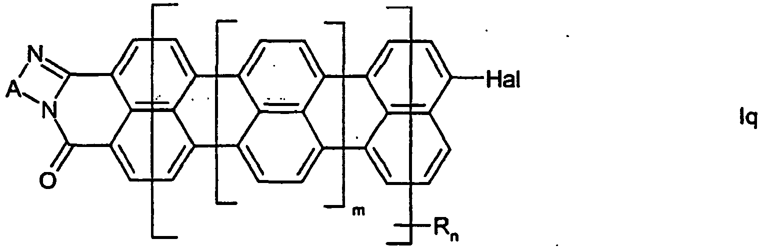 (Hal: Halogen, bevorzugt Chlor, Brom oder Iod, besonders bevorzugt Chlor oder Brom, ganz besonders bevorzugt Brom)
sind erfindungsgemäß schließlich durch Umsetzung der Rylendicarbonsäurederivate Ip 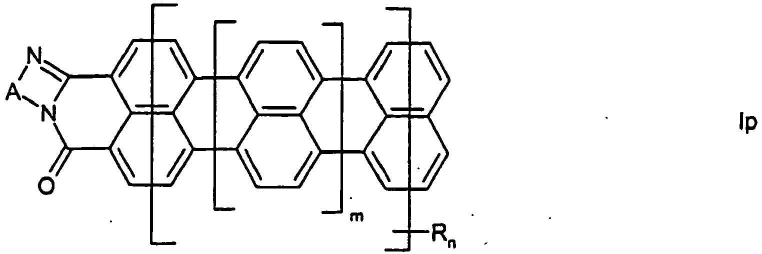 mit N-Halogensuccinimid in Gegenwart eines polaren organischen Lösungsmittels und einer Lewis-Säure als Katalysator zugänglich.

Hierbei kann analog zu der oben für die Rylendicarbonsäureimide Ic, Rylendicarbonsäureanhydride Ig und Rylene Id beschriebenen Halogenierung vorgegangen werden. Der Arylen- oder Hetarylenrest kann dabei auch ein- bis vierfach halogeniert werden.

Weiterhin können die peri-(Dioxaborolan-2-yl)rylendicarbonsäurederivate Ir 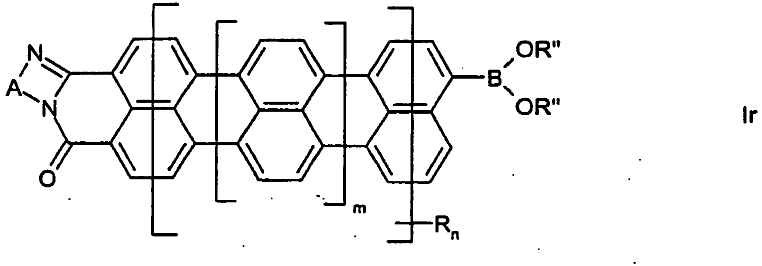 erfindungsgemäß durch Umsetzung der peri-halogenierten Rylehdicarbonsäurederivate Iq 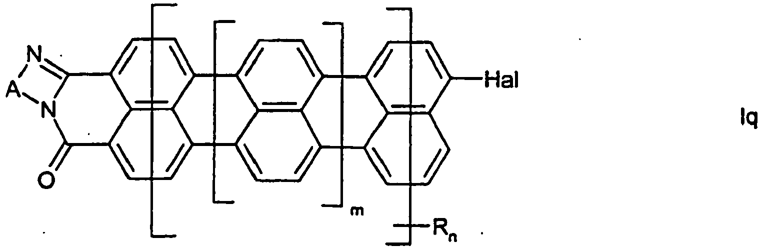 mit einem aromatischen Diboran **III** 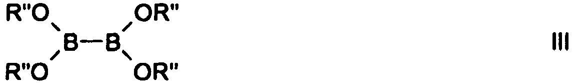 in Gegenwart eines aprotischen organischen Lösungsmittels, eines Übergangsmetallkatalysators und einer Base erhalten werden.

Vorteilhaft geht man dabei analog zu der oben beschriebenen Umsetzung der peri-halogenierten Rylendicarbonsäureimide Ie, der peri-halogenierten Rylendicarbonsäureanhydride Ih und der Halogenrylene If mit dem Diboran III vor.

Die erfindungsgemäßen Rylenderivate I zeigen starke Absorption im Infrarotbereich bei Wellenlängen von 550 bis 900 nm. Ihre Funktionalisierung kann gezielt gewählt werden, so daß sie direkt an den gewünschten Anwendungszweck angepaßt werden können.

Sie eignen sich für eine Vielzahl von Anwendungen, wie die Einfärbung von hochmolekularen organischen und anorganischen Materialien, z.B. von Lacken, Druckfarben und Kunststoffen, zur Herstellung im nahinfraroten Bereich des elektromagnetischen Spektrums absorbierender wäßriger Polymerisatdispersionen, zur Erzeugung für das menschliche Auge nicht sichtbarer, Infrarotlicht absorbierender Markierungen und Beschriftungen, als Infrarotabsorber für das Wärmemanagement, als IR-Iaserstrahlenabsorbierende Materialien beim Schweißbehandeln von Kunststoffteilen, als Halbleiter in der organischen Elektronik, als Emitter in Elektro- und Chemilumineszenzanwendungen sowie als Aktivkomponenten in der Photovoltaik.

Außerdem können sie vorteilhaft als Edukte zur Herstellung längerwellig absorbierender höherer Rylene eingesetzt werden.

### Beispiele

### Beispiel

N-(2,6-Diisopropylphenyl)-1,6,11,16-tetra[4-(1,1,3,3-tetramethy)butyl)phenoxy]quaterrylen-3,4:13,14-tetracarbonsäuremonoimidmonoanhydrid Ia" und 1,6,11,16-Tetra[4-(1,1,3,3-tetramethylbutyl)phenoxy]quaterrylen-3,4:13,14-tetracarbonsäuredianhydrid Ib"

Eine Mischung von 5,0 g (2,8 mmol) N,N'-Di(2,6-düsopropylphenyl)-1,6,11,16-tetra[4-(1,1,3,3-tetramethylbutyl)phenoxy]quaterrylen-3,4:13,14-tetracarbonsäurediimid II", 7,2 g (112,5 mmol) Kaliumhydroxid und 250 ml tert.-Butanol wurde auf 80°C erhitzt und 7 h bei dieser Temperatur gerührt. Nach dünnschichtchromatographischer Überprüfung der Vollständigkeit der Umsetzung wurde die Reaktionsmischung bei 75°C in 20 min mit einer Lösung von 33,8 g Eisessig in 170 ml Wasser (entspricht einer etwa 17 gew.-%igen Essigsäure) versetzt, dann auf etwa 90°C erhitzt und noch 4 h bei dieser Temperatur gerührt. Das Reaktionsprodukt wurde durch Zugabe von 250 ml Wasser gefällt, bei 50°C abfiltriert, zunächst mit heißem Wasser und dann mehrfach mit Methanol gewaschen und im Vakuum bei 60°C getrocknet. Das Rohprodukt wurde einer Säulenchromatographie an Kieselgel zuerst mit Toluol und dann mit Aceton als Eluens unterzogen.

Es wurden 1,92 g (40%) Ia" in Form eines grünen Feststoffs sowie 1,26 g (31%) Ib" in Form eines grünen Feststoffs erhalten, was einer Gesamtausbeute von 70% entspricht.

Bei analoger Vorgehensweise, jedoch unter Hydrolyse mit 50 gew.%iger Essigsäure wurden 0,51 g (10%) Ia" und 2,85 g (70%) Ib" erhalten, was einer Gesamtausbeute von 80% entspricht.

Analytische Daten von Ia":
¹H-NMR (500 MHz, CD₂Cl₂, 25°C): δ = 9,0 (s, 2H); 8,75 (s, 2H); 8,12 (s, 2H); 7,7 (s, 3H); 7,45-7,43 (m, 13H); 7,1 (d, 6H); 6,7 (d, 4H); 2,70 (m, 2H); 1,76 (d, 8H); 1,40 (d, 24); 1,02 (d, 12 H); 0,75 (d, 36H) ppm;
UV-Vis (CHCl₃): λₘₐₓ = 784, 718, 384 nm;
MS (FD): m/z (rel. Int.) = 1617 (100%) [M⁺].

Analytische Daten zu Ib":
¹H-NMR (500 MHz, CD₂Cl₂, 25°C): δ = 9,5 (s, 4H); 8,45 (s, 4H); 8,1 (s, 4H); 7,45 (d, 8H); 7,10 (d, 8H); 1,78 (d, 8H); 1,41 (s, 24H); 0,78 (s, 36H) ppm;
UV-Vis (CHCl₃): λₘₐₓ = 790, 720 nm;
MS (FD): m/z (rel. Int.) = 1457 (100%) [M⁺].

### Beispiel 2

### N-(2,6-Diisopropylphenyl)-1,6,11,16-tetra[4-(1,1,3,3-tetramethylbutyl)phenoxy]quaterrylen-3,4-dicarbonsäureimid Ic" und 1,6,11,16-Tetra[4-(1,1,3,3-tetramethylbutyl)-phenoxy]quaterrylen Id"

Zu einer unter Stickstoff gerührten Mischung von 4,4 g eines analog Beispiel 8 erhaltenen, im wesentlichen aus Ia" und Ib" bestehenden Reaktionsgemischs und 50 ml Chinolin wurden 0,4 g (2,7 mmol) Kupfer(I)oxid gegeben. Dann wurde die Mischung auf 210°C erhitzt und 1 h bei dieser Temperatur gerührt. Nach Abkühlen auf Raumtemperatur, Zugabe von 200 ml 1 m Salzsäure zur Fällung des Reaktionsprodukts, erneutem Erhitzen auf 60°C und einstündigem Rühren bei dieser Temperatur wurde das Reak-tionsprodukt abfiltriert, mit heißem Wasser gewaschen und dann in Methylenchlorid gelöst. Die erhaltene Lösung wurde über Magnesiumsulfat getrocknet, von diesem abgetrennt und eingeengt. Das Rohprodukt wurde in Toluol gelöst und an Kieselgel fraktioniert chromatographiert. Die erhaltenen Fraktionen wurden am Rotationsverdampfer eingeengt und im Vakuum bei 70°C getrocknet.

Es wurden 1,55 g (36%, bezogen auf das in Beispiel 8 eingesetzte II") Ic" in Form eines grünen Feststoffs sowie 1,25 g (34%, bezogen auf das in Beispiel 8 eingesetzte II") Id" in Form eines blauen Feststoffs erhalten, was einer Gesamtausbeute von 70%, bezogen auf das in Beispiel 8 eingesetzte II", entspricht.

Analytische Daten von Ic":
¹H-NMR (500 MHz, CD₂Cl₂, 25°C): δ = 9,35(d, 2H); 9,2 (d, 2H); 8,25 (d, 2H); 8,15 (s, 2H); 8,12 (d, 2H); 7,9 (s, 2H); 7,5 (m, 9H); 7,25 (d, 2H); 7,15 (dd, 8H); 2,70 (m, 2H); 1,76 (d, 8H); 1,40 (d, 24); 1,02 (d, 12 H); 0,75 (d, 36H) ppm;
UV-Vis (CHCl₃): λₘₐₓ = 738, 430, 352 nm;
MS (FD): m/z (rel. Int.) = 1545 (100%) [M⁺].

Analytische Daten von Id":
UV-Vs (CHCl₃): λₘₐₓ = 660, 604, 558 nm;
MS (FD): m/z (rel. Int.) = 1317 (100%) [M⁺].

### Beispiel 3

### N-(2,6-Diisopropylphenyl)-1,6,11,16-tetra[4-(1,1,3,3-tetramethylbutyl)phenoxy]-13-chlorquaterrylen-3,4-dicarbonsäureimid Ie"

Zu einer unter Stickstoff gerührten Mischung von 0,2 g (0,12 mmol) Ic" und 10 ml Dimethylformamid wurden 0,1 g (0,5 mmol) N-Bromsuccinimid und 0,002 g (0,01 mmol) Eisen(III)chlorid gegeben. Dann wurde die Mischung auf 60°C erhitzt und 1 h bei dieser Temperatur gerührt. Nach Abkühlen auf Raumtemperatur wurde das Reaktionsprodukt auf 100 ml 1 m Natronlauge gefällt und mit Methyl-tert.-butylether aus dem Niederschlag extrahiert. Die organische Phase wurde über Magnesiumsulfat getrocknet, von diesem abgetrennt und eingeengt. Das Produkt wurde in Methylenchlorid gelöst und durch Filtration an Kieselgel von Verunreinigungen befreit.

Es wurden 0,08 g Ie" in Form eines grünen Feststoffs erhalten, was einer Ausbeute von 42% entspricht.

Analytische Daten von Ie":
¹H-NMR (500 MHz, CD₂Cl₂, 25°C): δ = 9,05(d, 2H); 8,75 (d, 1 H); 8,65 (d, 1 H); 8,19 (s, 1 H); 8,14 (s, 1 H); 7,9 (s, 2H); 7,5 (m, 9H); 7,25 (d, 2H); 7,15 (dd, 1 H); 2,70 (m, 2H); 1,76 (d, 8H); 1,40 (d, 24); 1,02 (d, 12 H); 0,75 (d, 36H) ppm;
UV-Vis (CHCl₃): λₘₐₓ = 676, 742 nm;
MS (FD): m/z (rel. Int.) = 1580 (100%) [M⁺].

### Beispiel 4

### 1,6,11,16-Tetra[4-(1,1,3,3-tetramethylbutyl)phenoxy]quaterrylen-3,4-dicarbonsäureanhydrid Ig'

Eine Mischung von 5,0 g (3,2 mmol) Ic", 7,2 g (112,5 mmol) Kaliumhydroxid und 250 ml tert.-Butanol wurde auf 80°C erhitzt und 7 h bei dieser Temperatur gerührt. Nach dünnschichtchromatographischer Überprüfung der Vollständigkeit der Umsetzung wurde die Reaktionsmischung bei 75°C in 20 min mit einer Lösung von 33,8 g Eisessig in 170 ml Wasser (entspricht einer etwa 17 gew.%igen Essigsäure) versetzt, dann auf etwa 90°C erhitzt und noch 4 h bei dieser Temperatur gerührt. Das Reaktionsprodukt wurde durch Zugabe von 250 ml Wasser gefällt, bei 50°C abfiltriert, zunächst mit hei-βem Wasser und dann mehrfach mit Methanol gewaschen und im Vakuum bei 60°C getrocknet. Das Rohprodukt wurde einer Säulenchromatographie an Kieselgel mit Chloroform als Eluens unterzogen.

Es wurden 2,6 g Ig' in Form eines grünen Feststoffs erhalten, was einer Ausbeute von 60% entspricht.

Analytische Daten von Ig':
UV-Vis (CHCl₃): λₘₐₓ = 628, 687 nm;
MS (FD): m/z (rel. Int.) = 1317 (100%) [M⁺].

### Beispiel 5

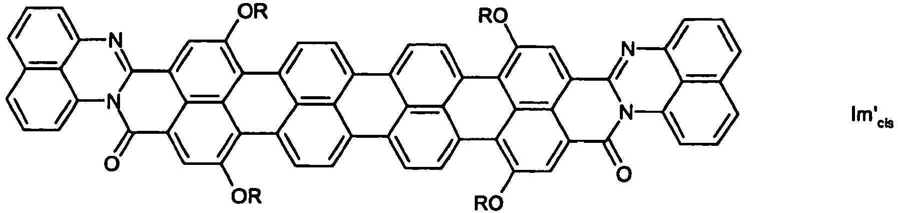

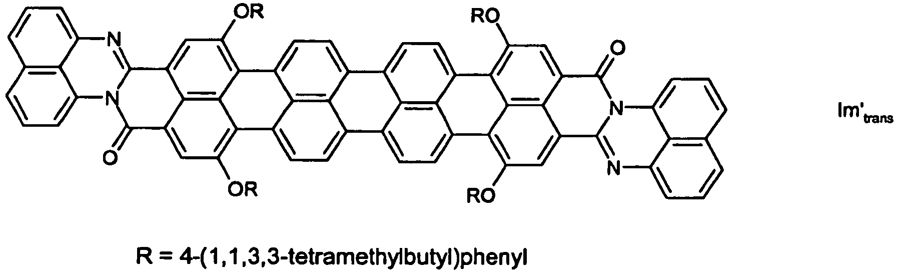

Eine Mischung von 1,0 g (0,68 mmol) Ib", 0,4 g (2,4 mmol) 1,8-Diaminonaphthalin, 0,2 g (1,3 mmol) Zinkacetatdihydrat und 20 ml Chinolin wurde 10 min unter Stickstoff gerührt, dann auf 220°C erhitzt und 4 h bei dieser Temperatur gerührt. Nach dünnschichtchromatographischer Überprüfung der Vollständigkeit der Umsetzung und Abkühlen auf Raumtemperatur wurde das Reaktionsprodukt durch Zugabe von 200 ml 5 gew.%iger Salzsäure gefällt, abfiltriert, mit Wasser neutral gewaschen und im Vakuum bei 70°C getrocknet.

Es wurden 1,1 g des cis/trans-Isomerengemischs Im'_{cis} und Im'ₜᵣₐₙₛ erhalten, was einer Ausbeute von 98% entspricht.

Analytische Daten:
UV-Vis (H₂SO₄): λₘₐₓ = 962 nm;
MS (FD): m/z (ret. Int.) = 1700 (100%) [M⁺].

### Beispiel 6

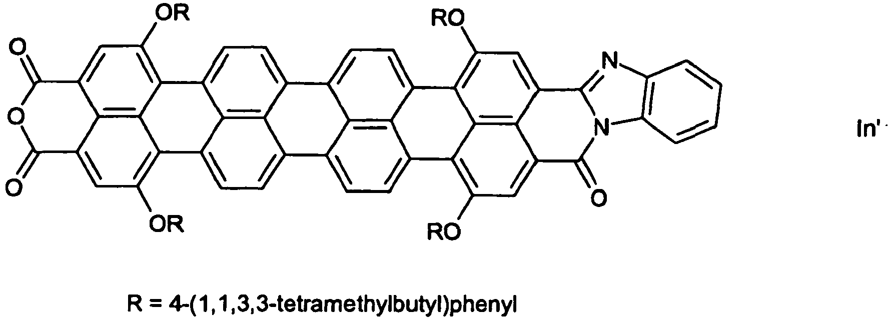

Eine Mischung von 0,5 g (0,34 mmol) Ib", 0,04 g (0,37 mmol) o-Phenylendiamin, 0,06 g (0,37 mmol) Zinkacetatdihydrat und 20 ml Chinolin wurde 10 min unter Stickstoff gerührt, dann auf 215°C erhitzt und 2 h bei dieser Temperatur gerührt. Nach Abkühlen auf Raumtemperatur wurde das Reaktionsprodukt durch Zugabe von 200 ml 5 gew.-%iger Salzsäure gefällt, abfiltriert, mit Wasser neutral gewaschen und im Vakuum bei 70°C getrocknet.

Es wurden 0,15 g In' erhalten, was einer Ausbeute von 30% entspricht.

Analytische Daten:
UV-Vis (H₂SO₄): λₘₐₓ = 733, 791 nm;
MS (FD): m/z (rel. Int.) = 1528 (100%) [M⁺].

## Patentansprüche

1. Rylenderivate der allgemeinen Formel I 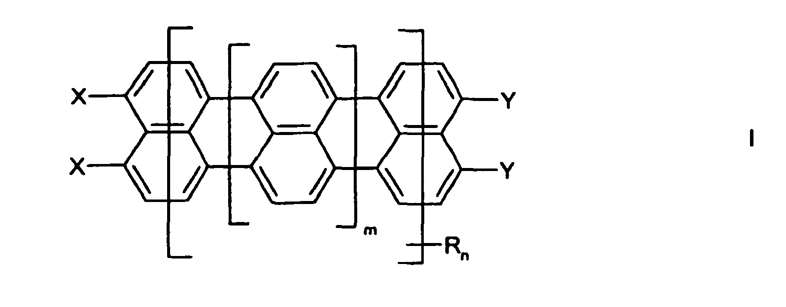 in der die Variablen folgende Bedeutung haben:
X miteinander unter Ausbildung eines Sechsrings verbunden zu einem Rest der Formel (a), (b) oder (c) 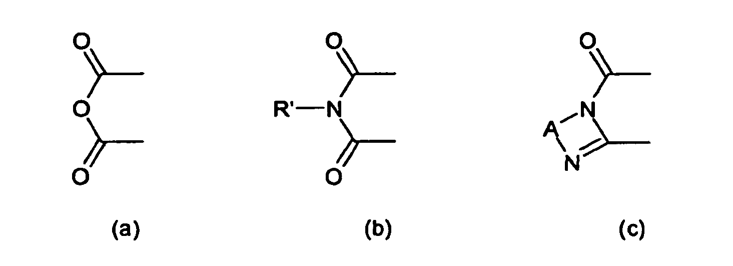 beide ein Rest -COOM;
beide Wasserstoff oder einer der beiden Reste Wasserstoff und der andere Rest Halogen oder ein Rest der Formel (d) 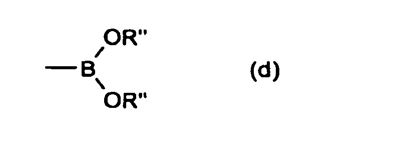
Y miteinander unter Ausbildung eines Sechsrings verbunden zu einem Rest der Formel (a), wenn einer der beiden Reste X Wasserstoff und der andere Rest X Halogen oder einen Rest der Formel (d) bedeutet oder wenn beide Reste X Wasserstoff oder zusammen einen Rest der Formel (a), (b) oder (c) bedeuten;
miteinander unter Ausbildung eines Sechsrings verbunden zu einem Rest der Formel (b), wenn einer der beiden Reste X Wasserstoff und der andere Rest X Halogen oder einen Rest der Formel (d) bedeutet oder wenn beide Reste X Wasserstoff oder einen Rest -COOM oder zusammen einen Rest der Formel (c) bedeuten;
miteinander unter Ausbildung eines Sechsrings verbunden zu einem Rest der Formel (c), wenn einer der beiden Reste X Wasserstoff und der an-dere Rest X Halogen oder einen Rest der Formel (d) bedeutet oder wenn beide Reste X Wasserstoff oder einen Rest -COOM oder zusammen einen Rest der Formel (c), der cis- oder trans-ständig zum anderen Rest (c) angeordnet sein kann, bedeuten; beide ein Rest -COOM, wenn einer der beiden Reste X Wasserstoff und der andere Rest X Halogen oder einen Rest der Formel (d) bedeutet oder wenn beide Reste X Wasserstoff oder einen Rest -COOM bedeuten, wobei für den Fall, daß ein Rest X einen Rest der Formel (d) bedeutet, M von Wasserstoff verschieden ist;
beide Wasserstoff oder einer der beiden Reste Wasserstoff und der andere Rest Halogen oder ein Rest der Formel (d), wenn beide Reste X Wasserstoff oder einer der beiden Reste X Wasserstoff und der andere Rest X Halogen oder einen Rest der Formel (d) bedeutet;
R Phenoxy, Phenylthio, Pyridyloxy, Pyrimidyloxy, Pyridylthio oder Pyrimidyl-thio, das jeweils ein- oder mehrfach durch C₁-C₁₂-Alkyl, C₁-C₁₂-Alkoxy und/oder Aryl substituiert sein kann;
R' Wasserstoff;
C₁-C₃₀-Alkyl, dessen Kohlenstoffkette durch eine oder mehrere Gruppierungen -O-und/oder -CO- unterbrochen sein kann und das ein- oder mehrfach substituiert sein kann durch: C₁-C₆-Alkoxy, Cyano und/oder Aryl, das durch C₁-C₁₈-Alkyl oder C₁-C₆-Alkoxy substituiert sein kann;
Phenyl, Naphthyl, Pyridyl oder Pyrimidyl, das jeweils ein- oder mehrfach substituiert sein kann durch: C₁-C₁₈-Alkyl, C₁-C₆-Alkoxy, Halogen, Cyano, Nitro, -CONR²R³, - SO₂NR²R³, Phenyl- und/oder Naphthylazo, das jeweils durch C₁-C₁₀-Alkyl, C₁-C₆-Alkoxy und/oder Cyano substituiert sein kann;
C₅-C₈-Cycloalkyl, das ein- oder mehrfach durch C₁-C₆-Alkyl substituiert sein kann;
A 1,2-Phenylen oder 1,8-Naphthylen;
M Alkalimetallkation;
R" Wasserstoff, C₁-C₅-Alkyl oder Pinacolato;
R², R³ unabhängig voneinander Wasserstoff;
C₁-C₁₈-Alkyl, das ein- oder mehrfach durch C₁-C₆-Alkoxy, Hydroxy, Halogen und/oder Cyano substituiert sein kann;
Aryl oder Hetaryl, das jeweils ein- oder mehrfach durch C₁-C₆-Alkyl und/oder die vorstehenden, als Substituenten für Alkyl genannten Reste substituiert sein kann;
m 2;
n 4 oder 6,
und deren Mischungen.

2. Verfahren zur Herstellung von Rylentetracarbonsäuremonoimidmonoanhydriden der allgemeinen Formel Ia 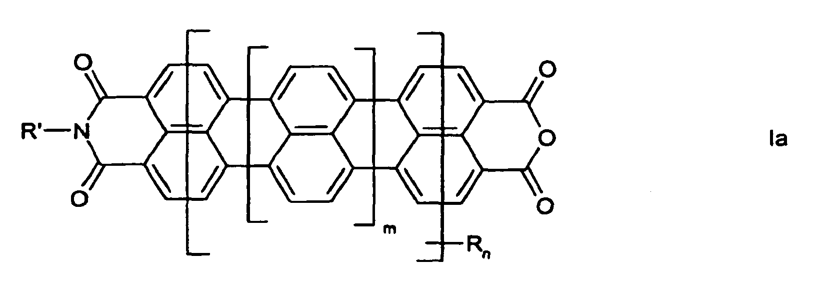 in der die Variablen die in Anspruch 1 genannte Bedeutung haben, **dadurch gekennzeichnet, daß** man
a) ein Rylentetracarbonsäurediimid der allgemeinen Formel II 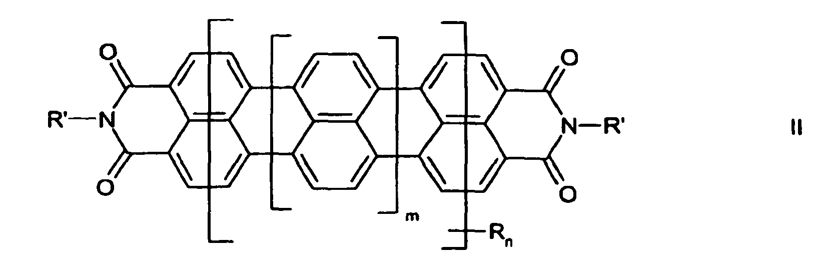 einer Verseifung unter alkalischen Bedingungen in Gegenwart eines polaren organischen Lösungsmittels unterzieht und das Rylentetracarbonsäuremonoimidmonoanhydrid Ia von dem hierbei ebenfalls entstehenden Rylentetracarbonsäuredianhydrid Ib abtrennt
oder
b) das Rylentetracarbonsäurediimid II durch Anwendung milder Reaktionsbedingungen direkt weitgehend einseitig zum Rylentetracarbonsäuremono-imidmonoanhydrid Ia verseift.

3. Verfahren zur Herstellung von Rylentetracarbonsäuredianhydriden der allgemeinen Formel Ib 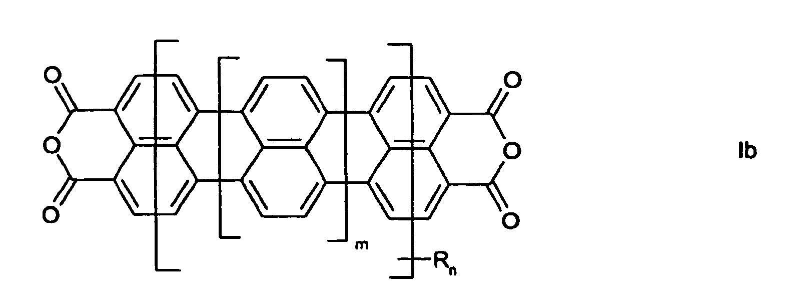 in der die Variablen die in Anspruch 1 genannte Bedeutung haben, **dadurch gekennzeichnet, daß** man
a) ein Rylentetracarbonsäurediimid der allgemeinen Formel **II** 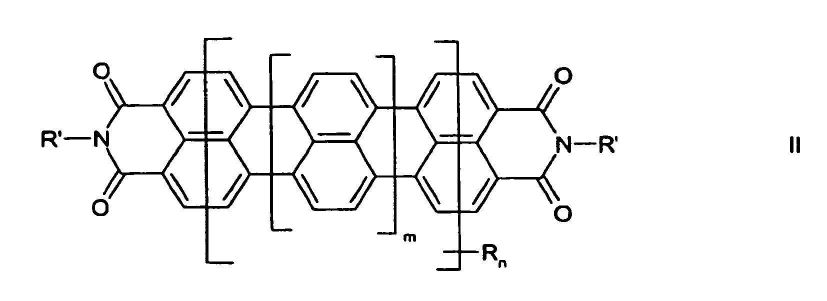 in der die Reste R' die in Anspruch 1 genannte Bedeutung haben, einer Verseifung unter alkalischen Bedingungen in Gegenwart eines polaren organischen Lösungsmittels unterzieht und das Rylentetracarbonsäure-dianhydrid Ib von dem hierbei ebenfalls entstehenden Rylentetacarbonsäuremonoimidmonoanhydrid Ia abtrennt
oder
b) das Rylentetracarbonsäurediimid II durch Anwendung verschärfter Reaktionsbedingungen direkt weitgehend beidseitig zum Rylentetracarbonsäure-dianhydrid Ib verseift.

4. Verfahren zur Herstellung von Rylendicarbonsäureimiden der allegemeinen Formel Ic 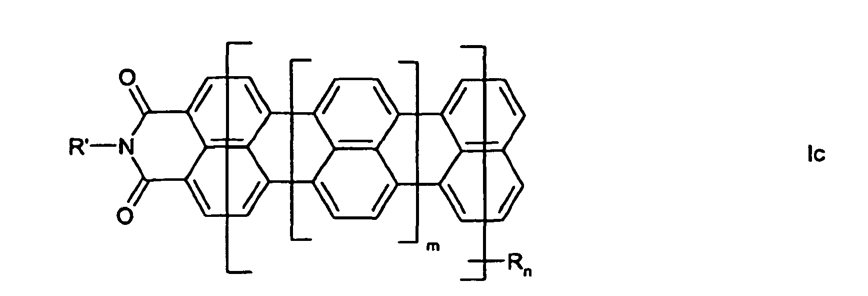 in der die Variablen die in Anspruch 1 genannte Bedeutung haben, **dadurch gekennzeichnet, daß** man
a) ein Rylentetracarbonsäuremonoimidmonoanhydrid der allgemeinen Formel la 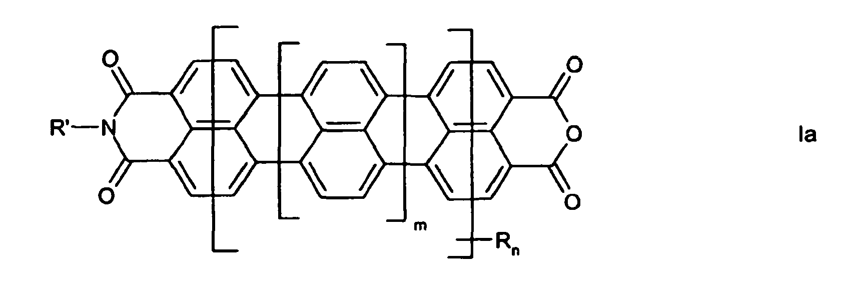 oder
b) das bei der alkalischen Verseifung eines Rylentetracarbonsäurediimids der allgemeinen Formel II 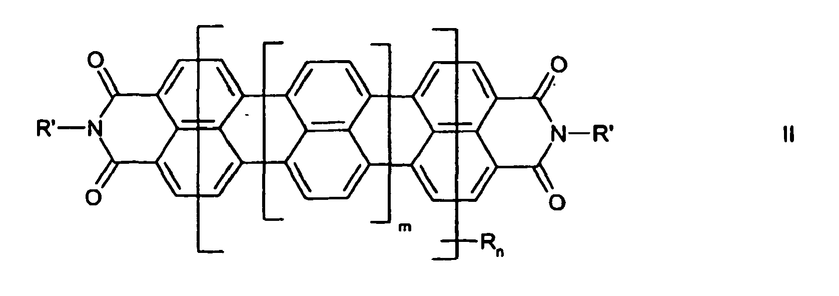 anfallende Gemisch von Rylentetracarbonsäuremonoimidmonoanhydrid Ia und Rylentetracarbonsäuredianhydrid Ib
einer Decarboxylierung in Gegenwart einer tertiären stickstoffbasischen Verbindung als Lösungsmittel und eines Übergangsmetallkatalysators unterzieht und im Fall b) das Rylendicarbonsäureimid Ic von dem ebenfalls entstehenden vollständig decarboxylierten Rylen Id trennt.

5. Verfahren zur Herstellung von Rylenen der allgemeinen Formel Id 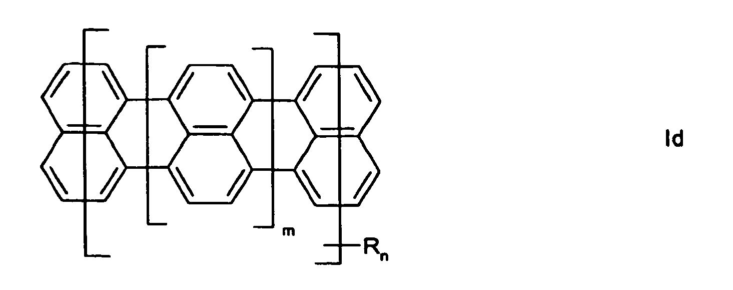 in der die Variablen die in Anspruch 1 genannte Bedeutung haben, **dadurch gekennzeichnet, daß** man
a) ein Rylentetracarbonsäuredianhydrid der allgemeinen Formel Ib 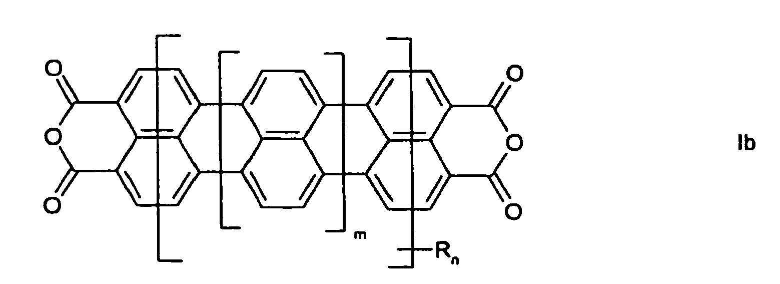 oder
b) das bei der alkalischen Verseifung eines Rylentetracarbonsäurediimids der allgemeinen Formel II 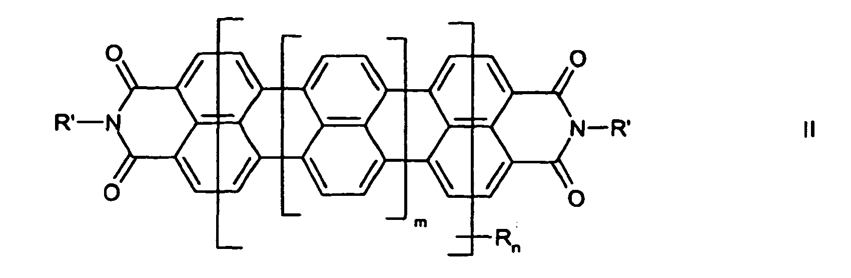 in der die Reste R' die in Anspruch 1 genannte Bedeutung, anfallende Gemisch von Rylentetracarbonsäuremonoimidmonoanhydrid Ia und Rylentetracarbonsäuredianhydrid Ib
einer Decarboxylierung in Gegenwart einer tertiären stickstoffbasischen Verbindung als Lösungsmittel und eines Übergangsmetallkatalysators unterzieht und im Fall b) das Rylen Id von dem ebenfalls entstehenden Rylen-dicarbonsäureimid Ic trennt.

6. Verfahren zur Herstellung von peri-halogenierten Rylendicarbonsäureimiden der allgemeinen Formel Ie 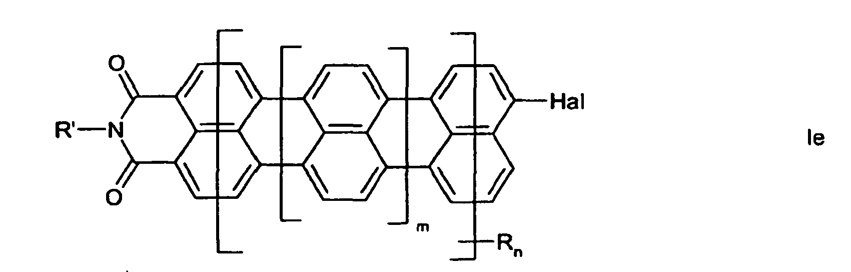 in der Hal Halogen bedeutet und die weiteren Variablen die in Anspruch 1 genannte Bedeutung haben, **dadurch gekennzeichnet, daß** man
a) ein Rylendicarbonsäureimid der allgemeinen Formel Ic 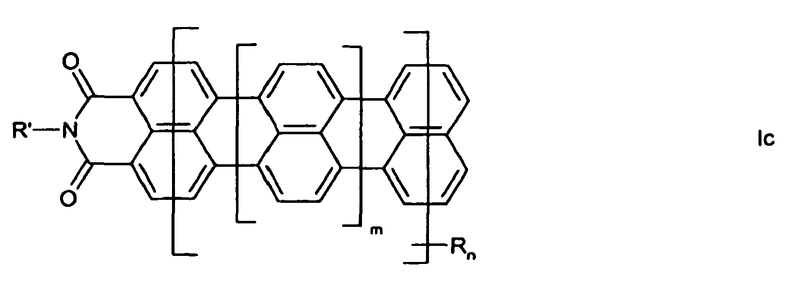 oder
b) das Gemisch von Rylendicarbonsäureimid Ic und Rylen Id, das bei der Decarboxylierung des bei der alkalischen Verseifung des Rylentetracarbonsäurediimids der allgemeinen Formel II 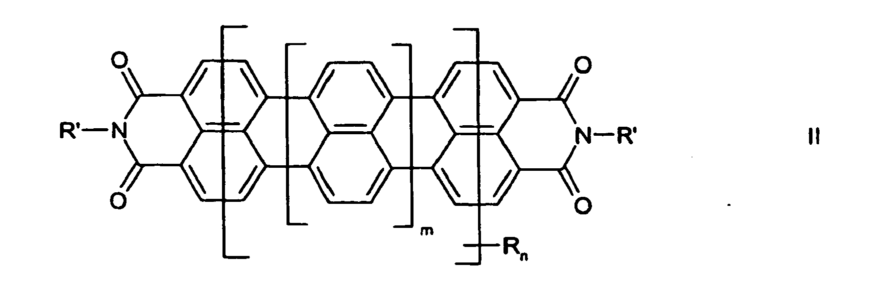 anfallenden Gemischs von Rylentetracarbonsäuremonoimidmonoanhydrid Ia und Rylentetracarbonsäuredianhydrid Ib anfällt,
in Gegenwart eines polaren organischen Lösungsmittels und gewünschtenfalls einer Lewis-Säure als Katalysator mit 1 bis 6 mol N-Halogensuccinimid pro einzuführendes Halogenatom umsetzt und im Fall b) das peri-haloge-nierte Rylendicarbonsäureimid Ie von dem ebenfalls halogenierten Rylen If trennt.

7. Verfahren zur Herstellung von halogenierten Rylenen der allgemeinen Formel If 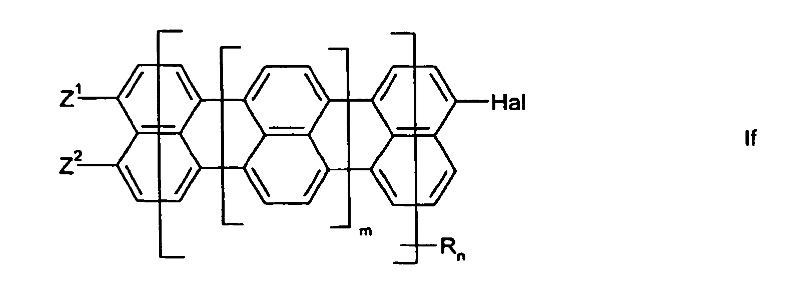 in der Hal für Halogen steht, Z¹ und Z² Wasserstoff bedeuten oder einer der beiden Reste Z¹ oder Z² Halogen und der andere Rest Wasserstoff bedeutet und die weiteren Variablen die in Anspruch 1 genannte Bedeutung haben, **dadurch gekennzeichnet, daß** man
a) ein Rylen der allgemeinen Formel Id 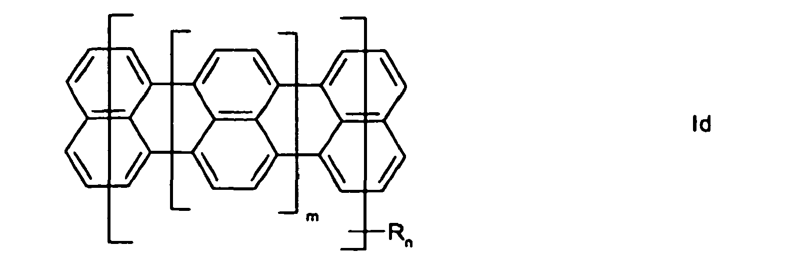 in Gegenwart eines polaren organischen Lösungsmittels und einer Lewis-Säure als Katalysator
a1) direkt mit der zur Einführung der insgesamt gewünschten Anzahl Halogenatome erforderlichen Menge von 1 bis 6 mol N-Halogensuccinimid pro einzuführendes Halogenatom
oder
a2) zunächst mit 1 bis 3 mol/mol N-Halogensuccinimid zum monohalogenierten Rylen If (Z¹=Z²=H) und dann mit weiteren 1 bis 6 mol/mol N-Halogen-succinimid zum dihalogenierten Rylen If (Z¹ oder Z²=Halogen) umsetzt
oder
b) das Gemisch von Rylendicarbonsäureimid Ic und Rylen Id, das bei der Decarboxylierung des bei der alkalischen Verseifung des Rylentetracarbonsäurediimids der allgemeinen Formel II 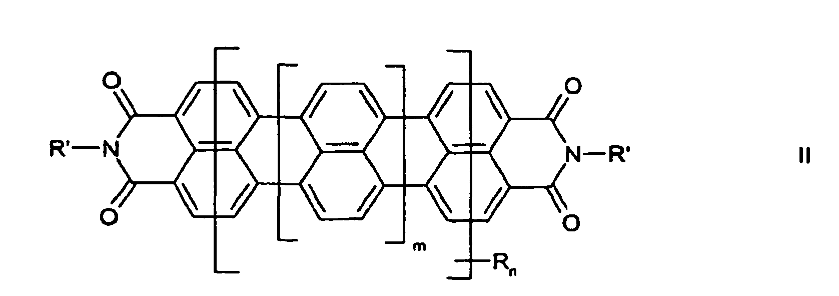 in der die Reste R' die in Anspruch 1 genannte Bedeutung haben, anfallenden Gemischs von Rylentetracarbonsäuremonoimidmonoanhydrid Ia und Rylentetracarbonsäuredianhydrid Ib anfällt,
in Gegenwart eines polaren organischen Lösungsmittels und einer Lewis-Säure als Katalysator
b1) direkt mit der zur Einführung der insgesamt gewünschten Anzahl Halogenatome erforderlichen Menge von 1 bis 6 mol N-Halogensuccinimid pro einzuführendes Halogenatom
oder
b2) zunächst mit 1 bis 3 mol N-Hatogensuccinimid pro mol Id und Ic umsetzt und das monohalogenierte Rylen If (Z¹=Z²=H) von dem ebenfalls gebildeten peri-halogenierten Rylendicarbonsäureimid le trennt und dann mit weiteren 1 bis 6 mol/mol N-Halogensuccinimid pro mol zum dihalogenierten Rylen If (Z¹ oder Z²=Halogen) umsetzt.

8. Verfahren zur Herstellung von Rylendicarbonsäureanhydriden der allgemeinen Formel Igh 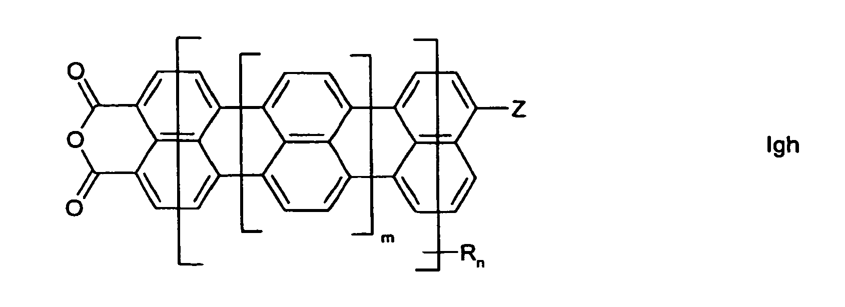 in der Z Wasserstoff oder Halogen bedeutet und die weiteren Variablen die in Anspruch 1 genannte Bedeutung haben, **dadurch gekennzeichnet, daß** man ein
Rylendicarbonsäureimid der allgemeinen Formel Ice 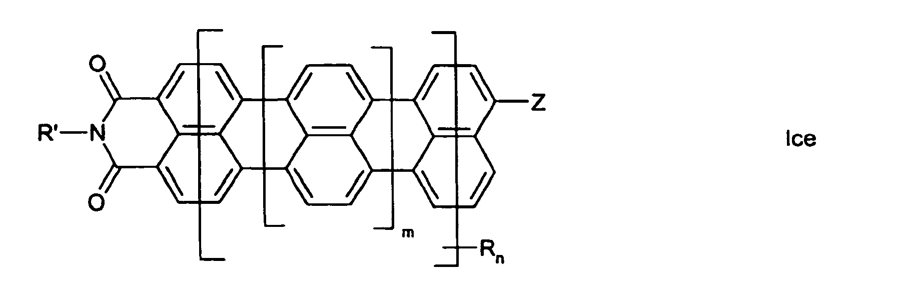 in der R' die in Anspruch 1 genannte Bedeutung hat, einer Verseifung unter alkalischen Bedingungen in Gegenwart eines polaren organischen Lösungsmittels unterzieht.

9. Verfahren zur Herstellung von peri-halogenierten Rylendicarbonsäureanhydriden der allgemeinen Formel Ih 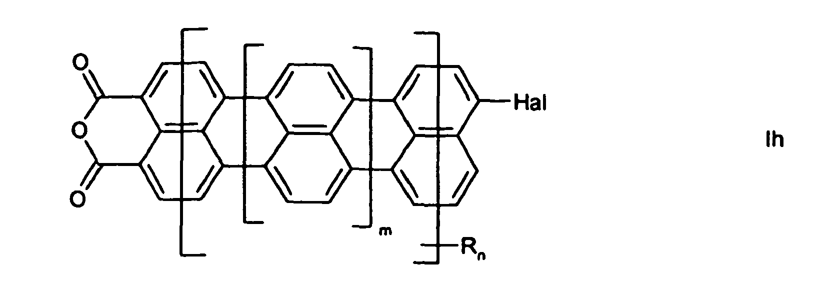 in der Hal Halogen bedeutet und die weiteren Variablen die in Anspruch 1 genannte Bedeutung haben, **dadurch gekennzeichnet, daß** man ein Rylendicarbonsäureanhydrid der allgemeinen Formel Ig 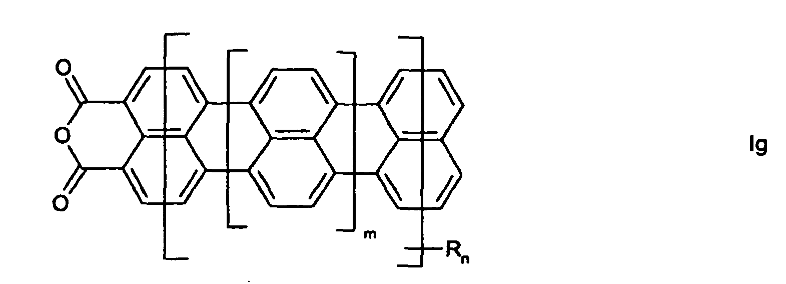 in Gegenwart eines polaren organischen Lösungsmittels und einer Lewis-Säure mit N-Halogensuccinimid umsetzt.

10. Verfahren zur Herstellung von peri-(Dioxaborolan-2-yl)rylendicarbonsäureimiden der
allgemeinen Formel Ii 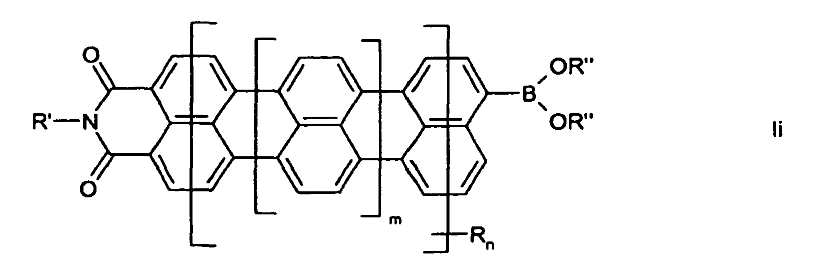 in der die Variablen die in Anspruch 1 genannte Bedeutung haben, **dadurch gekennzeichnet, daß** man ein peri-halogeniertes Rylendicarbonsäureimid der allgemeinen Formel Ie 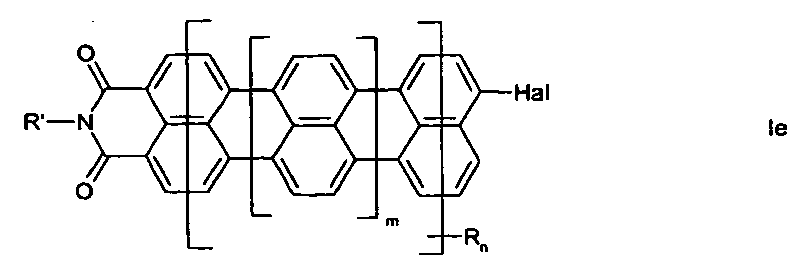 in der Hal Halogen bedeutet, in Gegenwart eines aprotischen organischen Lösungsmittels, eines Übergangsmetallkatalysators und einer Base mit einem Di-boran der allgemeinen Formel III 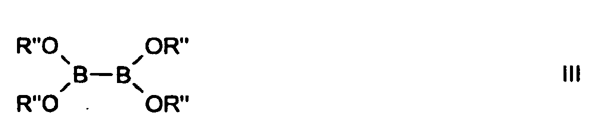 umsetzt.

11. Verfahren zur Herstellung von subsituierten Rylenen der allgemeinen Formel Ij 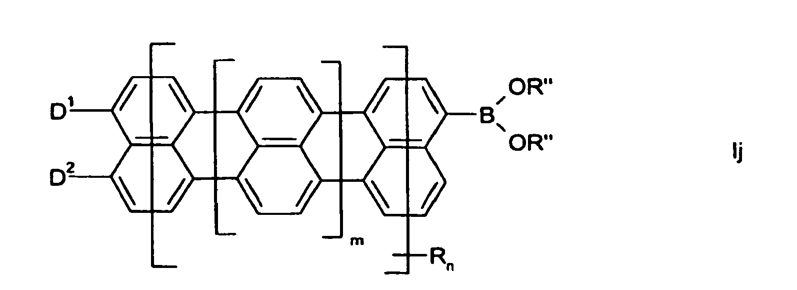 in der D¹ und D² Wasserstoff bedeuten oder einer der beiden Reste D¹ oder D² Halogen oder einen Rest der Formel (d) 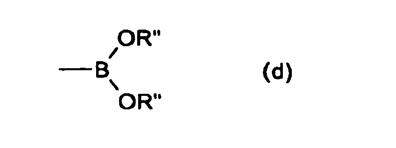 und der andere Rest Wasserstoff bedeutet und die weiteren Variablen die in Anspruch 1 genannte Bedeutung haben, **dadurch gekennzeichnet, daß** man
a) zur Herstellung von Mono(dioxaborolan-2-yl)rylenen Ij (D¹=D²=H) ein Halogenrylen der allgemeinen Formel If 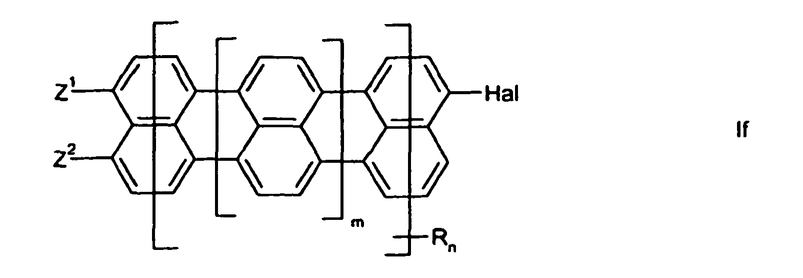 in der Z¹ und Z² Wasserstoff bedeuten,
oder
b) zur Herstellung von Bis(dioxaborolan-2-yl-)rylenen Ij (einer der beiden Reste D¹ oder D² ein Rest (d) und der andere Rest Wasserstoff) oder gemischtsubstituierten Rylenen Ij (einer der beiden Reste D¹ oder **D²** Halogen und der andere Rest Wasserstoff) ein Halogenrylen der Formel If 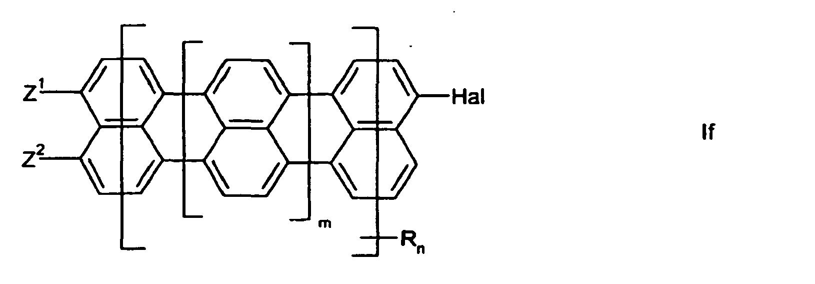 in der einer der beiden Reste Z¹ oder Z² Halogen und der andere Rest Wasserstoff bedeutet,
in Gegenwart eines aprotischen organischen Lösungsmittels, eines Übergangsmetallkatalysators und einer Base mit der zur Einführung der insgesamt gewünschten Anzahl an Dioxaborolan-2-ylresten erforderlichen Menge von 1 bis 3 mol bzw. im Fall der gemischtsubstituierten Rylene Ij 1 bis 1,5 mol eines Diborans der allgemeinen Formel III 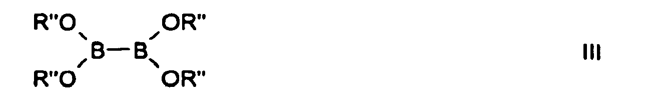 pro mol einzuführender Dioxaborolan-2-ylrest umsetzt.

12. Verfahren zur Herstellung von peri-(Dioxaborolan-2-yl)rylendicarbonsäure-anhydriden der allgemeinen Formel Ik 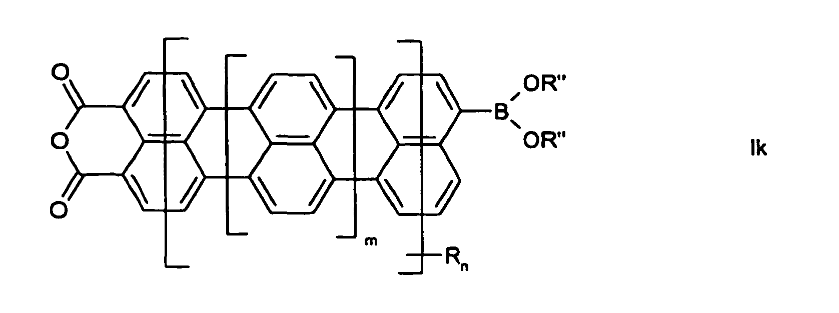 in der die Variablen die in Anspruch 1 genannte Bedeutung haben, **dadurch gekennzeichnet, daß** man ein peri-halogeniertes Rylendicarbonsäureanhydrid der allgemeinen Formel Ih 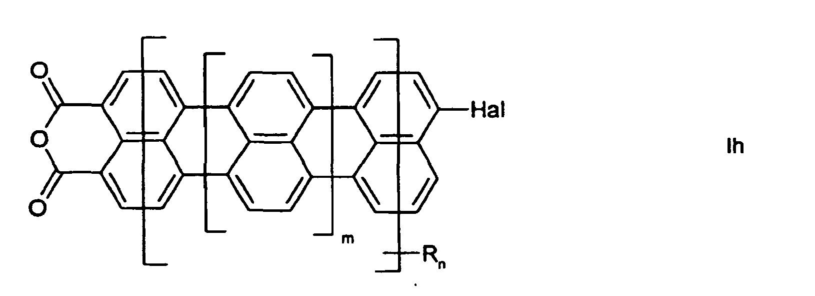 in der Hal Halogen bedeutet, in Gegenwart eines aprotischen organischen Lösungsmittels, eines Übergangsmetallkatalysators und einer Base mit einem Di-boran der allgemeinen Formel III 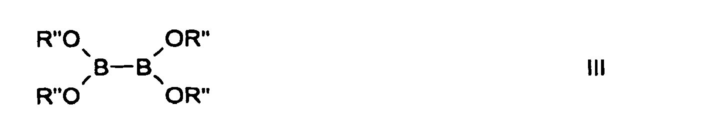 umsetzt.

13. Verfahren zur Herstellung von symmetrischen Rylentetracarbonsäurederivaten der allgemeinen Formel Im_{cis} oder Imₜᵣₐₙₛ 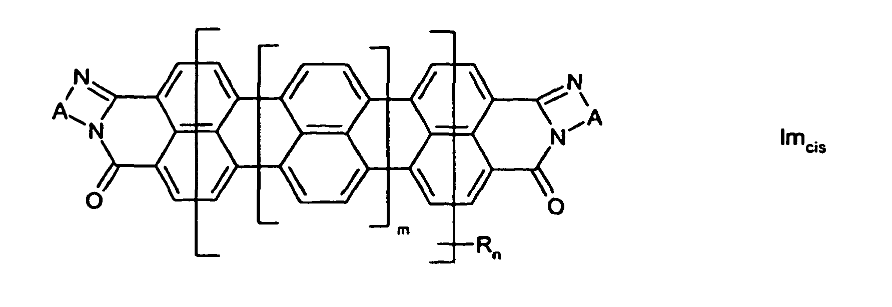 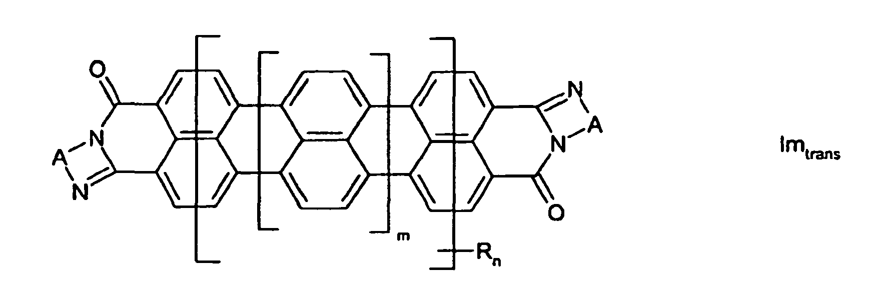 in der die Variablen die in Anspruch 1 genannte Bedeutung haben, wobei die beiden Reste A gleich sind, oder einer Mischung beider Isomere, **dadurch gekennzeichnet, daß** man ein Rylentetracarbonsäuredianhydrid der allgemeinen Formel Ib 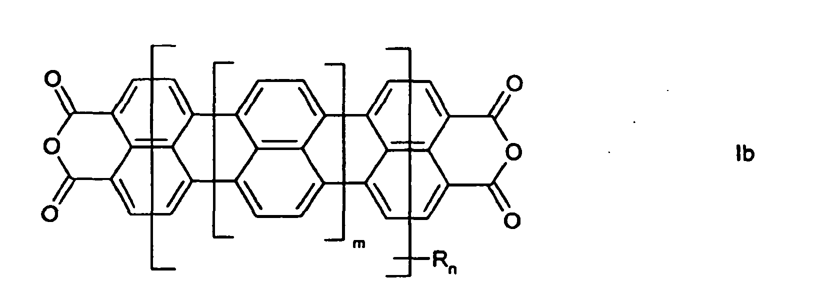 in Gegenwart einer stickstoffbasischen Verbindung oder von Phenol als Lösungs-mittel und einer Lewis-Säure oder von Piperazin als Katalysator mit 2 bis 3 mol/mol eines aromatischen Diamins der allgemeinen Formel IV
H₂N-A-NH₂ IV
kondensiert.

14. Verfahren zur Herstellung von unsymmetrischen Rylentetracarbonsäurederivaten der allgemeinen Formel Im'_{cis} oder Im'ₜᵣₐₙₛ 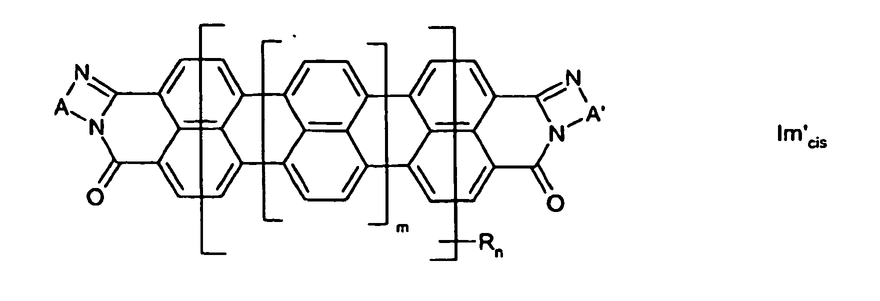 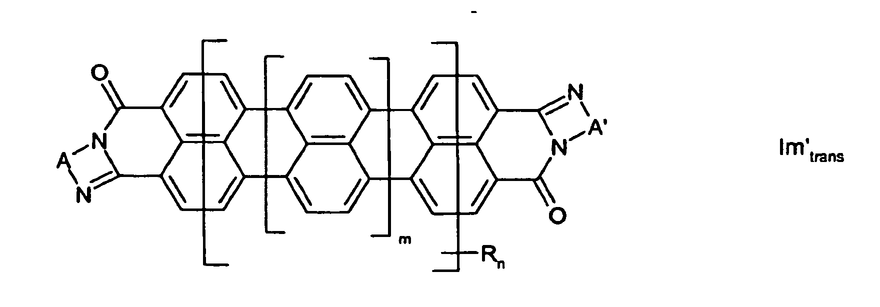 in der die Variablen die in Anspruch 1 genannte Bedeutung haben, wobei die Reste A und A' verschieden sind, oder einer Mischung beider Isomere, **dadurch gekennzeichnet, daß** man ein Rylentetracarbonsäuredianhydrid der allgemeinen Formel Ib 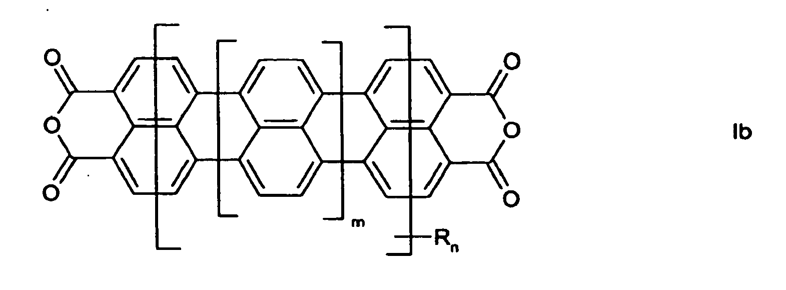 in Gegenwart einer stickstoffbasischen Verbindung oder von Phenol als Lösungsmittel und einer Lewis-Säure oder von Piperazin als Katalysator zunächst mit 1 bis 1,5 mol/mol eines aromatischen Diamins der allgemeinen Formel IV
H₂N-A-NH₂ IV
und dann mit 1 bis 1,5 mol/mol eines aromatischen Diamins der allgemeinen Formel IV
H₂N-A'-NH₂ IV'
kondensiert.

15. Verfahren zur Herstellung von Rylentetracarbonsäurederivaten der allgemeinen Formel In 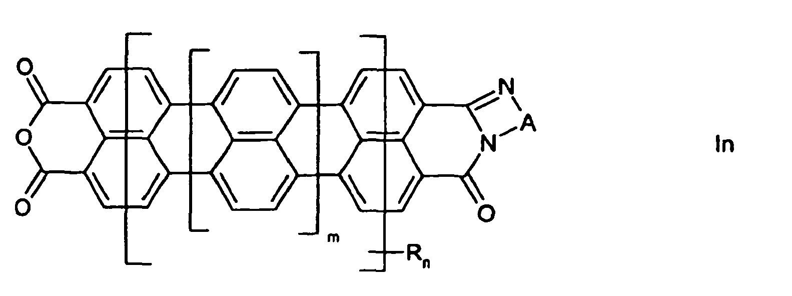 in der die Variablen die in Anspruch 1 genannte Bedeutung haben, **dadurch gekennzeichnet, daß** man ein Rylentetracarbonsäuredianhydrid der allgemeinen Formel Ib 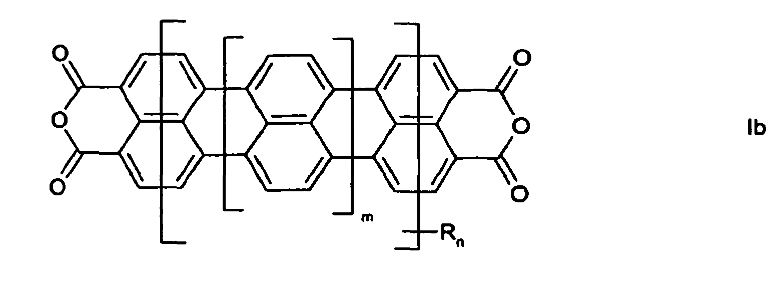 in Gegenwart einer stickstoffbasischen Verbindung oder von Phenol als Lösungs-mittel und einer Lewis-Säure oder von Piperazin als Katalysator mit 1 bis 1.5 mol/mol eines aromatischen Diamins der allgemeinen Formel IV
H₂N-A-NH₂ IV
kondensiert.

16. Verfahren zur Herstellung von Rylentetracarbonsäurederivaten der allgemeinen Formel Io 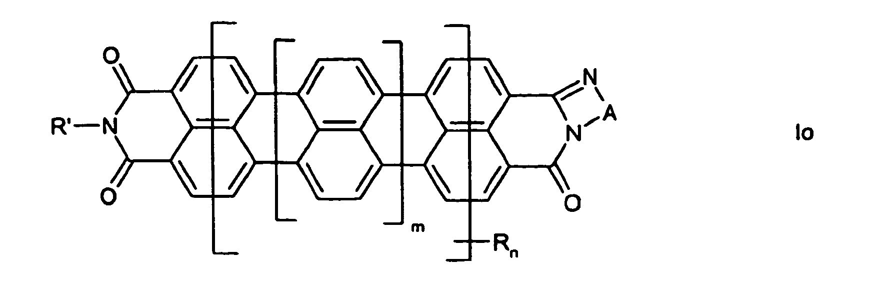 in der die Variablen die in Anspruch 1 genannte Bedeutung haben, **dadurch gekennzeichnet, daß** man ein Rylentetracarbonsäuremonoimidmonoanhydrid der allgemeinen Formel Ia 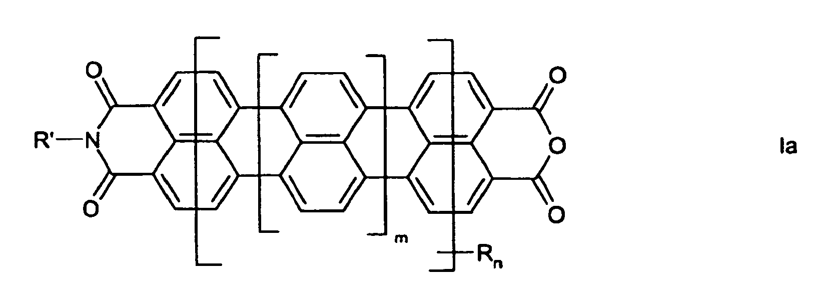 in Gegenwart einer stickstoffbasischen Verbindung oder von Phenol als Lösungs-mittel und einer Lewis-Säure oder von Piperazin als Katalysator mit 1 bis 1,5 mol/mol eines aromatischen Diamins der allgemeinen Formel IV
H₂N-A-NH₂ IV
kondensiert.

17. Verfahren zur Herstellung von Rylendicarbonsäurederivaten der allgemeinen Formel Ip 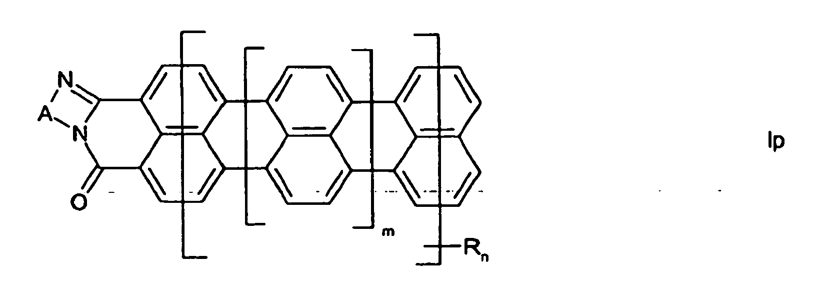 in der die Variablen die in Anspruch 1 genannte Bedeutung haben, **dadurch gekennzeichnet, daß** man
a) ein Rylentetracarbonsäurederivat der allgemeinen Formel In 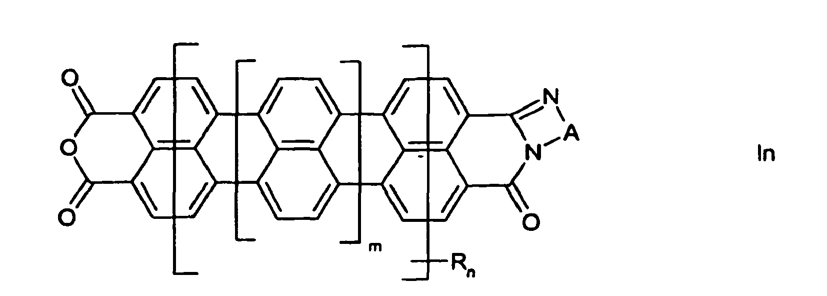 einer Decarboxylierung in Gegenwart einer tertiären stickstoffbasischen Verbindung und eines Übergangsmetallkatalysators unterzieht
oder
b) ein Rylendicarbonsäureanhydrid der allgemeinen Formel Ig 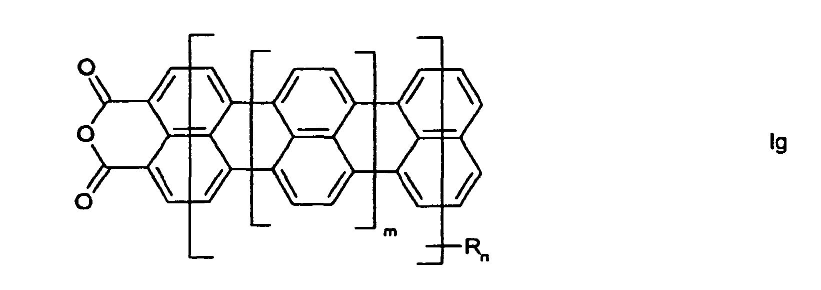 in Gegenwart einer stickstoffbasischen Verbindung oder von Phenol als Lösungsmittel und einer Lewis-Säure oder von Piperazin als Katalysator mit 1 bis 1,5 mol/mol eines aromatischen Diamins der allgemeinen Formel IV
H₂N-A-NH₂ IV
kondensiert.

18. Verfahren zur Herstellung von peri-halogenierten Rylendicarbonsäurederivaten der allgemeinen Formel Iq 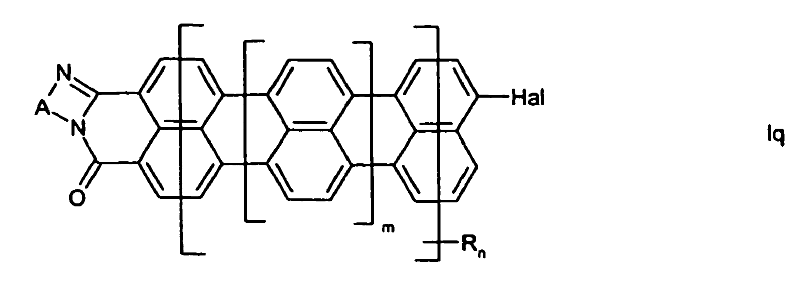 in der die Variablen die in Anspruch 1 genannte Bedeutung haben, **dadurch gekennzeichnet, daß** man ein Rylendicarbonsäurederivat der allgemeinen Formel Ip 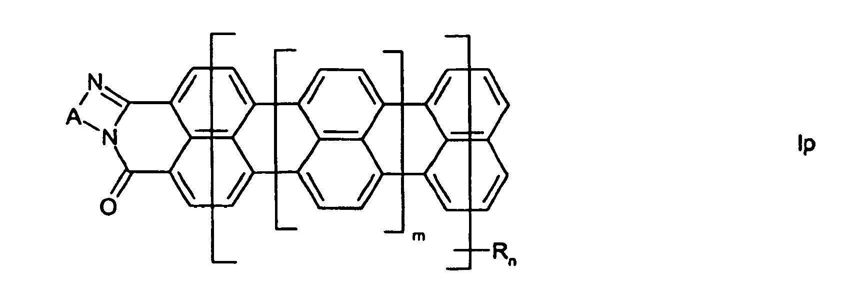 in Gegenwart eines polaren organischen Lösungsmittels und einer Lewis-Säure als Katalysator mit N-Halogensuccinimid umsetzt.

19. Verfahren zur Herstellung von peri-(Dioxaborolan-2-yl)rylendicarbonsäurederi-vaten der allgemeinen Formel Ir 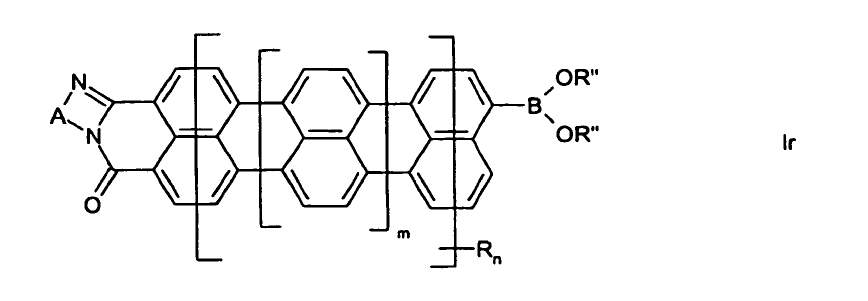 in der die Variablen die in Anspruch 1 genannte Bedeutung haben, **dadurch gekennzeichnet, daß** man ein peri-halogeniertes Rylendicarbonsäurederivat der allgemeinen Formel Iq 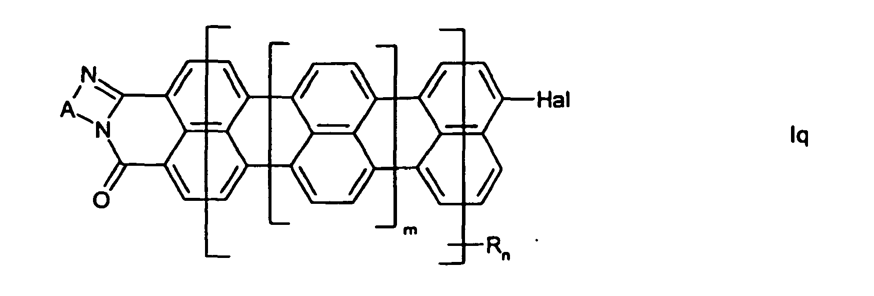 in der Hal Halogen bedeutet, in Gegenwart eines aprotischen organischen Lösungsmittels, eines Übergangsmetallkatalysators und einer Base mit einem Di-boran der allgemeinen Formel III 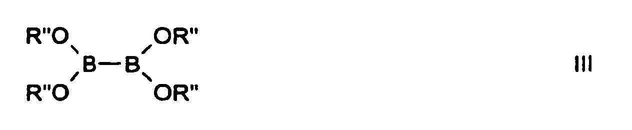 umsetzt.

20. Verwendung von Rylenderivaten der Formel I gemäß Anspruch 1 zur Einfärbung von hochmolekularen organischen und anorganischen Materialien.

21. Verwendung nach Anspruch 20, **dadurch gekennzeichnet, daß** die hochmolekularen Materialien Lacke, Druckfarben oder Kunststoffe sind.

22. Verwendung von Rylenderivaten der Formel I gemäß den Anspruch 1 als Dispergierhilfsmittel und Pigmentadditive für organische Pigmente.

23. Verwendung von Rylenderivaten der Formel I gemäß Anspruch 1 zur Herstellung im nahinfraroten Bereich des elektromagnetischen Spektrums absorbierender wäßriger Polymerisatdispersionen.

24. Verwendung von Rylenderivaten der Formel I gemäß Anspruch 1 zur Erzeugung für das menschliche Auge nicht sichtbarer, Infrarotlicht absorbierender Markierungen und Beschriftungen.

25. Verwendung von Rylenderivaten der Formel I gemäß Anspruch 1 als Infrarotabsorber für das Wärmemanagement.

26. Verwendung von Rylenderivaten der Formel I gemäß Anspruch 1 als IRlaserstrahlenabsorbierende Materialien beim Schweißbehandeln von Kunststoffteilen.

27. Verwendung von Rylenderivaten der Formel I gemäß Anspruch 1 als Halbleiter in der organischen Elektronik.

28. Verwendung von Rylenderivaten der Formel I gemäß Anspruch 1 als Emitter in Elektro-und Chemilumineszenzanwendungen.

29. Verwendung von Rylenderivaten der Formel I gemäß Anspruch 1 als Aktivkomponenten in der Photovoltaik.

## Claims

1. Rylene derivatives of the general formula I 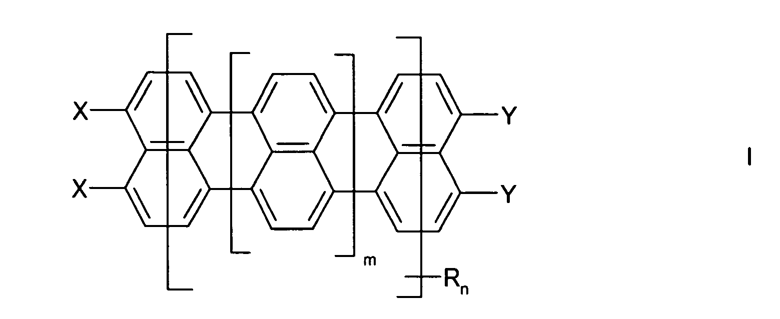 in which the variables are each defined as follows:
X are joined to one another with formation of a six-membered ring to give a radical of the formula (a), (b) or (c) 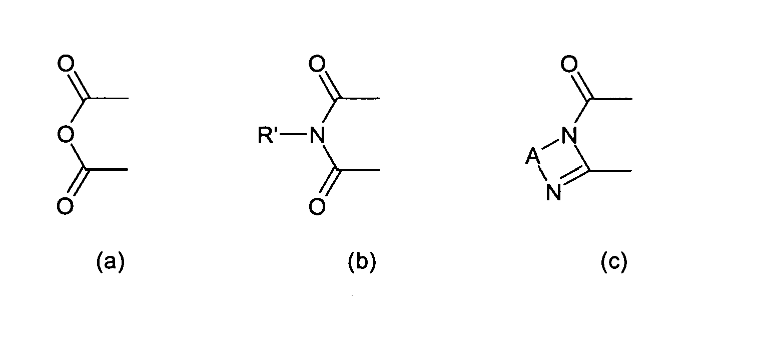 are both a -COOM radical;
are both hydrogen or one of the two radicals is hydrogen and the other radical is halogen or a radical of the formula (d) 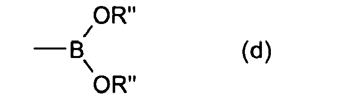
Y are joined to one another with formation of a six-membered ring to give a radical of the formula (a) when one of the two X radicals is hydrogen and the other X radical is halogen or a radical of the formula (d) or when both X radicals are hydrogen or together are a radical of the formula (a), (b) or (c);
are joined to one another with formation of a six-membered ring to give a radical of the formula (b) when one of the two X radicals is hydrogen and the other X radical is halogen or a radical of the formula (d) or when both X radicals are hydrogen or a -COOM radical or together are a radical of the formula (c); are joined to one another with formation of a six-membered ring to give a radical of the formula (c) when one of the two X radicals is hydrogen and the other X radical is halogen or a radical of the formula (d) or when both X radicals are hydrogen or a -COOM radical or together are a radical of the formula (c) which may be arranged in the cis or trans position to the other (c) radical;
are both a -COOM radical when one of the two X radicals is hydrogen and the other X radical is halogen or a radical of the formula (d) or when both X radicals are hydrogen or a -COOM radical, M being different from hydrogen in the case that one X radical is a radical of the formula (d);
are both hydrogen or one of the two radicals is hydrogen and the other radical is halogen or a radical of the formula (d) when both X radicals are hydrogen or one of the two X radicals is hydrogen and the other X radical is halogen or a radical of the formula (d);
R is phenoxy, phenylthio, pyridyloxy, pyrimidyloxy, pyridylthio or pyrimidylthio, each of which may be mono- or polysubstituted by C₁-C₁₂-alkyl, C₁C₁₂-alkoxy and/or aryl;
R¹ is hydrogen;
C₁-C₃₀-alkyl whose carbon chain may be interrupted by one or more -O- and/or -CO-moieties and which may be mono- or polysubstituted by: C₁-C₆-alkoxy, cyano and/or aryl which may be substituted by C₁-C₁₈-alkyl or C₁-C₆-alkoxy; phenyl, naphthyl, pyridyl or pyrimidyl, each of which may be mono- or polysubstituted by: C₁-C₁₈-alkyl, C₁-C₆-alkoxy, halogen, cyano, nitro, -CONR²R³, -SO₂NR²R³, phenyl- and/or naphthylazo, each of which may be substituted by C₁-C₁₀-alkyl, C₁-C₆-alkoxy and/or cyano;
C₅-C₈-cycloalkyl, which may be mono- or polysubstituted by C₁-C₆-alkyl;
A is 1,2-phenylene or 1,8-naphthylene;
M is alkali metal cation;
R" is hydrogen, C₁-C₅-alkyl or pinacolato;
R², R³ are each independently hydrogen;
C₁-C₁₈-alkyl which may be mono- or polysubstituted by C₁-C₆-alkoxy, hydroxyl, halogen and/or cyano;
aryl or hetaryl each of which may be mono- or polysubstituted by C₁-C₆-alkyl and/or the above radicals specified as substituents for alkyl;
m is 2;
n is 4 or 6,
and mixtures thereof.

2. A process for preparing rylenetetracarboxylic monoimide monoanhydrides of the general formula la 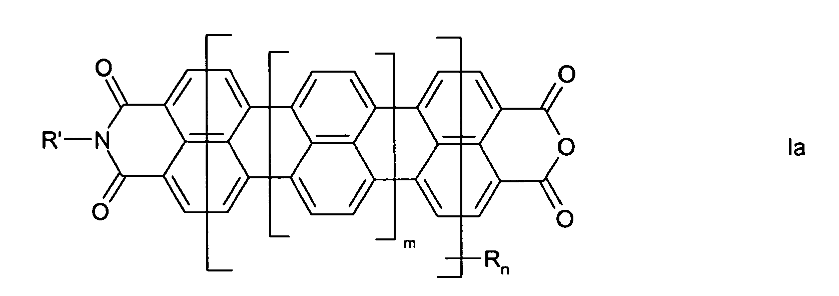 in which the variables are each as defined in claim 1,
which comprises
a) subjecting a rylenetetracarboximide of the general formula II 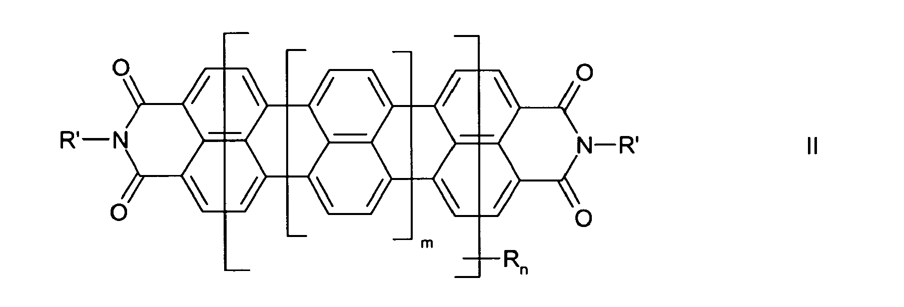 to a hydrolysis under alkaline conditions in the presence of a polar organic solvent and removing the rylenetetracarboxylic monoimide monoanhydride la from the rylenetetracarboxylic dianhydride Ib which is likewise formed
or
b) hydrolyzing the rylenetetracarboximide II by use of mild reaction conditions directly, substantially singly, to the rylenetetracarboxylic monoimide monoanhydride la.

3. A process for preparing rylenetetracarboxylic dianhydrides of the general formula Ib 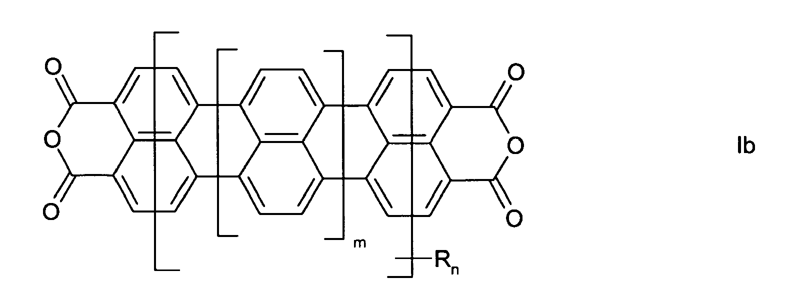 in which the variables are each as defined in claim 1, which comprises
a) subjecting a rylenetetracarboximide of the general formula II 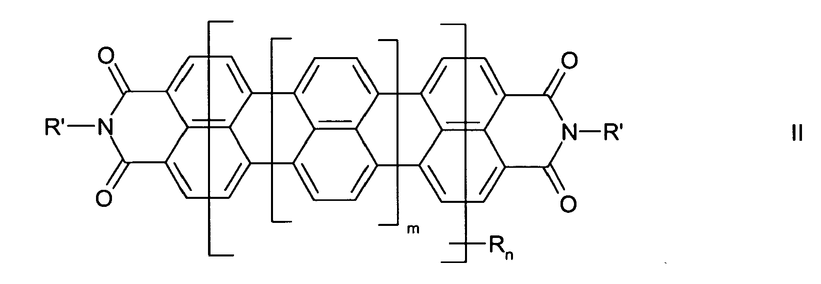 in which the R' radicals are each as defined in claim 1 to a hydrolysis under alkaline conditions in the presence of a polar organic solvent and removing the rylenetetracarboxylic dianhydride Ib from the rylenetetracarboxylic monoimide monoanhydride la which is likewise formed
or
b) hydrolyzing the rylenetetracarboximide II by use of more severe reaction conditions directly, substantially on both sides, to the rylenetetracarboxylic dianhydride Ib.

4. A process for preparing rylenedicarboximides of the general formula Ic 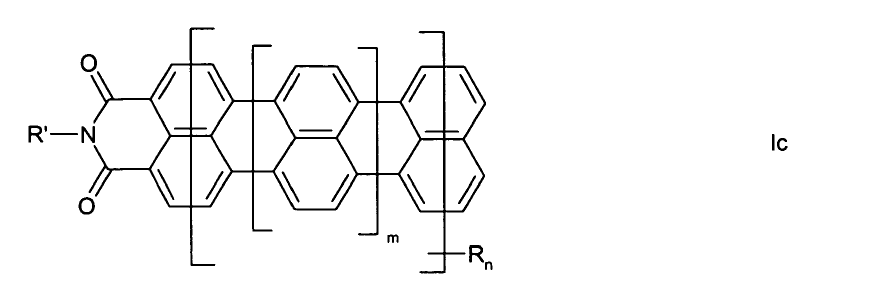 in which the variables are each as defined in claim 1, which comprises
a) subjecting a rylenetetracarboxylic monoimide monoanhydride of the general formula la 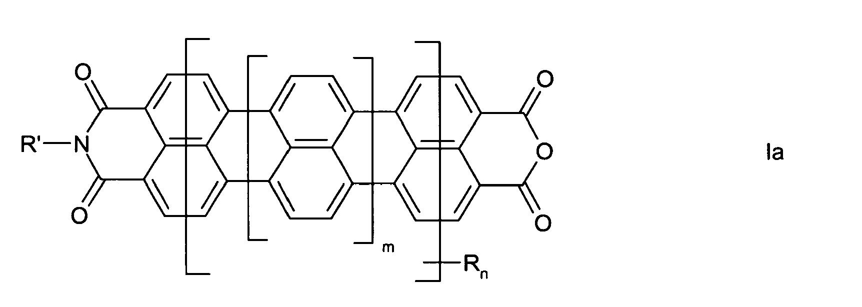
b) subjecting the mixture, obtained in the alkaline hydrolysis of a rylenetetracarboximide of the general formula II 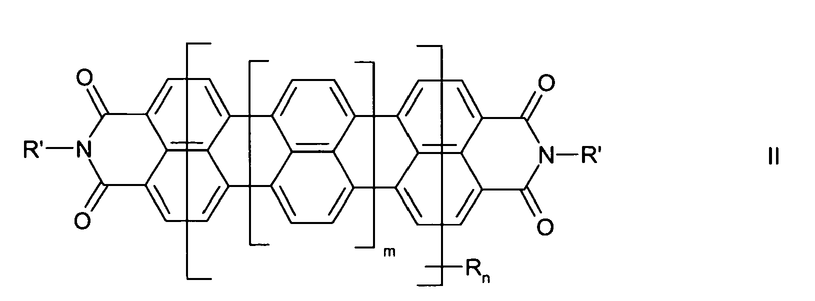 of rylenetetracarboxylic monoimide monoanhydride Ia and rylenetetracarboxylic dianhydride Ib
to a decarboxylation in the presence of a tertiary nitrogen-basic compound as a solvent and of a transition metal catalyst, and, in case b), separating the rylenedicarboximide Ic from the fully decarboxylated rylene Id which likewise forms.

5. A process for preparing rylenes of the general formula Id 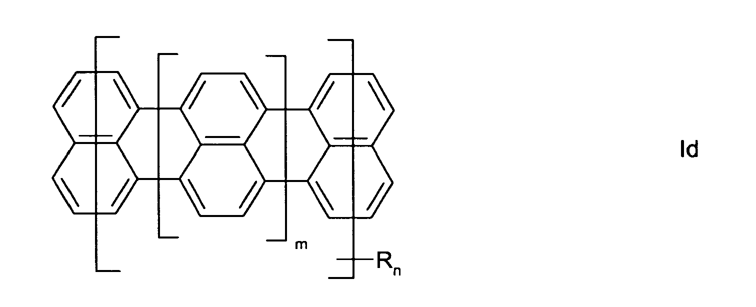 in which the variables are each as defined in claim 1, which comprises
a) subjecting a rylenetetracarboxylic dianhydride of the general formula Ib 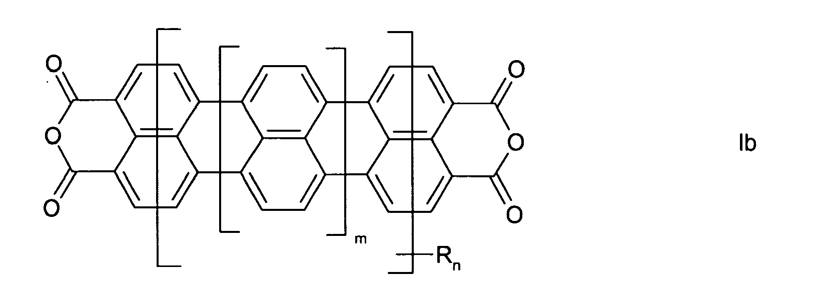 or
b) subjecting the mixture, obtained in the alkaline hydrolysis of a rylenetetracarboximide of the general formula II 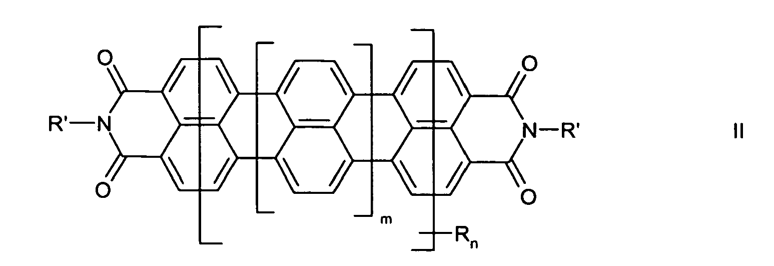
in which the R' radicals are each as defined in claim 1, of rylenetetracarboxylic monoimide monoanhydride Ia and rylenetetracarboxylic dianhydride Ib
to a decarboxylation in the presence of a tertiary nitrogen-basic compound as a solvent and of a transition metal catalyst, and, in case b), separating the rylene Id from the rylenedicarboximide Ic which likewise forms.

6. A process for preparing peri-halogenated rylenedicarboximides of the general formula Ie 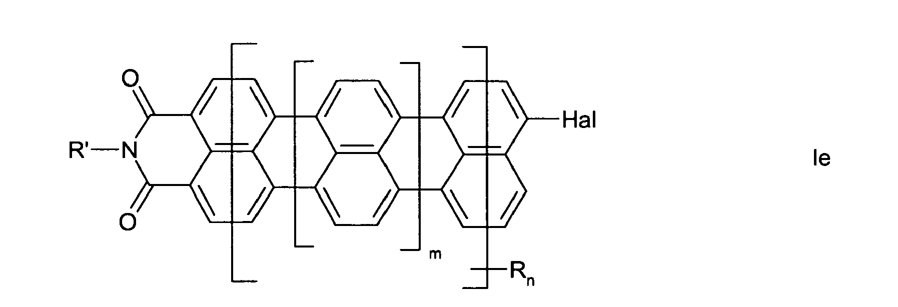 in which Hal is halogen and the further variables are each as defined in claim 1, which comprises
a) reacting a rylenedicarboximide of the general formula Ic 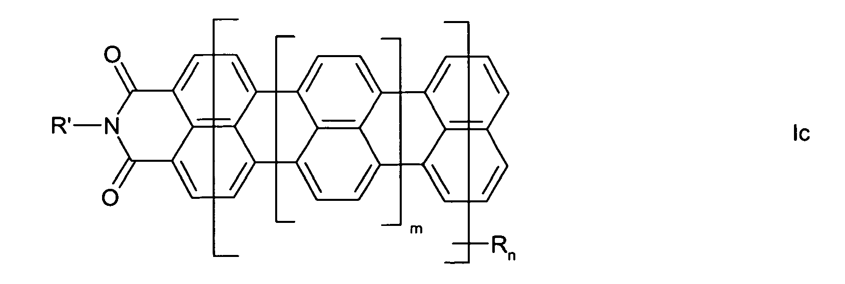 or
b) reacting the mixture of rylenedicarboximide Ic and rylene Id which is obtained in the decarboxylation of the mixture, obtained in the alkaline hydrolysis of the rylenetetracarboximide of the general formula II 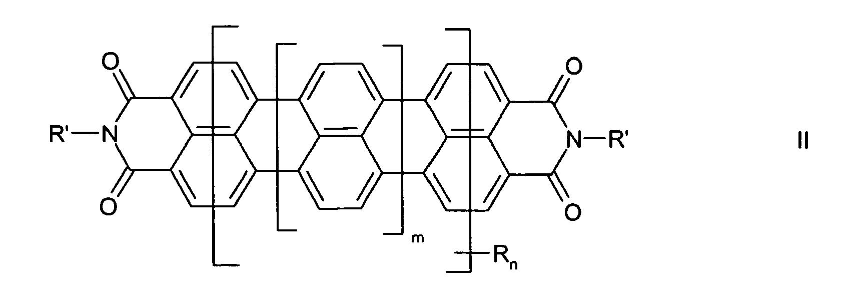 of rylenetetracarboxylic monoimide monoanhydride Ia and rylenetetracarboxylic dianhydride Ib,
in the presence of a polar organic solvent and if desired of a Lewis acid as a catalyst, with from 1 to 6 mol of N-halosuccinimide per halogen atom to be introduced, and, in case b), separating the peri-halogenated rylenedicarboximide Ie from the likewise halogenated rylene If.

7. A process for preparing halogenated rylenes of the general formula If 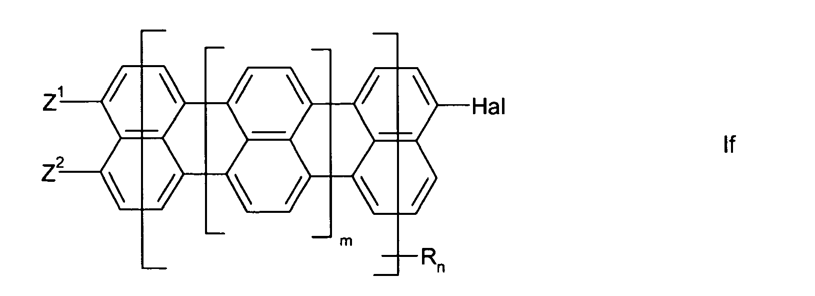 in which Hal is halogen, Z¹ and Z² are each hydrogen or one of the two Z¹ and Z² radicals is halogen and the other radical is hydrogen, and the further variables are each as defined in claim 1, which comprises
a) reacting a rylene of the general formula Id 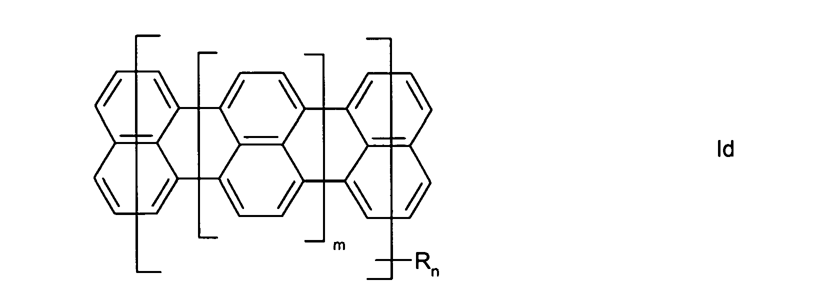
in the presence of a polar organic solvent and of a Lewis acid as a catalyst
a1) directly with the amount, required to introduce the total number of halogen atoms desired, of from 1 to 6 mol of N-halosuccinimide per halogen atom to be introduced
or
a2) first with from 1 to 3 mol/mol of N-halosuccinimide to give the monohalogenated rylene If (Z¹=Z²=H) and then with a further from 1 to 6 mol/mol of N-halosuccinimide to give the dihalogenated rylene If (Z¹ or Z²= halogen)
or
b) reacting the mixture of rylenedicarboximide Ic and rylene Id which is obtained in the decarboxylation of the mixture, obtained in the alkaline hydrolysis of the rylenetetracarboximide of the general formula II 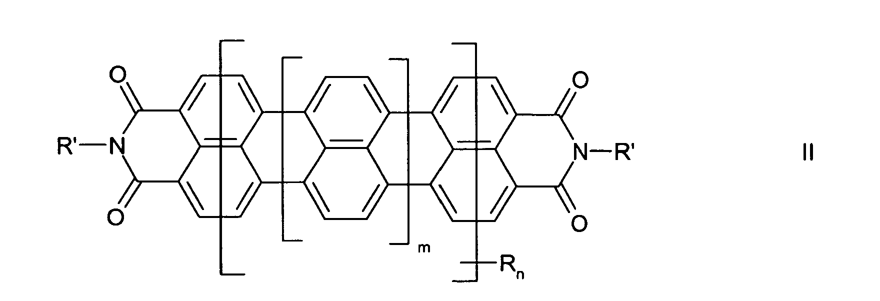 in which the R' radicals are each as defined in claim 1, of rylenetetracarboxylic monoimide monoanhydride Ia and rylenetetracarboxylic dianhydride Ib,
in the presence of a polar organic solvent and of a Lewis acid as a catalyst
b1) directly with the amount, required to introduce the total number of halogen atoms desired, of from 1 to 6 mol of N-halosuccinimide per halogen atom to be introduced
or
b2) first with from 1 to 3 mol of N-halosuccinimide per mole of Ic and Id and separating the monohalogenated rylene If (Z¹=Z²=H) from the peri-halogenated rylenedicarboximide Ie which is likewise formed and then reacting with a further from 1 to 6 mol/mol of N-halosuccinimide per mole to give the dihalogenated rylene If (Z¹ or Z²=halogen).

8. A process for preparing rylenedicarboxylic anhydrides of the general formula Igh 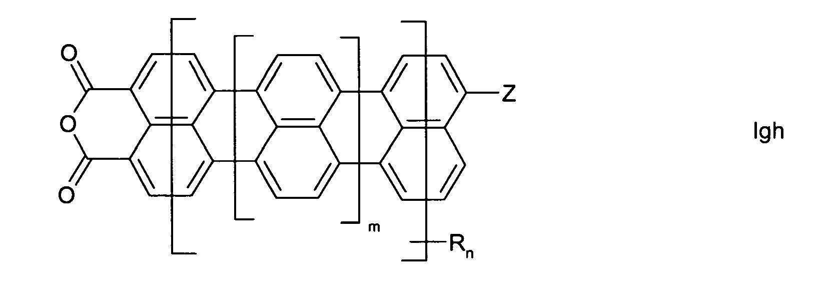 in which Z is hydrogen or halogen and the further variables are each as defined in claim 1, which comprises subjecting a rylenedicarboximide of the general formula Ice 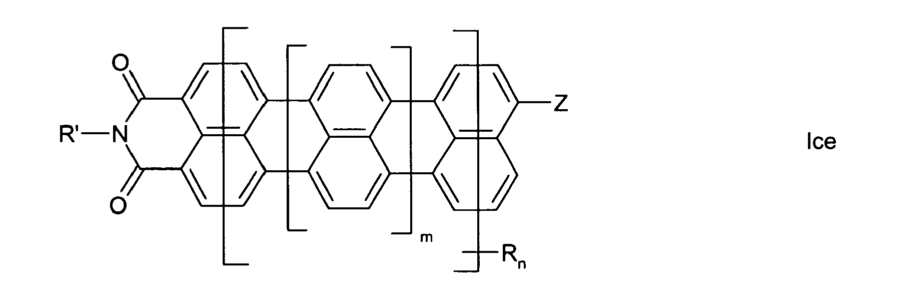 in which R' is as defined in claim 1 to a hydrolysis under alkaline conditions in the presence of a polar organic solvent.

9. A process for preparing peri-halogenated rylenedicarboxylic anhydrides of the general formula Ih 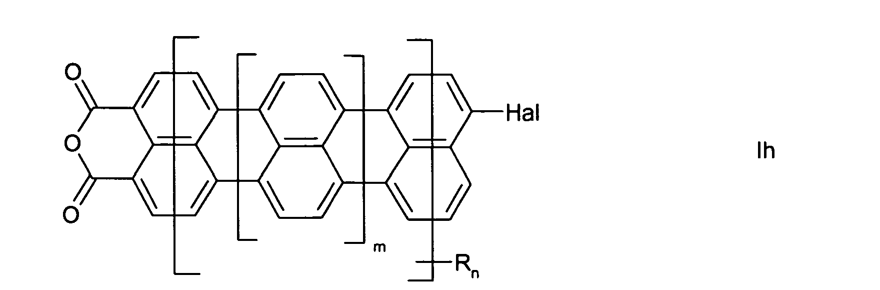 in which Hal is halogen and the further variables are each as defined in claim1, which comprises reacting a rylenedicarboxylic anhydride of the general formula Ig 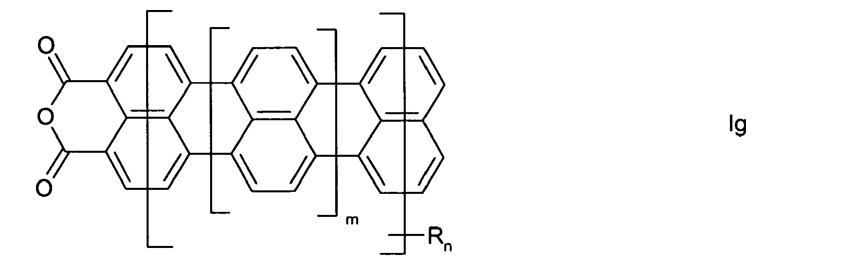 with N-halosuccinimide in the presence of a polar organic solvent and of a Lewis acid.

10. A process for preparing peri-(dioxaborolan-2-yl)rylenedicarboximides of the general formula Ii 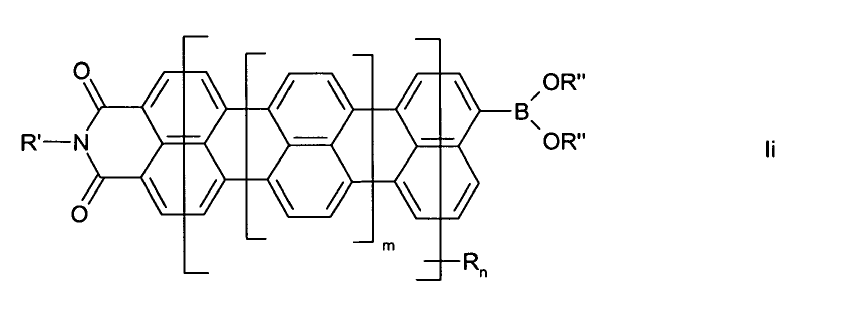 in which the variables are each as defined in claim 1, which comprises reacting a peri-halogenated rylenedicarboximide of the general formula Ie 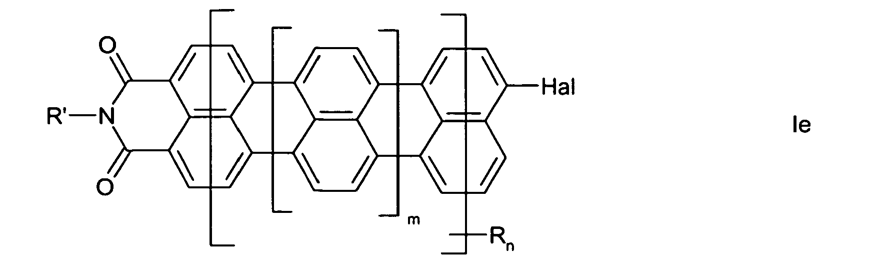 in which Hal is halogen, in the presence of an aprotic organic solvent, of a transition metal catalyst and of a base, with a diborane of the general formula III

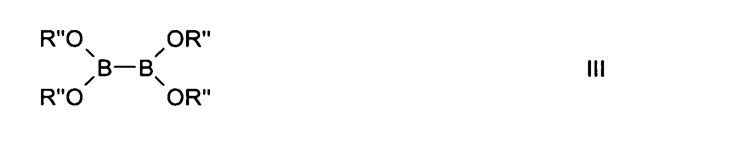

11. A process for preparing substituted rylenes of the general formula Ij 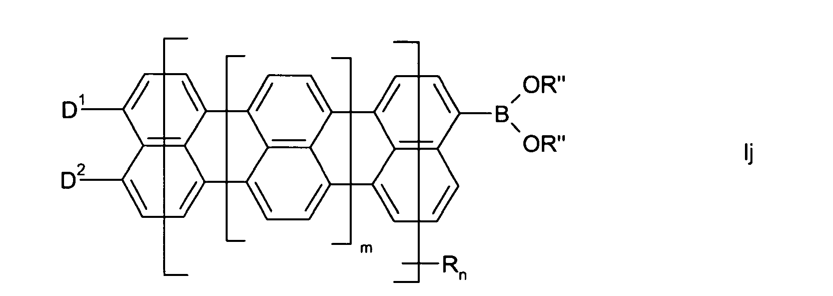 in which D¹ and D² are each hydrogen or one of the two D¹ and D² radicals is halogen or a radical of the formula (d) 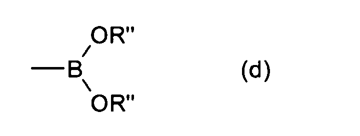 and the other radical is hydrogen and the further variables are each as defined in claim 1, which comprises
a) to prepare mono(dioxaborolan-2-yl)rylenes Ij (D¹=D²=H), reacting a halorylene of the general formula If 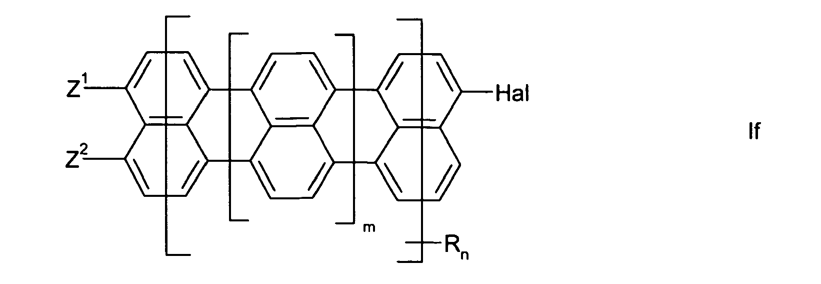 in which Z¹ and Z² are each hydrogen,
or
b) to prepare bis(dioxaborolan-2-yl-)rylenes Ij (one of the two D¹ and D² radicals is a (d) radical and the other radical is hydrogen) or mixed-substituted rylenes Ij (one of the two D¹ and D² radicals is halogen and the other radical is hydrogen), reacting a halorylene of the formula If 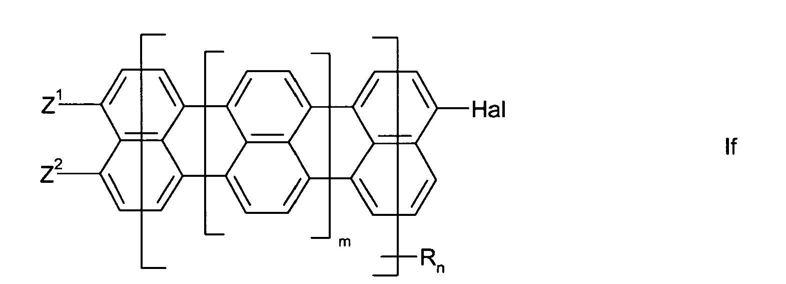
in which one of the two Z¹ and Z² radicals is halogen and the other radical is hydrogen, in the presence of an aprotic organic solvent, of a transition metal catalyst and of a base, with the amount, required to introduce the total number of dioxaborolan-2-yl radicals desired, of from 1 to 3 mol, or, in the case of the mixed-substituted rylenes Ij, from 1 to 1.5 mol, of a diborane of the general formula III 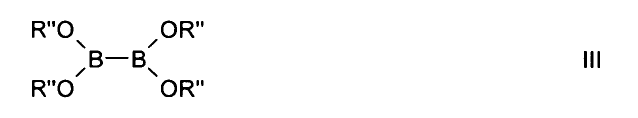 per mole of dioxaborolan-2-yl radical to be introduced.

12. A process for preparing peri-(dioxaborolan-2-yl)rylenedicarboxylic anhydrides of the general formula Ik 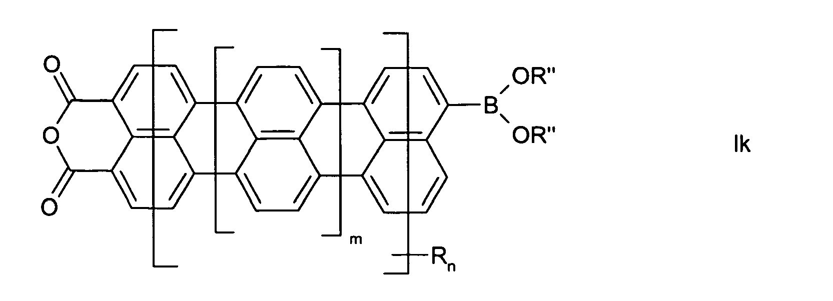 in which the variables are each as defined in claim 1, which comprises reacting a peri-halogenated rylenedicarboxylic anhydride of the general formula Ih 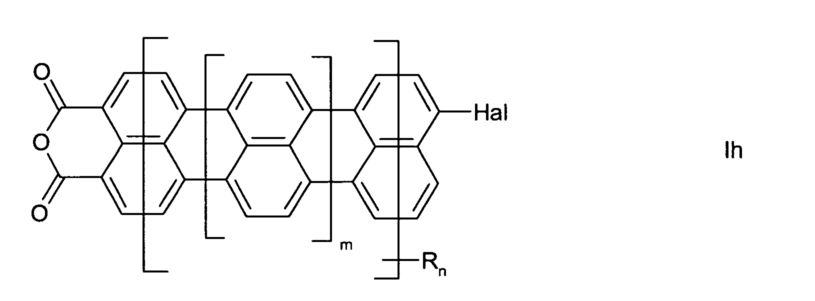 in which Hal is halogen, in the presence of an aprotic organic solvent, of a transition metal catalyst and of a base, with a diborane of the general formula III

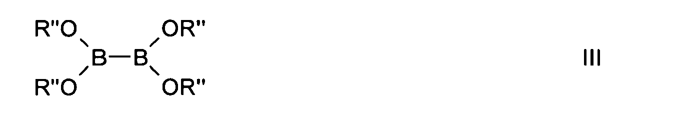

13. A process for preparing symmetrical rylenetetracarboxylic acid derivatives of the general formula Im_{cis} or Imₜᵣₐₙₛ 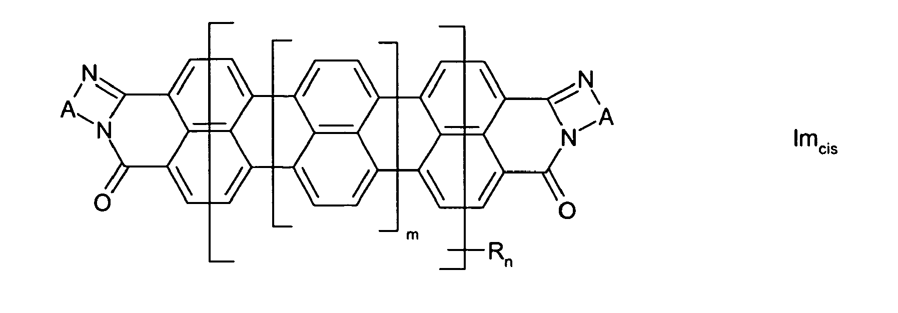 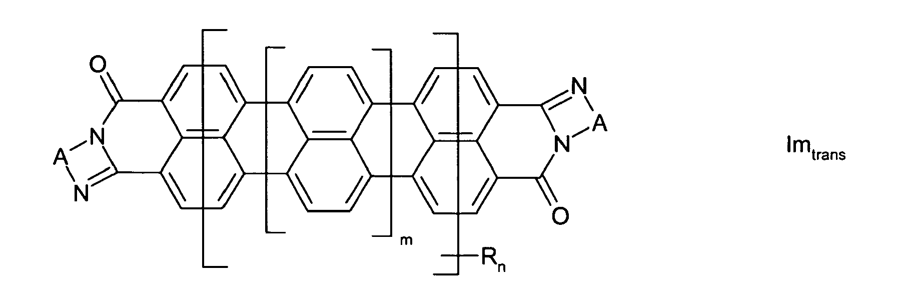 in which the variables are each as defined in claim 1, where the two A radicals are identical, or a mixture of the two isomers, which comprises condensing a rylenetetracarboxylic dianhydride of the general formula Ib 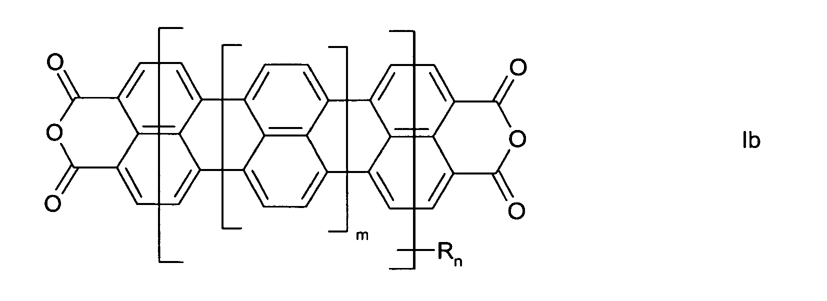 in the presence of a nitrogen-basic compound or of phenol as a solvent and of a Lewis acid, or of piperazine as a catalyst, with from 2 to 3 mol/mol of an aromatic diamine of the general formula IV
H₂N-A-NH₂ IV

14. A process for preparing unsymmetrical rylenetetracarboxylic acid derivatives of the general formula Im'_{cis} or Im'ₜᵣₐₙₛ 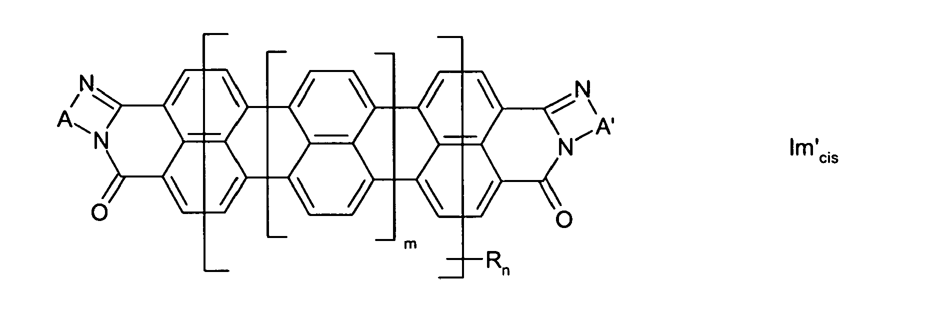 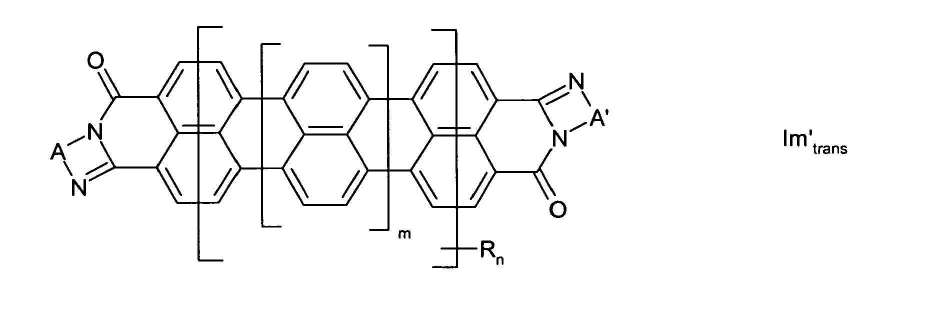 in which the variables are each as defined in claim 1, where the A and A' radicals are different, or a mixture of the two isomers, which comprises condensing a rylenetetracarboxylic dianhydride of the general formula Ib 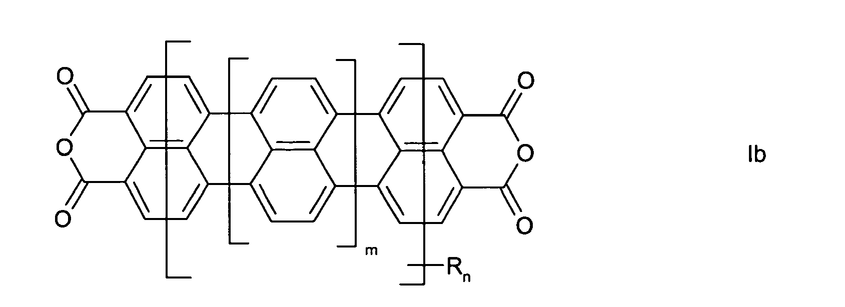 in the presence of a nitrogen-basic compound or of phenol as a solvent and of a Lewis acid or of piperazine as a catalyst, first with from 1 to 1.5 mol/mol of an aromatic diamine of the general formula IV
H₂N-A-NH₂ IV
and then with from 1 to 1.5 mol/mol of an aromatic diamine of the general formula IV'
H₂N-A'-NH₂ IV'

15. A process for preparing rylenetetracarboxylic acid derivatives of the general formula In 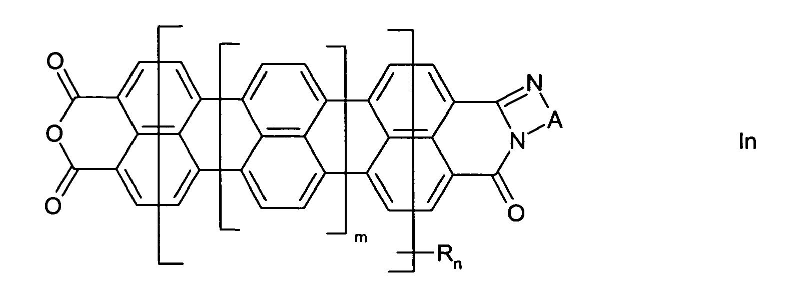 in which the variables are each as defined in claim 1, which comprises condensing a rylenetetracarboxylic dianhydride of the general formula Ib 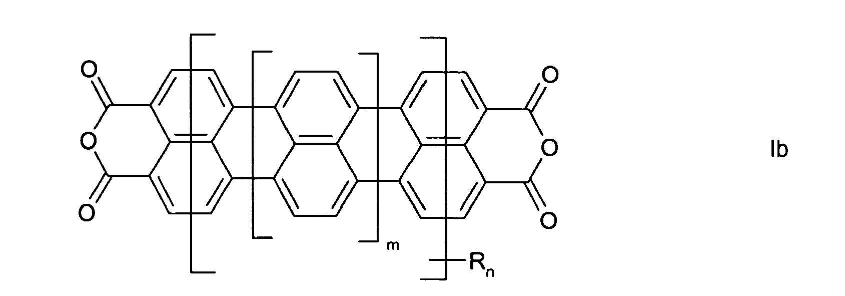 the presence of a nitrogen-basic compound or of phenol as a solvent and of a Lewis acid or of piperazine as a catalyst, with from 1 to 1.5 mol/mol of an aromatic diamine of the general formula IV
H₂N-A-NH₂ IV

16. A process for preparing rylenetetracarboxylic acid derivatives of the general formula Io 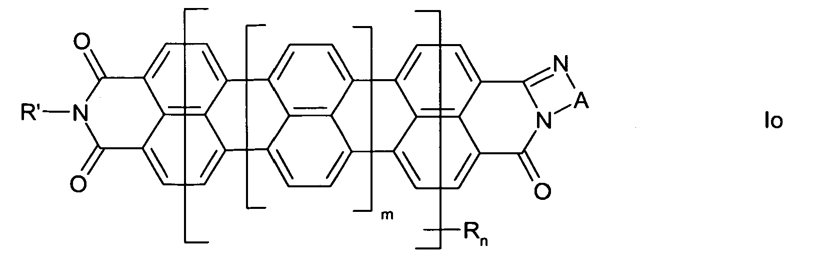 in which the variables are each as defined in claim 1, which comprises condensing a rylenetetracarboxylic monoimide monoanhydride of the general formula la 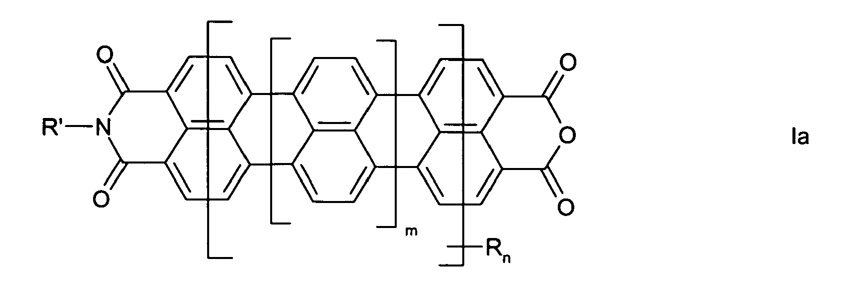 in the presence of a nitrogen-basic compound or of phenol as a solvent and of a Lewis acid or of piperazine as a catalyst, with from 1 to 1.5 mol/mol of an aromatic diamine of the general formula IV
H₂N-A-NH₂ IV

17. A process for preparing rylenedicarboxylic acid derivatives of the general formula Ip 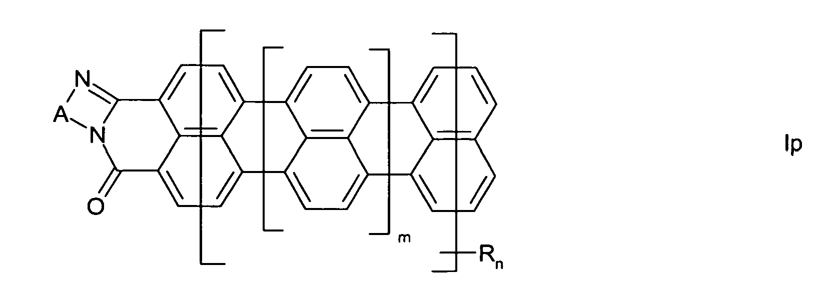 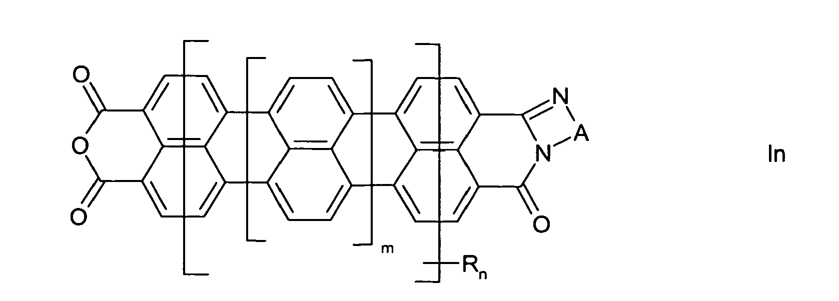 in which the variables are each as defined in claim 1, which comprises
a) subjecting a rylenetetracarboxylic acid derivative of the general formula In 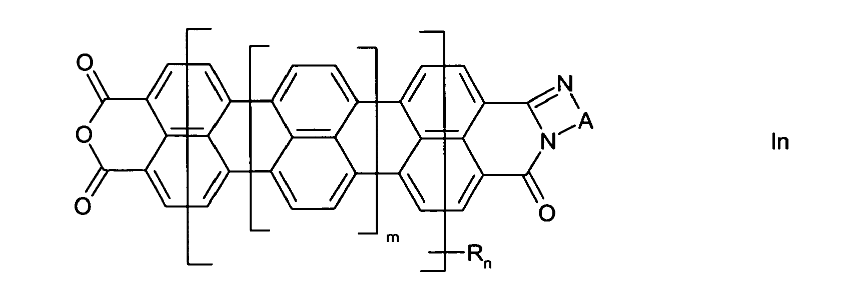 to a decarboxylation in the presence of a tertiary nitrogen-basic compound and of a transition metal catalyst
or
b) condensing a rylenedicarboxylic anhydride of the general formula Ig 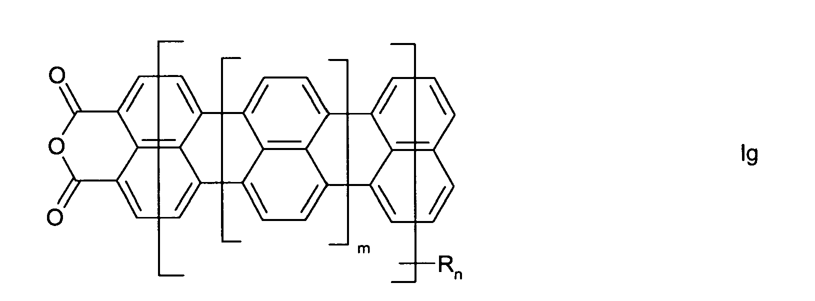
in the presence of a nitrogen-basic compound or of phenol as a solvent and of a Lewis acid or of piperazine as a catalyst, with from 1 to 1.5 mol/mol of an aromatic diamine of the general formula IV
H₂N-A-NH₂ IV

18. A process for preparing peri-halogenated rylenedicarboxylic acid derivatives of the general formula Iq 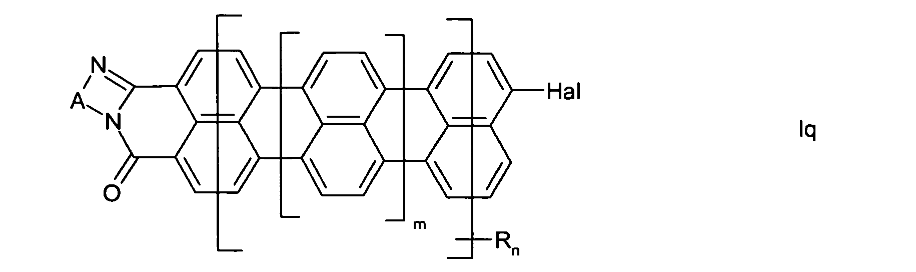 in which the variables are each as defined in claim 1, which comprises reacting a rylenedicarboxylic acid derivative of the general formula Ip 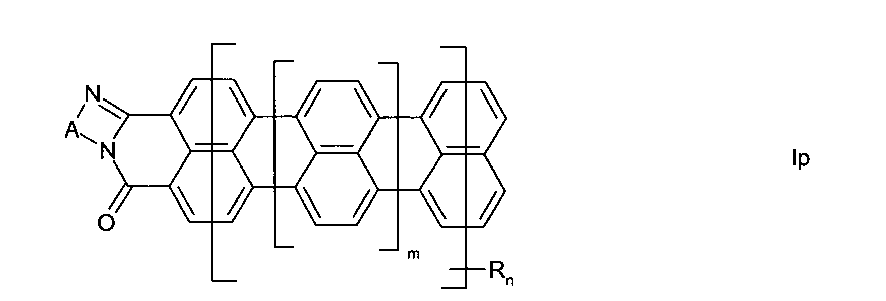 with N-halosuccinimide in the presence of a polar organic solvent and of a Lewis acid as a catalyst.

19. A process for preparing peri-(dioxaborolan-2-yl)rylenedicarboxylic acid derivatives of the general formula Ir 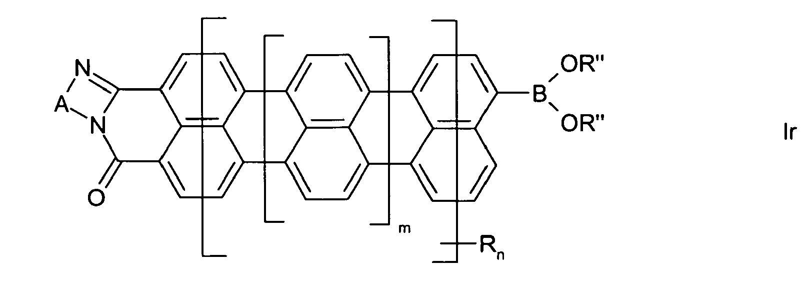 in which the variables are each as defined in claim 1, which comprises reacting a peri-halogenated rylenedicarboxylic acid derivative of the general formula Iq 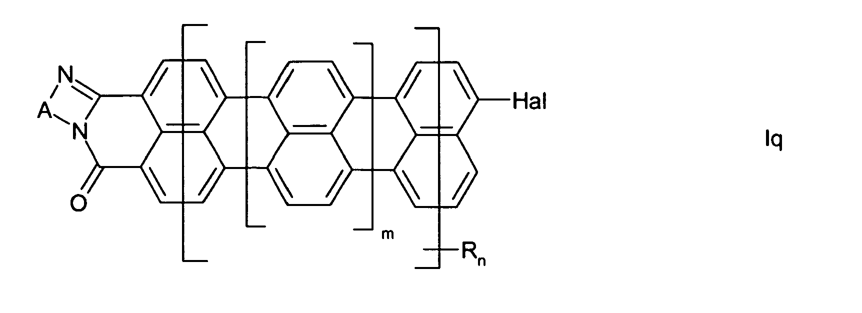 in which Hal is halogen, in the presence of an aprotic organic solvent, of a transition metal catalyst and of a base, with a diborane of the general formula III

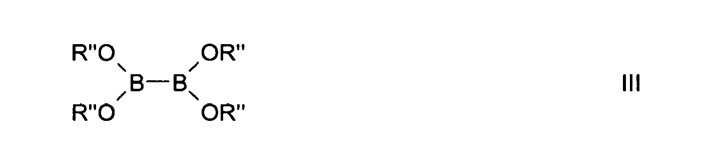

20. The use of rylene derivatives of the formula I according to claims 1 to 3 for coloring high molecular weight organic and inorganic materials.

21. The use according to claim 22, wherein the high molecular weight materials are coatings, printing inks or plastics.

22. The use of rylene derivatives of the formula I according to claims 1 to 3 as dispersing assistants and pigment additives for organic pigments.

23. The use of rylene derivatives of the formula I according to claims 1 to 3 for producing aqueous polymer dispersions which absorb in the near infrared region of the electromagnetic spectrum.

24. The use of rylene derivatives of the formula I according to claims 1 to 3 for obtaining markings and inscriptions which absorb infrared light and are invisible to the human eye.

25. The use of rylene derivatives of the formula I according to claims 1 to 3 as infrared absorbers for heat management.

26. The use of rylene derivatives of the formula I according to claims 1 to 3 as IR laser beam-absorbing materials in the fusion treatment of plastics parts.

27. The use of rylene derivatives of the formula I according to claims 1 to 3 as semiconductors in organic electronics.

28. The use of rylene derivatives of the formula I according to claims 1 to 3 as emitters in electro- and chemiluminescence applications.

29. The use of rylene derivatives of the formula I according to claims 1 to 3 as active components in photovoltaics.

## Revendications

1. Dérivés de rylène de formule générale I 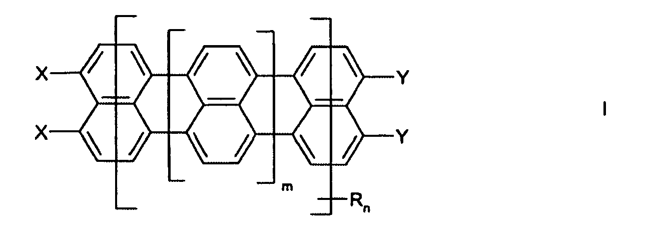 dans laquelle les variables ont la signification suivante :
X signifient, liés l'un à l'autre en formant un noyau hexagonal, un radical de formule (a), (b) ou (c) 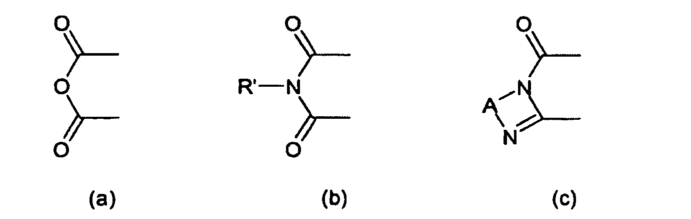 tous les deux un radical -COOM ;
tous les deux hydrogène ou un des deux radicaux signifie hydrogène et l'autre radical signifie halogène ou un radical de formule (d) 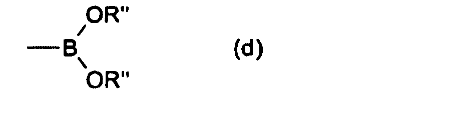
Y signifient, liés l'un à l'autre en formant un noyau hexagonal, un radical de formule (a), lorsqu'un des deux radicaux X signifie hydrogène et l'autre radical X signifie halogène ou un radical de formule (d) ou lorsque les deux radicaux X signifient hydrogène ou signifient ensemble un radical de formule (a), (b) ou (c) ; liés l'un à l'autre en formant un noyau hexagonal, un radical de formule (b), lorsqu'un des deux radicaux X signifie hydrogène et l'autre radical X signifie halogène ou un radical de formule (d) ou lorsque les deux radicaux X signifient hydrogène ou un radical -COOM ou signifient ensemble un radical de formule (c) ;
liés l'un à l'autre en formant un noyau hexagonal, un radical de formule (c), lorsqu'un des deux radicaux X signifie hydrogène et l'autre radical X signifie halogène ou un radical de formule (d) ou lorsque les deux radicaux X signifient hydrogène ou un radical -COOM ou signifient ensemble un radical de formule (c), qui peut être disposé en position cis ou trans par rapport à l'autre radical (c) ;
tous les deux un radical -COOM, lorsqu'un des deux radicaux X signifie hydrogène et l'autre radical X signifie halogène ou un radical de formule (d) ou lorsque les deux radicaux X signifient hydrogène ou un radical -COOM, où, dans le cas où un radical X signifie un radical de formule (d), M est différent d'hydrogène ;
tous les deux hydrogène ou un des deux radicaux signifie hydrogène et l'autre radical signifie halogène ou un radical de formule (d), lorsque les deux radicaux X signifient hydrogène ou un des deux radicaux X signifie hydrogène et l'autre radical X signifie halogène ou un radical de formule (d) ;
R signifie phénoxy, phénylthio, pyridyloxy, pyrimidyloxy, pyridylthio ou pyrimidylthio, qui peut à chaque fois être monosubstitué ou polysubstitué par C₁-C₁₂-alkyle, C₁-C₁₂-alcoxy et/ou aryle ;
R' signifie hydrogène ;
C₁-C₃₀-alkyle, dont la chaîne carbonée peut être interrompue par un ou plusieurs groupements -O-et/ou -CO- et qui peut être monosubstitué ou polysubstitué par : C₁-C₆-alcoxy, cyano et/ou aryle, qui peut être substitué par C₁-C₁₈-alkyle ou C₁-C₆-alcoxy ;
phényle, naphtyle, pyridyle ou pyrimidyle, qui peut être à chaque fois monosubstitué ou polysubstitué par : C₁-C₁₈-alkyle, C₁-C₆-alcoxy, halogène, cyano, nitro, -CONR²R³, -SO₂NR²R³, phénylazo et/ou naphtylazo, qui peut à chaque fois être substitué par C₁-C₁₀-alkyle, C₁-C₆-alcoxy et/ou cyano ;
C₅-C₈-cycloalkyle, qui peut être monosubstitué ou polysubstitué par C₁-C₆-alkyle ;
A signifie 1,2-phénylène ou 1,8-naphtylène ;
M signifie un cation de métal alcalin ;
R" signifient hydrogène, C₁-C₆-alkyle ou pinacolato ;
R², R³ indépendamment l'un de l'autre, signifient hydrogène ;
C₁-C₁₈-alkyle, qui peut être monosubstitué ou polysubstitué par C₁-C₆-alcoxy, hydroxy, halogène et/ou cyano ;
aryle ou hétaryle, qui peut être à chaque fois monosubstitué ou polysubstitué par C₁-C₆-alkyle et/ou les radicaux ci-dessus, mentionnés comme substituants pour alkyle ;
m vaut 2 ;
n vaut 4 ou 6,
et leurs mélanges.

2. Procédé pour la préparation de mono-imido-monoanhydrides de l'acide rylènetétracarboxylique de formule générale Ia, 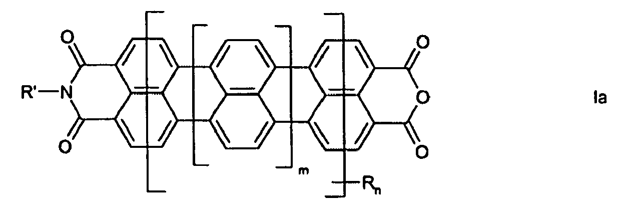 dans laquelle les variables présentent la signification mentionnée dans la revendication 1, **caractérisé en ce qu'**on
a) soumet un diimide de l'acide rylènetétracarboxylique de formule générale II 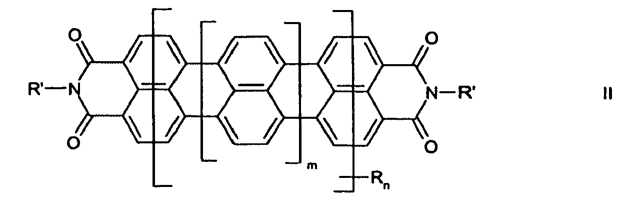 à une saponification dans des conditions alcalines en présence d'un solvant organique polaire, puis on sépare le mono-imido-monoanhydride de l'acide rylènetétracarboxylique Ia du dianhydride de l'acide rylènetétracarboxylique Ib qui se forme également ou
b) saponifie le diimide de l'acide rylènetétracarboxylique II par l'utilisation de conditions de réaction douces directement, dans une large mesure, d'un côté en mono-imido-monoanhydride de l'acide rylènetétracarboxylique Ia.

3. Procédé pour la préparation de dianhydrides de l'acide rylènetétracarboxylique de formule générale Ib 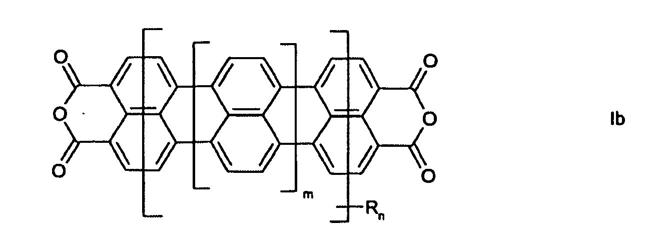 dans laquelle les variables présentent la signification mentionnée dans la revendication 1, **caractérisé en ce qu'**on
a) soumet un diimide de l'acide rylènetétracarboxylique de formule générale II 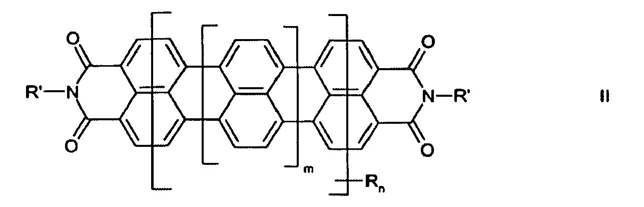 dans laquelle les radicaux R' ont la signification mentionnée dans la revendication 1 à une saponification dans des conditions alcalines en présence d'un solvant organique polaire, puis on sépare le dianhydride de l'acide rylènetétracarboxylique Ib du mono-imido-monoanhydride de l'acide rylènetétracarboxylique Ia qui se forme également ou
b) saponifie le diimide de l'acide rylènetétracarboxylique II par l'utilisation de conditions de réaction plus dures, directement dans une large mesure des deux côtés en dianhydride de l'acide rylènetétracarboxylique Ib.

4. Procédé pour la préparation d'imides de l'acide rylènedicarboxylique de formule générale Ic 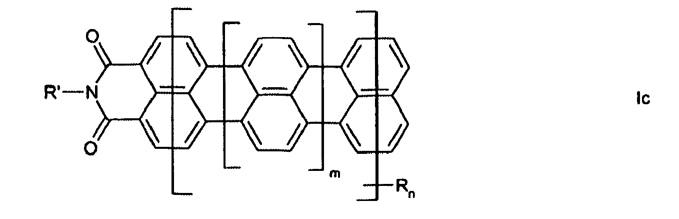 dans laquelle les variables présentent la signification mentionnée dans la revendication 1, **caractérisé en ce qu'**on
a) un mono-imido-monoanhydride de l'acide rylènetétracarboxylique de formule générale Ia 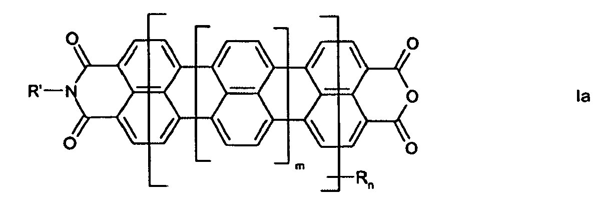 ou
b) le mélange de mono-imido-monoanhydride de l'acide rylènetétracarboxylique Ia et de dianhydride de l'acide rylènetétracarboxylique Ib produit lors de la saponification alcaline d'un diimide de l'acide rylènetétracarboxylique de formule générale II 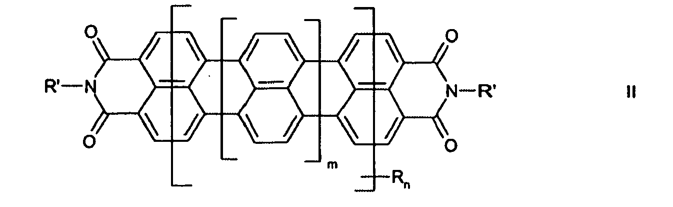 à une décarboxylation en présence d'un composé tertiaire basique d'azote comme solvant et d'un catalyseur de métal de transition et, dans le cas b), on sépare l'imide de l'acide rylènedicarboxylique Ic du rylène complètement décarboxylé Id qui se forme également.

5. Procédé pour la préparation de rylènes de formule générale Id 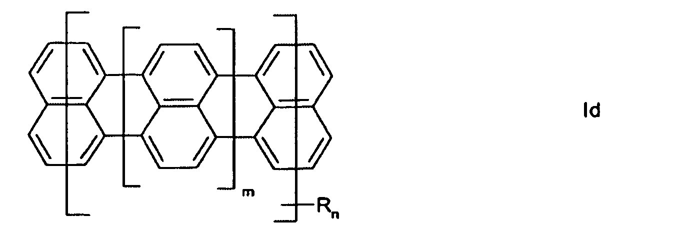 dans laquelle les variables présentent la signification mentionnée dans la revendication 1, **caractérisé en ce qu'**on
a) un dianhydride de l'acide rylènetétracarboxylique de formule générale Ib 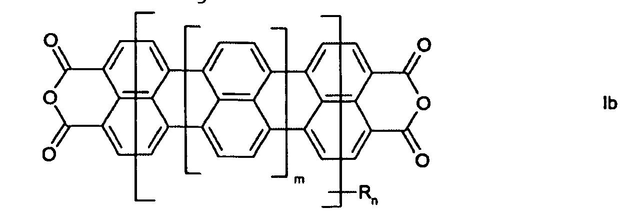 ou
b) le mélange de mono-imido-monoanhydride de l'acide rylènetétracarboxylique Ia et de dianhydride de l'acide rylènetétracarboxylique Ib produit lors de la saponification alcaline d'un diimide de l'acide rylènetétracarboxylique de formule générale II 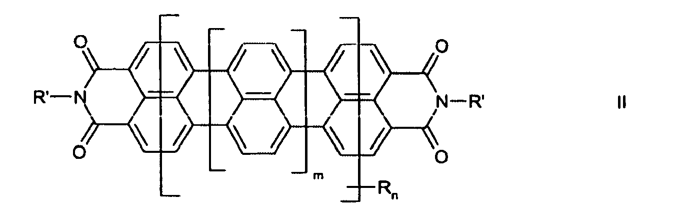 dans laquelle les radicaux R' ont la signification mentionnée dans la revendication 1,
à une décarboxylation en présence d'un composé tertiaire basique d'azote comme solvant et d'un catalyseur de métal de transition et, dans le cas b), on sépare le rylène Id de l'imide de l'acide rylènedicarboxylique Ic qui se forme également.

6. Procédé pour la préparation d'imides de l'acide rylènedicarboxylique périhalogéné de formule générale Ie 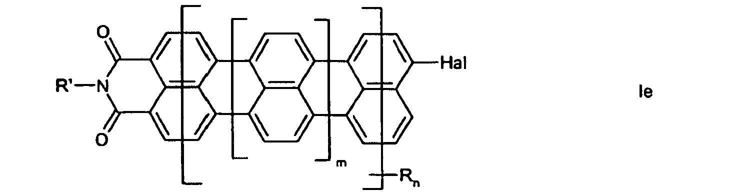 dans laquelle Hal signifie halogène et les autres variables présentent la signification mentionnée dans la revendication 1, **caractérisé en ce qu'**on transforme
a) un imide de l'acide rylènedicarboxylique de
formule générale Ic 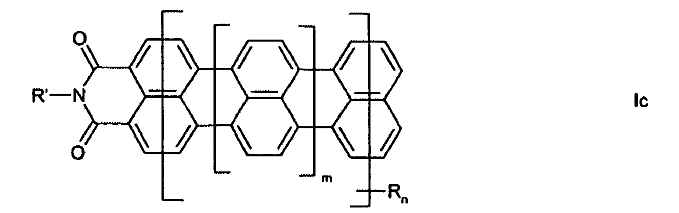 ou
b) le mélange d'imide de l'acide rylènedicarboxylique Ic et de rylène Id, qui se forme lors de la décarboxylation du mélange de mono-imido-monoanhydride de l'acide rylènetétracarboxylique Ia et de dianhydride de l'acide rylènetétracarboxylique Ib qui est produit lors de la saponification alcaline du diimide de l'acide rylènetétracarboxylique de formule générale II 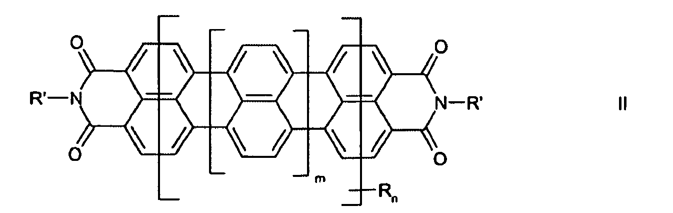 en présence d'un solvant organique polaire et si souhaité d'un acide de Lewis comme catalyseur avec 1 à 6 moles de N-halogénosuccinimide par atome d'halogène à introduire et, dans le cas b), on sépare l'imide de l'acide rylènedicarboxylique périhalogéné Ie du rylène également halogéné If.

7. Procédé pour la préparation de rylènes halogénés de formule générale If 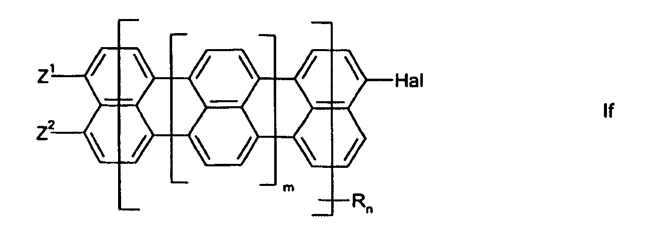 dans laquelle Hal signifie halogène, Z¹ et Z² signifient hydrogène ou un des deux radicaux Z¹ ou Z² signifie halogène et l'autre radical signifie hydrogène et les autres variables présentent la signification mentionnée dans la revendication 1, **caractérisé en ce qu'**on transforme
a) un rylène de formule générale Id 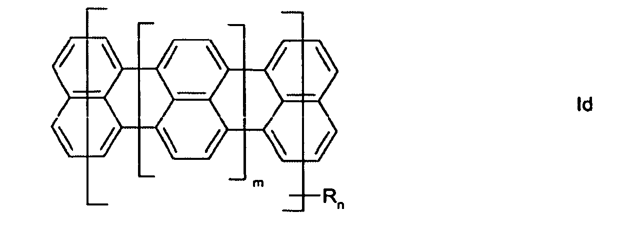 en présence d'un solvant organique polaire et d'un acide de Lewis comme catalyseur
a1) directement avec une quantité nécessaire pour l'introduction du nombre total d'atomes d'halogène de 1 à 6 moles de N-halogénosuccinimide par atome d'halogène à introduire ou
a2) d'abord avec 1 à 3 moles/mole de N-halogénosuccinimide en rylène monohalogéné If (Z¹=Z²=H) puis avec 1 à 6 moles supplémentaires/mole de N-halogénosuccinimide en rylène dihalogéné If (Z¹ ou Z²=halogène)
ou
b) le mélange d'imide de l'acide rylènedicarboxylique Ic et de rylène Id, qui se forme lors de la décarboxylation du mélange de mono-imido-monoanhydride de l'acide rylènetétracarboxylique Ia et de dianhydride de l'acide rylènetétracarboxylique Ib qui est produit lors de la saponification alcaline du diimide de l'acide rylènetétracarboxylique de formule générale II 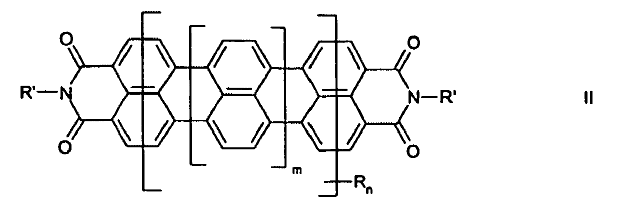 dans laquelle les radicaux R' ont la signification mentionnée dans la revendication 1,
en présence d'un solvant organique polaire et d'un acide de Lewis comme catalyseur
b1) directement avec la quantité nécessaire pour l'introduction du nombre total souhaité d'atomes d'halogène de 1 à 6 moles de N-halogénosuccinimide par atome d'halogène à introduire ou
b2) d'abord avec 1 à 3 moles/mole de N-halogénosuccinimide par mole de Id et de Ic, puis on sépare le rylène monohalogéné If (Z¹=Z²=H) de l'imide de l'acide rylènedicarboxylique périhalogéné Ie également formé, puis avec 1 à 6 moles supplémentaires de N-halogénosuccinimide/mole en rylène dihalogéné If (Z¹ ou Z²=halogène)

8. Procédé pour la préparation d'anhydrides de l'acide rylènedicarboxylique de formule générale Igh 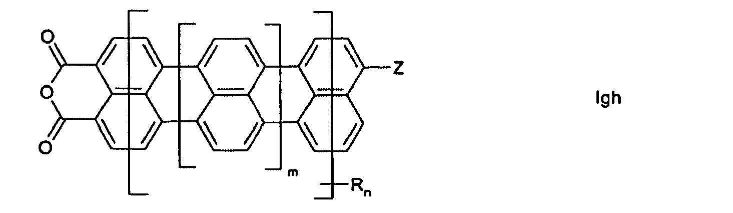 dans laquelle Z signifie hydrogène ou halogène et les autres variables présentent la signification mentionnée dans la revendication 1, **caractérisé en ce qu'**on soumet un imide de l'acide rylènedicarboxylique de formule générale Ice 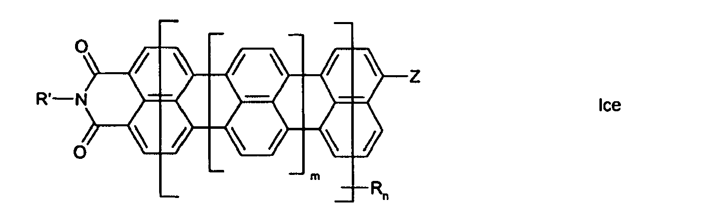 dans laquelle R' présente la signification mentionnée dans la revendication 1 à une saponification dans des conditions alcalines en présence d'un solvant organique polaire.

9. Procédé pour la préparation d'anhydrides de l'acide rylènedicarboxylique périhalogéné de formule générale Ih 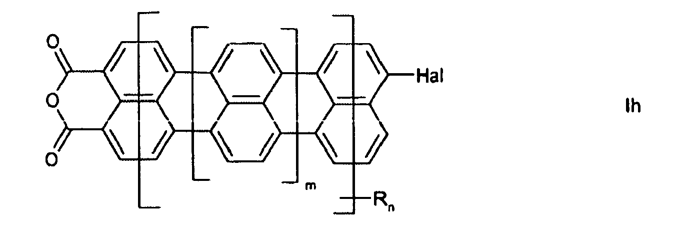 dans laquelle Hal signifie halogène et les autres variables présentent la signification mentionnée dans la revendication 1, **caractérisé en ce qu'**on transforme un anhydride de l'acide rylènedicarboxylique de formule générale Ig 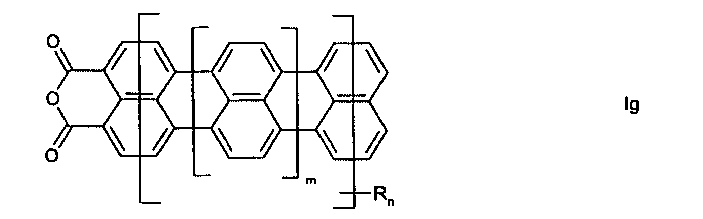 en présence d'un solvant organique polaire et d'un acide de Lewis avec du N-halogénosuccinimide.

10. Procédé pour la préparation d'imides de l'acide péri-(dioxaborolan-2-yl)rylènedicarboxylique de formule générale Ii 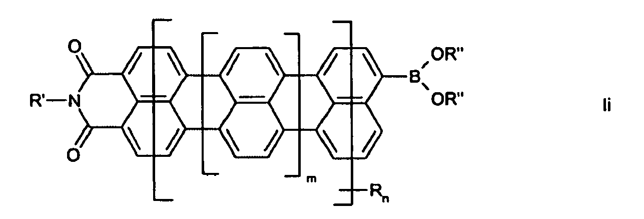 dans laquelle les variables présentent la signification mentionnée dans la revendication 1, **caractérisé en ce qu'**on transforme
un imide de l'acide rylènedicarboxylique périhalogéné de formule générale Ie 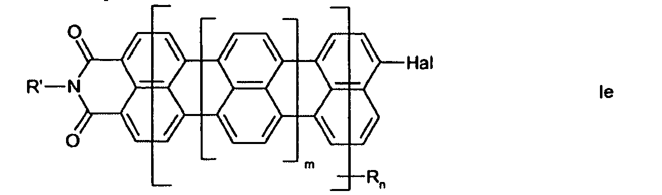 dans laquelle Hal signifie halogène, en présence d'un solvant organique aprotique, d'un catalyseur de métal de transition et d'une base avec un diborane de formule générale III

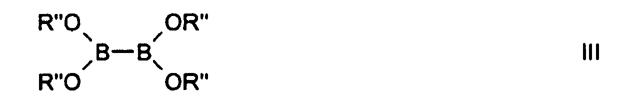

11. Procédé pour la préparation de rylènes
substitués de formule générale Ij 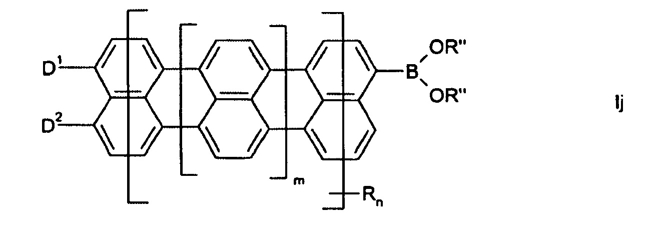 dans laquelle D¹ et D² signifient hydrogène ou un des deux radicaux D¹ ou D² signifie halogène ou un radical de formule (d) 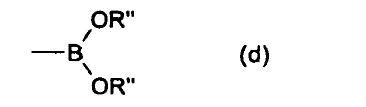 et l'autre radical signifie hydrogène et les autres variables présentent la signification mentionnée dans la revendication 1, **caractérisé en ce qu'**on transforme
a) pour la préparation de mono(dioxaborolan-2-yl)rylènes Ij (D¹=D²=H), un halogénorylène de formule générale If 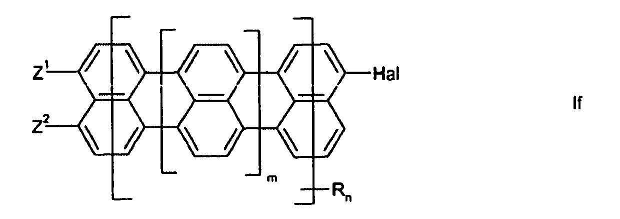 dans laquelle Z¹ et Z² signifient hydrogène, ou
b) pour la préparation de bis(dioxaborolan-2-yl)rylènes Ij (un des deux radicaux D¹ ou D² signifie un radical (d) et l'autre radical signifie hydrogène) ou de rylènes Ij substitués de manière mixte (un des deux radicaux D¹ ou D² signifie halogène et l'autre radical signifie hydrogène), un halogénorylène de formule If 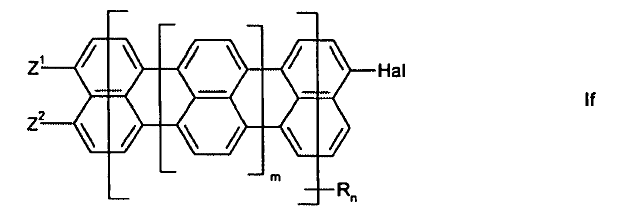 dans laquelle un des deux radicaux Z¹ ou Z² signifie halogène ou l'autre radical signifie hydrogène,
en présence d'un solvant organique aprotique, d'un catalyseur de métal de transition et d'une base avec la quantité nécessaire pour l'introduction du nombre total souhaité de radicaux dioxaborolan-2-yle de 1 à 3 moles ou, selon le cas, dans le cas des rylènes substitués de manière mixte Ij, de 1 à 1,5 mole d'un diborane de formule III 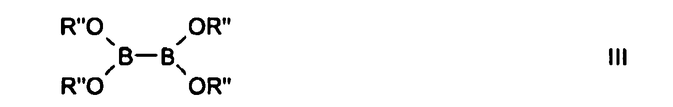 par mole de radical dioxaborolan-2-yle.

12. Procédé pour la préparation d'anhydrides de l'acide péri-(dioxaborolan-2-yl)rylènedicarboxylique de formule générale Ik 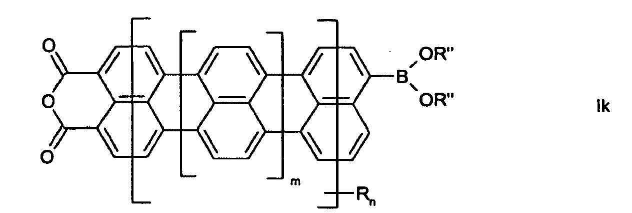 dans laquelle les variables présentent la signification mentionnée dans la revendication 1, **caractérisé en ce qu'**on transforme
un anhydride de l'acide rylènedicarboxylique périhalogéné de formule générale Ih 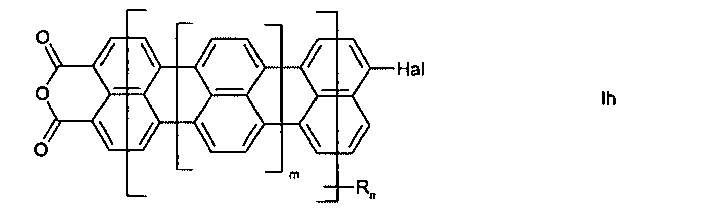 dans laquelle Hal signifie halogène, en présence d'un solvant organique aprotique, d'un catalyseur de métal de transition et d'une base avec un diborane de formule générale III

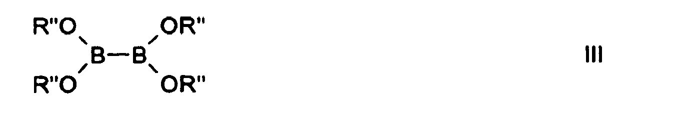

13. Procédé pour la préparation de dérivés symétriques de l'acide rylènetétracarboxylique des formules générales Im_{cis} ou Imₜᵣₐₙₛ 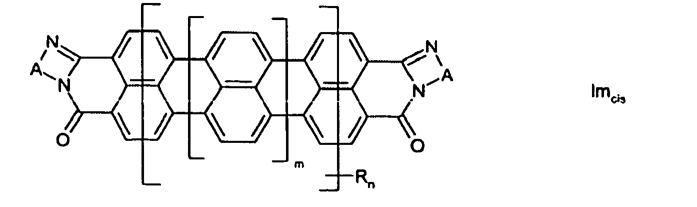 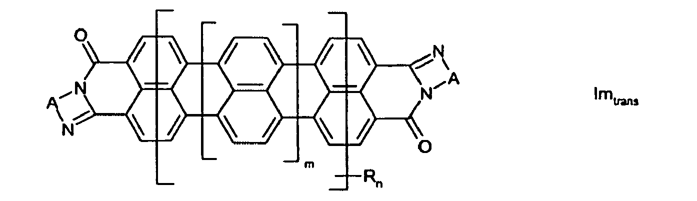 dans laquelle les variables présentent la signification mentionnée dans la revendication 1, les deux radicaux A étant identiques ou d'un mélange des deux isomères, **caractérisé en ce qu'**on condense un dianhydride de l'acide rylènetétracarboxylique de formule générale Ib 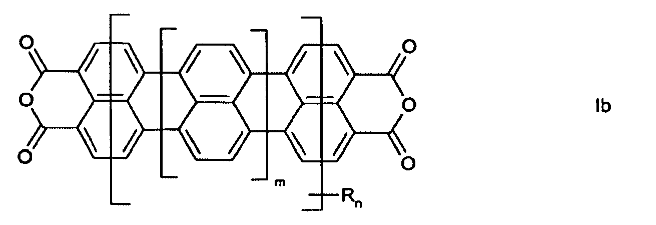 en présence d'un composé basique d'azote ou de phénol comme solvant et d'un acide de Lewis ou de pipérazine comme catalyseur, avec 2 à 3 moles/mole d'une diamine aromatique de formule générale IV
H₂N-A-NH₂ IV

14. Procédé pour la préparation de dérivés asymétriques de l'acide rylènetétracarboxylique des formules générales Im'_{cis} ou Im'ₜᵣₐₙₛ 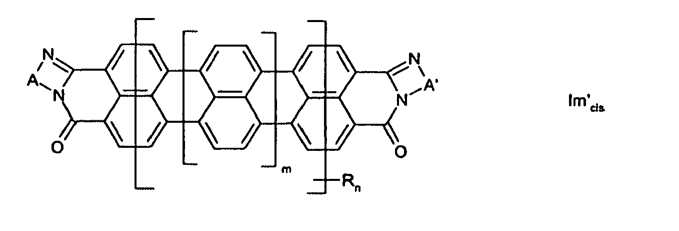 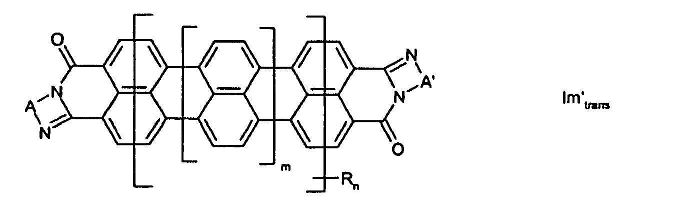 dans laquelle les variables présentent la signification mentionnée dans la revendication 1, les deux radicaux A et A' étant différents, ou d'un mélange des deux isomères, **caractérisé en ce qu'**on condense un dianhydride de l'acide rylènetétracarboxylique de formule générale Ib 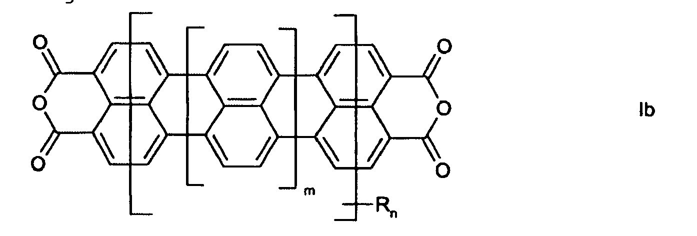 en présence d'un composé basique d'azote ou de phénol comme solvant et d'un acide de Lewis ou de pipérazine comme catalyseur, d'abord avec 1 à 1,5 mole/mole d'une diamine aromatique de formule générale IV
H₂N-A-NH₂ IV
puis avec 1 à 1,5 mole/mole d'une diamine aromatique de formule générale IV'
H₂N-A'-NH₂ IV'

15. Procédé pour la préparation de dérivés de l'acide rylènetétracarboxylique de formule générale In 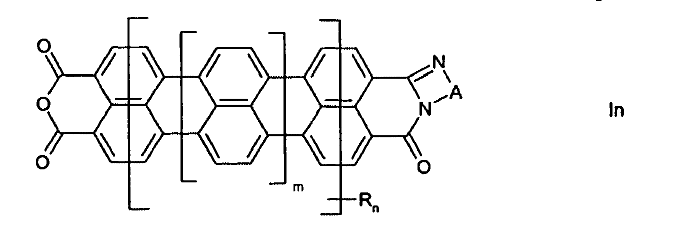 dans laquelle les variables présentent la signification mentionnée dans la revendication 1, **caractérisé en ce qu'**on condense
un dianhydride de l'acide rylènetétracarboxylique de formule générale Ib 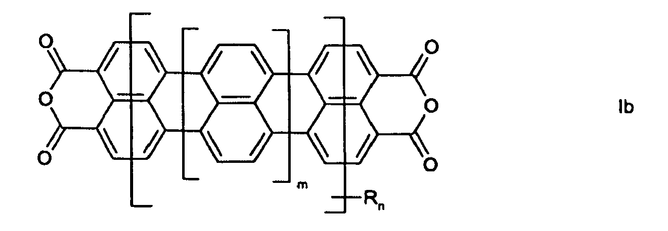 en présence d'un composé basique d'azote ou de phénol comme solvant et d'un acide de Lewis ou de pipérazine comme catalyseur, avec 1 à 1,5 mole/mole d'une diamine aromatique de formule générale IV
H₂N-A-NH₂ IV

16. Procédé pour la préparation de dérivés de l'acide rylènetétracarboxylique de formule générale Io 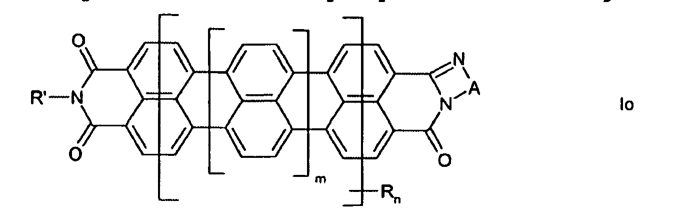 dans laquelle les variables présentent la signification mentionnée dans la revendication 1, **caractérisé en ce qu'**on condense
un mono-imido-monoanhydride de l'acide rylènetétracarboxylique de formule générale Ia 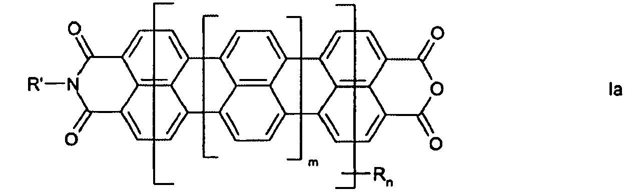 en présence d'un composé basique d'azote ou de phénol comme solvant et d'un acide de Lewis ou de pipérazine comme catalyseur, avec 1 à 1,5 mole/mole d'une diamine aromatique de formule générale IV
H₂N-A-NH₂ IV

17. Procédé pour la préparation de dérivés de l'acide rylènedicarboxylique de formule générale Ip 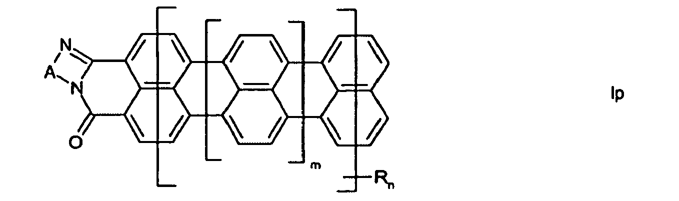 dans laquelle les variables présentent la signification mentionnée dans la revendication 1, **caractérisé en ce qu'**on
a) soumet un dérivé de l'acide rylènetétracarboxylique de formule générale In 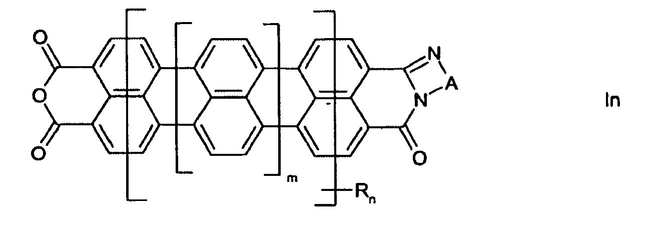 à une décarboxylation en présence d'un composé tertiaire basique d'azote et d'un catalyseur de métal de transition ou
b) condense un anhydride de l'acide rylènedicarboxylique de formule générale Ig 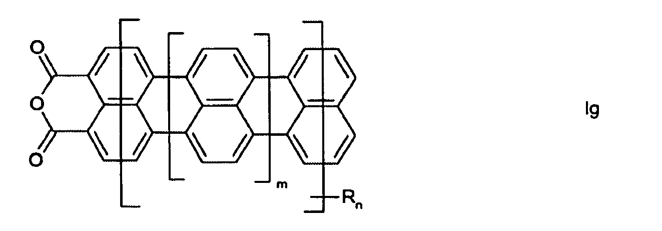 en présence d'un composé basique d'azote ou de phénol comme solvant et d'un acide de Lewis ou de pipérazine comme catalyseur, avec 1 à 1,5 mole/mole d'une diamine aromatique de formule générale IV
H₂N-A-NH₂ IV

18. Procédé pour la préparation de dérivés de l'acide rylènedicarboxylique périhalogéné de formule générale Iq 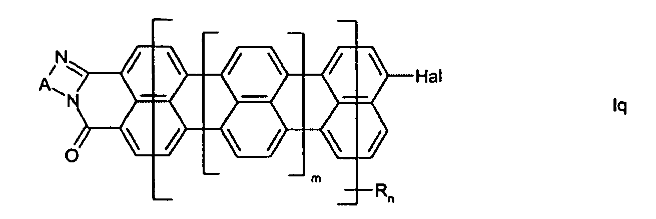 dans laquelle les variables présentent la signification mentionnée dans la revendication 1, **caractérisé en ce qu'**on transforme
un dérivé de l'acide rylènedicarboxylique de formule générale Ip 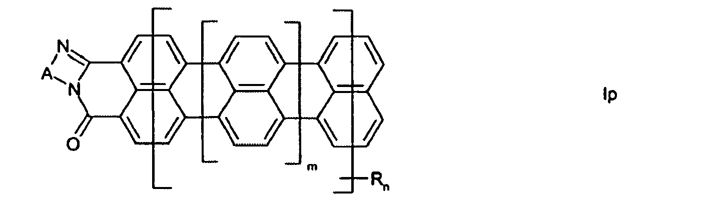 en présence d'un solvant organique polaire et d'un acide de Lewis comme catalyseur avec du N-halogénosuccinimide.

19. Procédé pour la préparation de dérivés de l'acide péri-(dioxaborolan-2-yl)rylènedicarboxylique de formule générale Ir 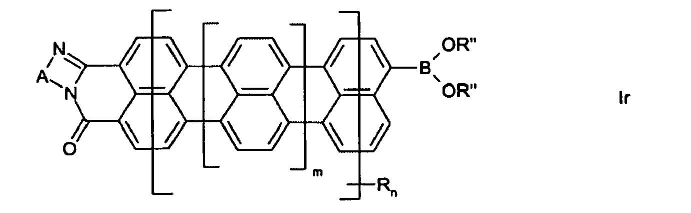 dans laquelle les variables présentent la signification mentionnée dans la revendication 1, **caractérisé en ce qu'**on transforme
un dérivé de l'acide rylènedicarboxylique périhalogéné de formule générale Iq 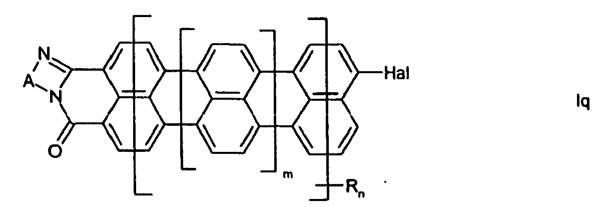 dans laquelle Hal signifie halogène, en présence d'un solvant organique aprotique, d'un catalyseur de métal de transition et d'une base avec un diborane de formule générale III

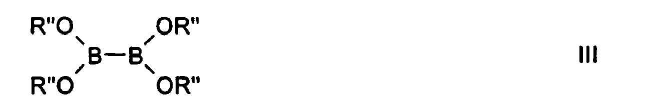

20. Utilisation des dérivés de rylène de formule I selon la revendication 1 pour teinter des matériaux organiques et inorganiques de haut poids moléculaire.

21. Utilisation selon la revendication 20, **caractérisée en ce que** les matériaux de haut poids moléculaire sont des laques, des encres d'imprimerie ou des matériaux synthétiques.

22. Utilisation de dérivés de rylène de formule I selon la revendication 1 comme adjuvants de dispersion et additifs pigmentaires pour des pigments organiques.

23. Utilisation de dérivés de rylène de formule I selon la revendication 1 pour la préparation de dispersions aqueuses de polymères absorbant dans la plage infrarouge proche du spectre électromagnétique.

24. Utilisation de dérivés de rylène de formule I selon la revendication 1 pour obtenir des marquages et des inscriptions absorbant la lumière infrarouge, invisibles pour l'oeil humain.

25. Utilisation de dérivés de rylène de formule I selon la revendication 1 comme absorbant d'infrarouges pour la gestion de la chaleur.

26. Utilisation de dérivés de rylène de formule I selon la revendication 1 comme matériaux absorbant les rayons laser IR lors du traitement par soudure de pièces en matériau synthétique.

27. Utilisation des dérivés de rylène de formule I selon la revendication 1 comme semi-conducteurs dans l'électronique organique.

28. Utilisation des dérivés de rylène de formule I selon la revendication 1 comme émetteurs dans les utilisations d'électroluminescence et de chimiluminescence.

29. Utilisation des dérivés de rylène de formule I selon la revendication 1 comme composants actifs dans la photovoltaïque.
